# EUROPEAN PATENT APPLICATION

(11) **EP 4 793 646 A1**
(43) Date of publication of application: **19.08.2026**
(21) Application number: 26158380.1
(22) Date of filing: 13.02.2026
(51) Int. Cl.: G01N 35/00, G16H 30/40, G16H 50/20, G01N 1/28, G01N 15/1433, G06V 20/69

(54) **SYSTEM**

(30) Priority: 17.02.2025 JP 2025023585; 17.02.2025 JP 2025023586; 17.02.2025 JP 2025023587; 17.02.2025 JP 2025023588
(71) Applicant: SYSMEX CORPORATION, Kobe-shi, Hyogo 651-0073 (JP)
(72) Inventor: NAKAMURA, Tsukasa, Kobe-shi, Hyogo 651-0073 (JP); KARINO, Takashi, Kobe-shi, Hyogo 651-0073 (JP)
(74) Representative: Becker Kurig & Partner Patentanwälte mbB

(57) **Abstract**

Disclosed is a specimen measurement system operable to optically perform a measurement on a specimen, the specimen measurement system comprising: a specimen analyzer configured to optically perform the measurement on the specimen, the specimen analyzer including: (i) a specimen suction part configured to suction the specimen, the specimen suction part comprising a nozzle to suction the specimen in a specimen container, a movement mechanism configured to move the nozzle into the specimen container and a pump configured to apply a negative pressure so that the nozzle suctions the specimen; (ii) a sample preparator configured to prepare a measurement sample by mixing the suctioned specimen and a reagent in a container; (iii) an optical detector configured to detect light from the prepared measurement sample; and (iv) a controller configured to control, according to control software, the specimen suction part, the sample preparator and the optical detector so that the specimen analyzer optically performs the measurement on the specimen, wherein the specimen analyzer analyzes, in order to obtain a measurement result, optical information which is derived from the detected light, and manages, according to management software, data relating to the specimen analyzer, wherein the data includes the measurement result; an imaging apparatus configured to capture images of blood cells in a smear of the specimen; and a computer capable of running an interface which interacts with application software programmed to utilize the data and the image, wherein the application software is independent of the specimen analyzer, wherein the interface executes operations including: (a) receiving a request from the application software; (b) retrieving, according to a predetermined rule, the images corresponding to the request, wherein the retrieved images are associated with metadata including morphological features of the blood cells; and (c) providing the retrieved images to the application software so that the application software displays, based on the metadata, a plurality of candidates indicating classification for a specific blood cell in the retrieved images that visually demonstrate the morphological features of the blood cells.

## Description

### FIELD OF THE INVENTION

The present disclosure relates to a system.

### BACKGROUND OF THE INVENTION

Chinese Patent Application Publication No. 114255833 discloses a graphical user interface for displaying cell images and auxiliary information related to a classification result of the cell images in a smear of the same subject, in a system for measuring and analyzing a specimen.

### SUMMARY OF THE INVENTION

Chinese Patent Application Publication No. 114255833 disclosed a graphical user interface for displaying the cell images and the auxiliary information related to a classification of the cell images in the smear of the same subject. If a function of software is to be expanded by adding such a new function, a modification of the software may be necessary. However, when the software has been certified by a certification institution as a component of a medical device, it may become necessary to acquire the certification again in accordance with the modification. Due to these circumstances, expanding the functions of the software would not be easily performed in a conventional specimen measurement system.

An object of the present invention is to provide a system capable of easily expanding a function of displaying a plurality of candidates indicating classification for a specific blood cell in images that visually demonstrate morphological features of blood cells.

The present invention relates to a specimen measurement system operable to optically perform a measurement on a specimen, the specimen measurement system comprising: a specimen analyzer configured to optically perform the measurement on the specimen, the specimen analyzer including: (i) a specimen suction part configured to suction the specimen, the specimen suction part comprising a nozzle to suction the specimen in a specimen container, a movement mechanism configured to move the nozzle into the specimen container and a pump configured to apply a negative pressure so that the nozzle suctions the specimen; (ii) a sample preparator configured to prepare a measurement sample by mixing the suctioned specimen and a reagent in a container; (iii) an optical detector configured to detect light from the prepared measurement sample; and (iv) a controller configured to control, according to control software, the specimen suction part, the sample preparator and the optical detector so that the specimen analyzer optically performs the measurement on the specimen, wherein the specimen analyzer analyzes, in order to obtain a measurement result, optical information which is derived from the detected light, and manages, according to management software, data relating to the specimen analyzer, wherein the data includes the measurement result; an imaging apparatus configured to capture images of blood cells in a smear of the specimen; and a computer capable of running an interface which interacts with application software programmed to utilize the data and the image, wherein the application software is independent of the specimen analyzer, wherein the interface executes operations including: (a) receiving a request from the application software; (b) retrieving, according to a predetermined rule, the images corresponding to the request, wherein the retrieved images are associated with metadata including morphological features of the blood cells; and (c) providing the retrieved images to the application software so that the application software displays, based on the metadata, a plurality of candidates indicating classification for a specific blood cell in the retrieved images that visually demonstrate the morphological features of the blood cells.

According to the present invention, it is possible to provide a system capable of easily expanding a function of displaying the plurality of candidates indicating classification for the specific blood cell in the images that visually demonstrate the morphological features of the blood cells.

### BRIEF DESCRIPTION OF THE DRAWINGS

FIG. 1 is a diagram showing components of a specimen measurement system according to the present embodiment.
FIG. 2 is a diagram showing a configuration example of a computer.
FIG. 3 is a diagram showing a configuration example 1 of the specimen measurement system according to the present embodiment.
FIG. 4 is a diagram showing a configuration example 1 of the specimen measurement system according to the present embodiment.
FIG. 5 is a diagram showing a configuration example 2 of the specimen measurement system according to the present embodiment.
FIG. 6 is a diagram showing a configuration example 3 of the specimen measurement system according to the present embodiment.
FIG. 7 is a diagram showing a configuration example 4 of the specimen measurement system according to the present embodiment.
FIG. 8 is a diagram showing a configuration example 4 of the specimen measurement system according to the present embodiment.
FIG. 9 is a diagram showing a configuration example 5 of the specimen measurement system according to the present embodiment.
FIG. 10 is a diagram showing a configuration example 5 of the specimen measurement system according to the present embodiment.
FIG. 11 is a diagram showing a configuration example 6 of the specimen measurement system according to the present embodiment.
FIG. 12 is a diagram showing a configuration example 7 of the specimen measurement system according to the present embodiment.
FIG. 13 is a diagram showing a configuration example of the specimen measurement apparatus and the imaging apparatus.
FIG. 14 is a diagram showing a hardware configuration example of the control unit.
FIG. 15 is a diagram showing a configuration example of the control software and the data management software.
FIG. 16 is a diagram showing a modification example of the configuration example of the control unit.
FIG. 17 is a diagram showing a configuration example of the measurement unit.
FIG. 18 is a diagram showing a configuration example when the measurement unit is a blood cell analyzer.
FIG. 19 is a schematic diagram illustrating the specimen suction part and the sample preparator when supplying a measurement specimen to the FCM detector.
FIG. 20 is a schematic diagram illustrating the specimen suction part and the sample preparator when supplying a measurement specimen to the RBC/PLT detector.
FIG. 21 is a schematic diagram illustrating the specimen suction part and the sample preparator when supplying a measurement specimen to the HGB detector.
FIG. 22 is a diagram showing a configuration example of the imaging unit.
FIG. 23 is a diagram showing a configuration example of an interface.
FIG. 24 is a flow chart showing an example of the flow (linking method) of the process performed by the interface of the specimen measurement apparatus.
FIG. 25 is a diagram showing a configuration example of an interface of a data management system.
FIG. 26 is a diagram showing an example of the data structure of an HTTP request body when lot information of quality control is updated.
FIG. 27 is a diagram showing examples of a request for information regarding the specimen measurement apparatus/a response to the request.
FIG. 28 is a diagram showing examples of a request for information regarding specimen measurement/a response to the request.
FIG. 29 is a diagram showing examples of a request for information regarding maintenance of the specimen measurement apparatus/a response to the request.
FIG. 30 is a diagram showing examples of a request regarding manipulation of the specimen measurement apparatus.
FIG. 31 is a diagram showing an example of a request for information regarding the imaging apparatus /a response to the request.
FIG. 32 is a diagram showing an example of a request for information regarding capturing /a response to the request.
FIG. 33 is a diagram showing an example of a request for information regarding maintenance of the imaging apparatus/ a response to the request.
FIG. 34 is a diagram showing an example of a request regarding manipulation of the imaging apparatus.
FIG. 35 is a diagram showing a configuration example of a PUSH notification from the specimen measurement apparatus to the data management system.
FIG. 36 is a diagram showing an example of an event and examples of information (information to serve as the target of PUSH notification) corresponding to the event.
FIG. 37 is a diagram showing a configuration example of an application.
FIG. 38 is a diagram showing a configuration example of providing the function of the application as a Web service.
FIG. 39 is a diagram showing another configuration example of providing the function of the application as a Web service.
FIG. 40 is a diagram showing still another configuration example of providing the function of the application as a Web service.
FIG. 41 is a diagram showing a configuration example of an application providing server that provides the application.
FIG. 42 is a diagram showing a configuration example of providing the application in a secure communication environment.
FIG. 43 is a diagram showing a configuration example of providing the application by utilizing MDM or MAM.
FIG. 44 is a diagram showing an example of a data structure when a database of the MDM/MAM system is a relational database.
FIG. 45 is a diagram showing an example 1 of providing a result of capturing an image of blood cells and a plurality of candidates of a specific blood cell through a GUI.
FIG. 46 is a diagram showing an example 2 of providing a result of capturing an image of blood cells and a plurality of candidates of a specific blood cell through a GUI.
FIG. 47 is a diagram showing an example of a data structure in a data storage according to the present embodiment.
FIG. 48 is a flowchart showing a flow of processing by software according to the present embodiment.
FIG. 49 is a flowchart showing details of a flow of processing by software according to the present embodiment.
FIG. 50 is a flowchart showing a modification example of a flow of processing by software according to the present embodiment.
FIG. 51 is a diagram showing an example 3 of providing a result of capturing an image of blood cells and a plurality of candidates of a blood cell through a GUI.
FIG. 52 is a diagram showing the example 3 of providing the result of capturing the image of the blood cells and the plurality of candidates of the blood cell through the GUI.
FIG. 53 is a diagram showing an example 4 of providing a result of capturing an image of blood cells and a plurality of candidates of a blood cell through a GUI.
FIG. 54 is a diagram showing the example 4 of providing the result of capturing the image of the blood cells and the plurality of candidates of the blood cell through the GUI.
FIG. 55 is a diagram showing an example 5 of providing a result of capturing an image of blood cells and a plurality of candidates of a blood cell through a GUI.
FIG. 56 is a diagram showing an example 6 of providing a result of capturing an image of blood cells and a plurality of candidates of a blood cell through a GUI.

### DETAILED DESCRIPTION

### (Components of Specimen Measurement System)

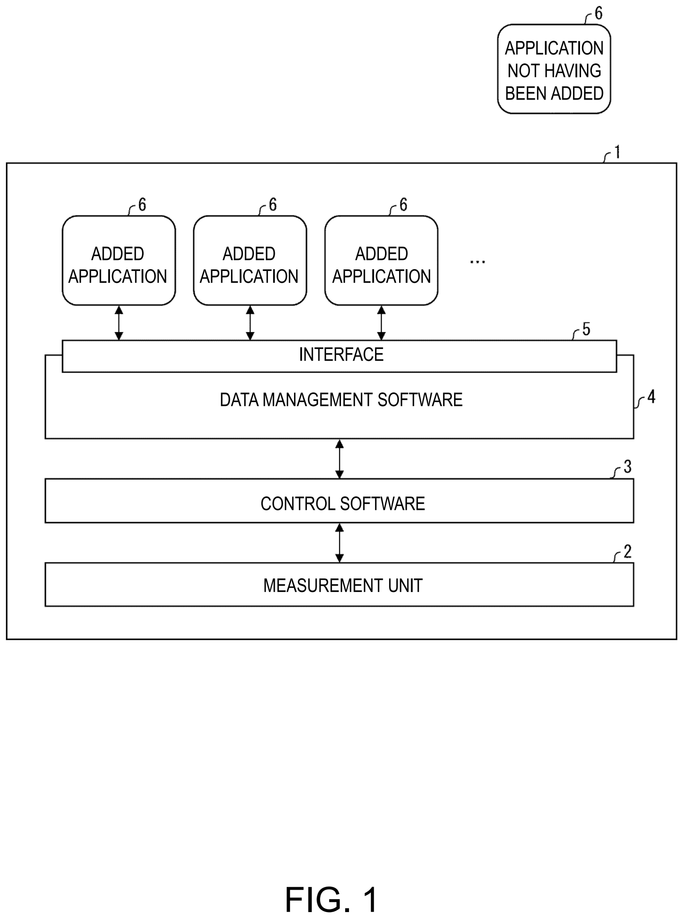

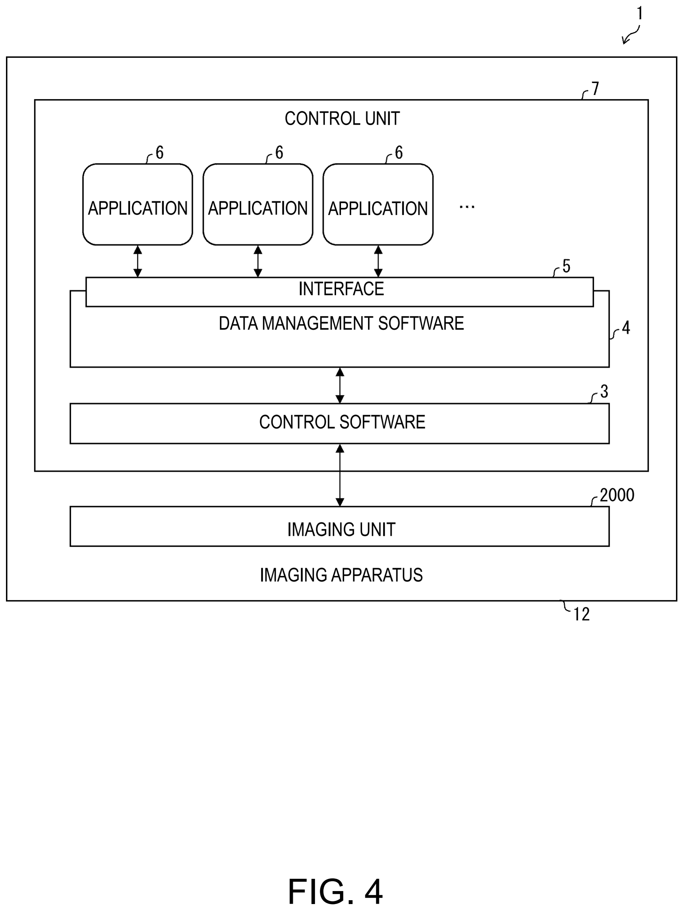

As shown in FIG. 1, a specimen measurement system 1 according to the present embodiment includes, as an example, a measurement unit 2, control software 3, data management software 4, and an interface 5, as components. The measurement unit 2, for example, measures a specimen and acquires measurement data. The control software 3 and the data management software 4 may be integrally formed. For example, the specimen measurement system 1 is the specimen measurement apparatus including the measurement unit 2, the control software 3, the data management software 4, and the interface 5. The specimen measurement system 1 may include, for example, in addition to the specimen measurement apparatus, an imaging apparatus 12 that includes an imaging unit 2000, the control software 3, the data management software 4, and the interface 5, as shown in FIG. 4. The imaging unit 2000, for example, captures an image of a smear by smearing a specimen on a slide. For example, the specimen measurement system 1 is a system including: the specimen measurement apparatus including the measurement unit 2, the control software 3, the data management software 4, and the interface 5, other apparatuses; and other pieces of software. The specimen measurement apparatus is, for example, a blood cell analyzer that acquires a hematology test result by classifying and counting blood cell components contained in a blood specimen. The imaging apparatus 12 is, for example, a blood cell imaging apparatus that captures an image of a smear by smearing a blood specimen on a slide.

The measurement unit 2 measures a specimen allocated to the measurement unit 2, and acquires data corresponding to a measurement result. For example, the measurement unit 2 may be a unit configured to execute a measurement for blood cell analysis. The measurement unit 2 operates under control by the control software 3. The measurement unit 2 provides acquired data to the data management software 4 via the control software 3. The imaging unit 2000 captures an image of a smear loaded into the imaging unit 2000 and acquires data corresponding to the image. An example of the imaging unit 2000 is a unit that performs capturing the image for blood cell analysis. The imaging unit 2000 operates under control by the control software 3. The imaging unit 2000 provides the acquired data to the data management software 4 via the control software 3.

The control software 3 controls the operation of the measurement unit 2 in cooperation with the data management software 4. The control software 3 orders the measurement unit 2 to execute measurement operation corresponding to a measurement order with respect to the specimen allocated to the measurement unit 2. The control software 3 may analyze data acquired by the measurement unit 2, and acquires measurement data (i.e., a measurement result). The control software 3 also controls the operation of the imaging unit 2000 in cooperation with the data management software 4. The control software 3 orders the imaging unit 2000 to execute a imaging operation corresponding to an imaging order for the smear loaded into the imaging unit 2000. The control software 3 analyzes data acquired by the imaging unit 2000, and acquires imaging data that is the image.

The data management software 4 manages data regarding the measurement unit 2. The data management software 4 has a function (for example, UI: User interface) of providing the measurement result acquired by the control software 3, to an operator. The data management software 4 has a function to operate the specimen measurement apparatus such as registration and acquisition of a measurement order, and a function of providing information regarding the measurement order to the control software 3. The data management software 4 may classify the data acquired by the measurement unit 2, and may manage the data based on the classification. For example, the data management software 4 may classify and manage the data according to a type of the cooperating measurement unit 2. For example, the type of the measurement unit 2 may include a unit configured to execute a measurement for blood cell analysis. The control software 3 and the data management software 4 may be different software which are independent with each other. Software having a function equivalent to that of the control software 3 and a function equivalent to that of the data management software 4 may execute control of the measurement unit 2 and management of data regarding the measurement unit 2. In addition, the data management software 4 manages data regarding the imaging unit 2000. The data management software 4 has a function (e.g., UI: User interface) of providing the image acquired by the control software 3, to the operator. The data management software 4 has a function to operate the imaging apparatus operation such as registration and acquisition of an imaging order, and a function of providing information regarding the imaging order to the control software 3. The data management software 4 may manage the data based on classification according to the type of data regarding the imaging unit 2000. For example, the data management software 4 may classify and manage the data according to the type of the cooperating imaging unit 2000. The type of the imaging unit 2000 may be, for example, a unit that performs capturing an image for blood cell analysis. The control software 3 and the data management software 4 may be different software which are independent with each other. Software having a function equivalent to that of the control software 3 and a function equivalent to that of the data management software 4 may execute control of the imaging unit 2000 and management of data regarding the imaging unit 2000.

The measurement unit 2 or imaging unit 2000, the control software 3, and the data management software 4 described above may require certification by a certification institution as a medical device, for example. A medical device requiring certification is an in vitro diagnostic medical device, for example. In this case, the measurement unit 2, the control software 3 and the data management software 4 provide functions corresponding to the intended use of the certified medical device, for example. The intended use of the medical device is to measure a specimen and provide a measurement result, for example. When the medical device is a blood cell analyzer, for example, the intended use is to measure a blood specimen and provide a measurement result (for example, red blood cell count, white blood cell count, white blood cell classification, and the like) regarding blood cells.

The application software (hereinafter, simply referred to as "application") 6 is software independent of the control software 3 and the data management software 4. For example, the application 6 has a function having been incorporated in software of a prior art regarding an apparatus for measuring a specimen. The application 6 has, for example, a function having been incorporated in software of a prior art regarding an apparatus for capturing an image of a smear of the specimen. That is, the application 6 has a function separated from conventional software regarding an apparatus for measuring the specimen or an apparatus for capturing an image of the smear. The application 6 may have a new function that was not incorporated into conventional software regarding an apparatus for measuring the specimen, for example. The application 6 may have a new function that was not incorporated into conventional software regarding an apparatus for capturing an image of the smear, for example. In the specimen measurement system 1 according to the present embodiment, for example, a plurality of candidates indicating classification for a specific blood cell are displayed based on metadata including information indicating a cell type of a blood cell image of the specimen. For example, the specimen measurement system 1 acquires information regarding the plurality of candidates from the metadata, and may display the plurality of candidates in association with each classification probability of the plurality of candidates, or may display the plurality of candidates in association with each classification probability in descending order of the classification probability. Displaying the plurality of candidates of the specific blood cell is executed by the application software 6 capable of being added to the specimen measurement system 1. The specimen measurement system 1 displays, for example, the plurality of candidates of the specific blood cell in the blood cell image in which the specific blood cell in the specimen is captured, based on the above-mentioned metadata. Displaying the plurality of candidates of the specific blood cell is executed by, for example, the application software 6 capable of being added to the specimen measurement system 1. As described above, the specimen measurement system 1 is capable of displaying the plurality of candidates of the specific blood cell in the blood cell image while displaying the blood cell image in which the specific blood cell in the specimen is captured. The specimen measurement system displays not only the blood cell image in which the specific blood cell in the specimen is captured but also the plurality of candidates of the specific blood cell in the blood cell image. Thereby, the specimen measurement system 1 can provide a function for multi-facetted evaluation of analysis results of the blood cell image. In the specimen measurement system 1 according to the present embodiment, for example, application software having a function to provide a hematology test result of the specimen and a result of capturing the image of blood cells in the specimen (a result of capturing the image of the smear of the specimen) can be added to the system. For example, in this specimen measurement system 1, the hematology test result of the specimen acquired by the specimen measurement apparatus 11 and the result of capturing the image of the blood cells in the specimen captured by the imaging apparatus 12 (a result of capturing the image of the smear of the specimen) can be displayed. Accordingly, the user can confirm the hematology test result and the result of capturing the image of the blood cells (a result of capturing the image of the smear) while comparing the hematology test result and the result of capturing the image of the blood cells. By allowing the user to review the hematology test result and the result of capturing the image of the blood cells (a result of capturing the image of the smear) while comparing them with each other, the user can, for example, evaluate the validity of the result of capturing the image of the blood cells (a result of capturing the image of the smear) with reference to the hematology test result, or evaluate the validity of the hematology test result with reference to the result of capturing the image of the blood cells (a result of capturing the image of the smear). For example, the application 6 provides a function different from a function corresponding to the "Intended Use" of a medical device. Examples of the application 6 are software for a healthcare usage other than a medical usage (hereinafter, referred to as "non-medical device software") and software for non-healthcare usage (hereinafter, referred to as "non-health software"). For example, the non-medical device software and the non-health software are software that does not require certification or that requires limited certification as a medical device. The application 6 may be an application requiring certification as the medical device.

The application 6 is capable of being added to the specimen measurement system 1. In other words, the application 6 is capable of being add-on to the specimen measurement system 1. The application 6 added to the specimen measurement system 1 serves as a component of the specimen measurement system 1. For example, the application 6 added to the specimen measurement system 1 provides a function of utilizing information (for example, data regarding the measurement unit 2, data regarding the imaging unit 2000) managed by the data management software 4, and a function of utilizing the function of the data management software 4. For example, the functions provided by the application 6 are (i) a function that the data management software 4 does not have, and (ii) a function that expands an existing function of the data management software 4. The functions provided by the application 6 are new functions different from the function corresponding to the intended use as the medical device, for example. The application 6 added to the specimen measurement system 1 may provide a function for expanding the specimen measurement system 1. In other words, the application 6 added to the specimen measurement system 1 may be said to provide a function that complements the data management software 4.

Since the application 6 is separated/isolated from the data management software 4 and the control software 3, the application 6 may be easily added to the specimen measurement system 1 without modifying the data management software 4 and the control software 3. Moreover, for example, if the application 6 is the non-medical device software or the non-health software that does not require certification as the medical device, it may not be necessary to acquire the certification by the certification institution when the application 6 is added to the specimen measurement system 1. When the application 6 is to be deleted from the specimen measurement system 1 as well, it may not be necessary to modify the data management software 4 and the control software 3.

The application 6 separated/isolated from the data management software 4 is capable of communicating with the data management software 4 via the interface 5. For example, the interface 5 is a software interface. For example, the interface 5 is an API (Application Programming Interface). For example, the interface 5 allows the application 6 to utilize information (for example, data regarding the measurement unit 2 and the imaging unit 2000 such as measurement data and imaging data) managed by the data management software 4 and the function of the data management software 4. For example, the interface 5 accepts a request from the application 6 and performs a response corresponding to the request against the application 6, thereby allowing the application 6 to utilize data corresponding to the request or utilize a function corresponding to the request. The request from the application 6 is a request for utilization of data managed by the data management software 4 and/or a request for utilization of a function of the data management software 4, for example. The response to the application 6 is provision of data corresponding to the request and/or provision of a function corresponding to the request, for example.

The application 6 may communicate with the interface 5 in accordance with a predetermined rule (hereinafter, referred to as "rule R") defined in advance. The interface 5 may allow the application 6 to utilize the information/function regarding the data management software 4 according to the rule R. For example, when the interface 5 is configured as a Web API, the rule R is, as an example, determined as a combination of a URI (Uniform Resource Identifier) for designating a processing subject by a predetermined syntax, and a predetermined HTTP (Hyper Text Transfer Protocol) method indicating manipulation with respect to the processing subject. According to the rule R, the application 6 creates, as a request to the interface 5, a combination of a URI and an HTTP method that the processing subject and manipulation with respect to the processing subject are specified. Hereinafter, the request is also referred to as "command C". The application 6 transmits a command C created according to the rule R to the interface 5. The interface 5 provides a response according to the command C transmitted from the application 6, to the application 6. For example, according to the command C transmitted from the application 6, the interface 5 allows the application 6 to utilize at least one of: (i) data regarding the measurement unit 2, (ii) data regarding measurement operation by the measurement unit 2, (iii) data regarding maintenance of the measurement unit 2, and (iv) data regarding manipulation of the measurement unit 2. For example, according to the command C transmitted from the application 6, the interface 5 allows the application 6 to utilize data regarding the imaging unit 2000, data regarding manipulation of the imaging unit 2000, and the like. Furthermore, for example, the interface 5 allows the application 6 to perform at least one of acquisition of data regarding the measurement unit 2, registration of the data, update of the data, and deletion of the data, based on the command C transmitted from the application 6. For example, there may be plurality of types of rule R depending on the type of the specimen measurement apparatus 11. For example, the manner of information management of the data management software 4 or the data management system 15 may differ depending on the type of the specimen measurement apparatus 11. In such a case, for example, by the interface 5/5A and the application 6 collaborating based on plurality of types of rule R corresponding to the manner of information management, the application 6 becomes capable of utilizing plurality of types of information each having a different manner of information management. The interface 5 allows the application 6 to perform at least one of acquisition of data regarding the imaging unit 2000, registration of the data, update of the data, and deletion of the data, based on the command C transmitted from the application 6.

The interface 5 may allow a plurality of types of applications 6 to utilize the data regarding the measurement unit 2 according to the rule R which is common among the plurality of types of applications 6. The plurality of types of applications 6 may provide different functions. The rule R may be common among the applications 6 which operate in different operating systems, respectively. For example, the interface 5 may allow the applications 6 to utilize data regarding the measurement unit 2 according to the rule R which is common among a plurality of types of the applications 6 respectively created for a plurality of types of operating systems.

The interface 5 may be incorporated in the data management software 4 or may be incorporated in a software program different from the data management software 4. The interface 5 may be software independent of (i) the data management software 4, (ii) the control software 3, and (iii) other software programs. The data management software 4 may be configured as one piece of software (for example, single executable file whose extension is ".exe"), or may be configured as a plurality of pieces of software (for example, a plurality of executable files whose extensions are ".exe"). For example, the software configured as the interface 5 and the software configured as the data management software 4 may be configured as a plurality of different executable files.

### (Example of Software Operating Environment)

The control software 3, the data management software 4, the interface 5, and the application 6 are executed, for example, in one or more computers 900. For example, the computer 900 may be (i) a smartphone (for example, iPhone, Android terminal), (ii) a tablet (for example, iPad, Android tablet, Windows tablet), (iii) a Windows PC having Windows OS installed therein, or (iv) a Mac having Mac OS installed therein (iPhone, iPad, Android, Windows, and Mac OS are each a registered trademark). The application 6 may be created for a plurality of types of operating systems, for example. For example, with respect to a certain application 6, one for Windows OS and one for iOS may be respectively created. That is, the application 6 may be created for a plurality of operating systems.

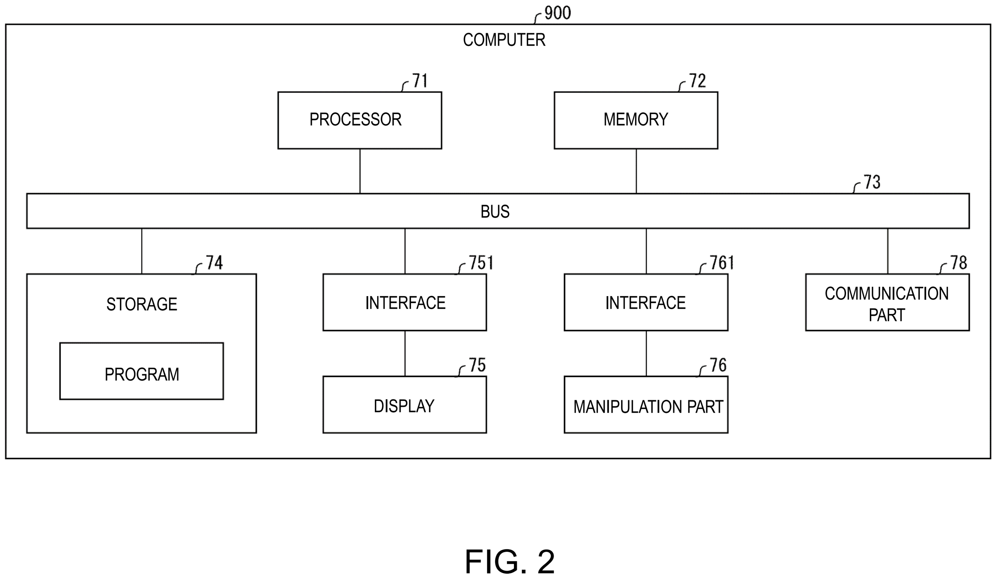

A configuration example of the computer 900 is shown in FIG. 2. As an example, the computer 900 includes a processor 71, a memory 72, a bus 73, a storage 74, a display 75, an interface 751, a manipulation part 76, an interface 761, and a communication part 78. A terminal apparatus 101 and a control unit 7, which will be described later, have a configuration similar to that of the computer 900 shown in FIG. 2.

The storage 74 is, for example, a nonvolatile memory such as an SSD (Solid State Drive), a flash memory, an HDD (Hard Disk Drive), or a combination of these. The storage 74 stores therein, for example, various types of programs including the control software 3, the data management software 4, the interface 5, and the application 6, and the like, and various types of data.

The processor 71 is, for example, an IPU (Infrastructure Processing Unit), a CPU (Central Processing Unit), a GPU (Graphic Processing Unit), a DSP (Digital Signal Processor), an MPU (Micro Processing Unit), or a combination of these. The processor 71 may be realized by a logic circuit, for example. The processor 71 reads out various types of programs from the storage 74 to be expanded into the memory 72, to execute the programs. The memory 72 is, for example, a volatile memory such as a RAM (Random Access Memory) or a DRAM (Dynamic Random Access Memory).

The interface 751 connects the bus 73, and the display 75 such as a display to each other. The interface 761 connects the bus 73, and a manipulation part 76 such as a keyboard and a mouse to each other. The communication part 78 is responsible for communication with an external apparatus.

Hereinafter, in order to simplify description of operation of the control software 3, the data management software 4, the interface 5, or the application 6, description may be given with omission of members respectively corresponding to the processor 71, the storage 74, and the memory 72 for executing these.

The computer 900 may further include a communication interface for communicating with other apparatuses. The computer 900 may further include an input/output interface for connecting input/output devices such as a keyboard, a mouse, a display, and a printer.

### (Configuration Examples of Specimen Measurement System)

### (Configuration Example 1)

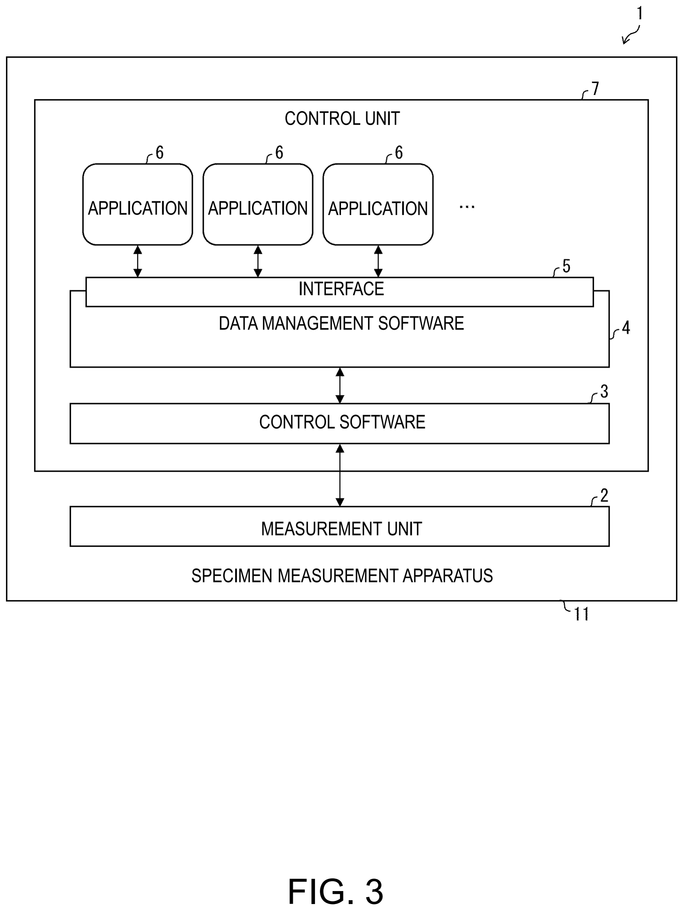

FIG. 3 shows a configuration example of the specimen measurement system 1. In the configuration example shown in the figure, the specimen measurement system 1 is configured as the specimen measurement apparatus 11 including a measurement unit 2, and the control unit 7 that executes the control software 3, the data management software 4, the interface 5, and the application 6. In the case of the present configuration example, the application 6 is executed in the specimen measurement apparatus 11, and utilizes, via the interface 5, information managed by the data management software 4 and the function of the data management software 4. The specimen measurement system 1 may include, for example, in addition to the specimen measurement apparatus 11, an imaging apparatus 12 shown in FIG. 4. The imaging apparatus 12 includes, for example, the imaging unit 2000, and a control unit 7 that executes the control software 3, the data management software 4, the interface 5, and the application 6.

### (Configuration Example 2)

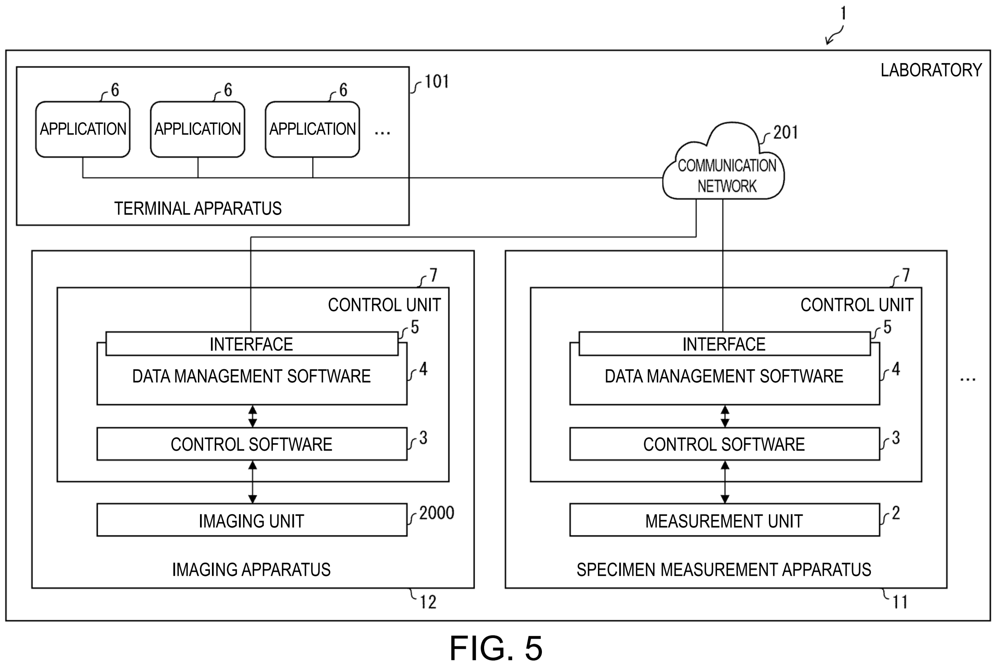

The application 6 may be installed in an apparatus different from the specimen measurement apparatus 11and the imaging apparatus 12. In the configuration example shown in FIG. 5, the specimen measurement system 1 includes: the specimen measurement apparatus 11 placed in a laboratory, the imaging apparatus 12 placed in the laboratory, and terminal apparatus 101 used in the laboratory and connected to the specimen measurement apparatus 11 and the imaging apparatus 12 via a communication network 201. The terminal apparatus 101 is used in the laboratory, for example. The communication network 201 is, for example, an intra network.

The specimen measurement apparatus 11 includes: the measurement unit 2; and the control unit 7 that executes the control software 3, the data management software 4, and the interface 5. The imaging apparatus 12 includes the imaging unit 2000, and a control unit 7 that executes the control software 3, the data management software 4, and the interface 5. The terminal apparatus 101 is a computer used by a user of the specimen measurement system 1, and is, for example, a computer such as a PC, a smartphone, or a tablet. The terminal apparatus 101 has the application 6 installed therein and executes the application 6.

In the case of the present configuration example, the application 6 executed in the terminal apparatus 101 accesses the interface 5 of the specimen measurement apparatus 11 and the imaging apparatus 12 via the communication network 201. The interface 5 allows the application 6 of the terminal apparatus 101 to utilize information managed by the data management software 4 and the function of the data management software 4 via the communication network 201.

### (Configuration Example 3)

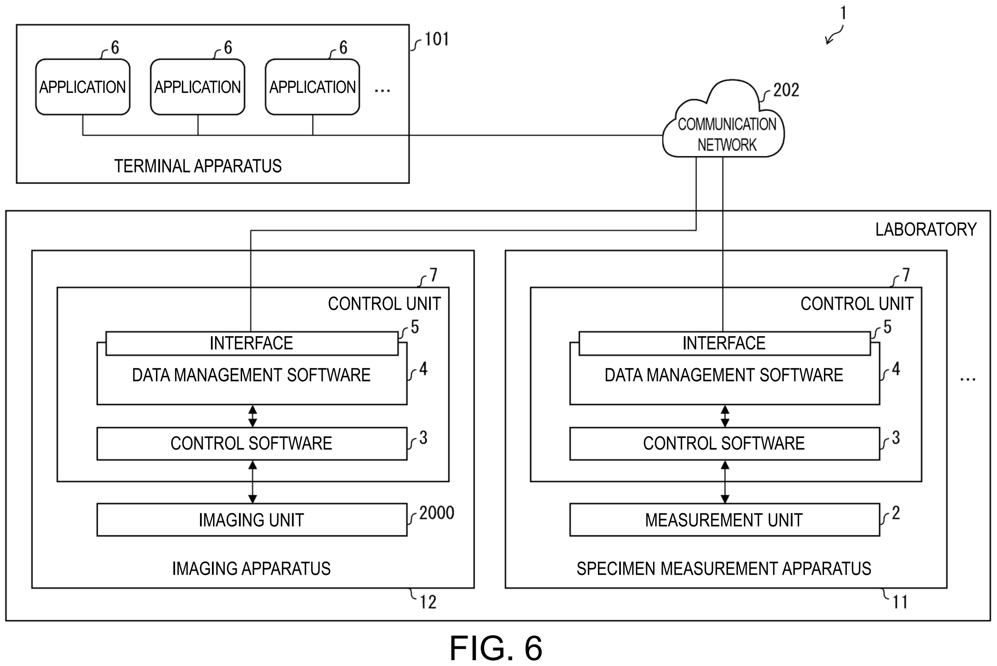

The terminal apparatus 101 including the application 6 installed therein may be used in a location (for example, outside the laboratory, another room in the same facility where the laboratory is present) different from the facility where the specimen measurement apparatus 11 and the imaging apparatus 12 are placed (e.g., outside the laboratory, or in a separate room in the same facility as the laboratory). In the configuration example shown in FIG. 6, the specimen measurement system 1 includes: the specimen measurement apparatus 11 and the imaging apparatus 12 placed in the laboratory; and a terminal apparatus 101 used in a facility different from the laboratory and connected to the specimen measurement apparatus 11 and the imaging apparatus 12 via a communication network 202. The communication network 202 is, for example, the Internet or an intranet. The specimen measurement apparatus 11 includes the measurement unit 2, and a control unit 7 which executes the control software 3, the data management software 4, and the interface 5. The imaging apparatus 12 includes the imaging unit 2000, and a control unit 7 that executes the control software 3, the data management software 4, and the interface 5. The terminal apparatus 101 includes the application 6 installed therein and executes the application 6.

In the case of the present configuration example, the application 6 executed in the terminal apparatus 101 accesses the interface 5 of the specimen measurement apparatus 11 via the communication network 202. The interface 5 allows the application 6 of the terminal apparatus 101 to utilize information managed by the data management software 4 and the function of the data management software 4, via the communication network 202. The application 6 executed in the terminal apparatus 101 accesses the interface 5 of the imaging apparatus 12 via the communication network 202. The interface 5 allows the application 6 of the terminal apparatus 101 to utilize information managed by the data management software 4, and the function of the data management software 4 via the communication network 202.

### (Configuration Example 4)

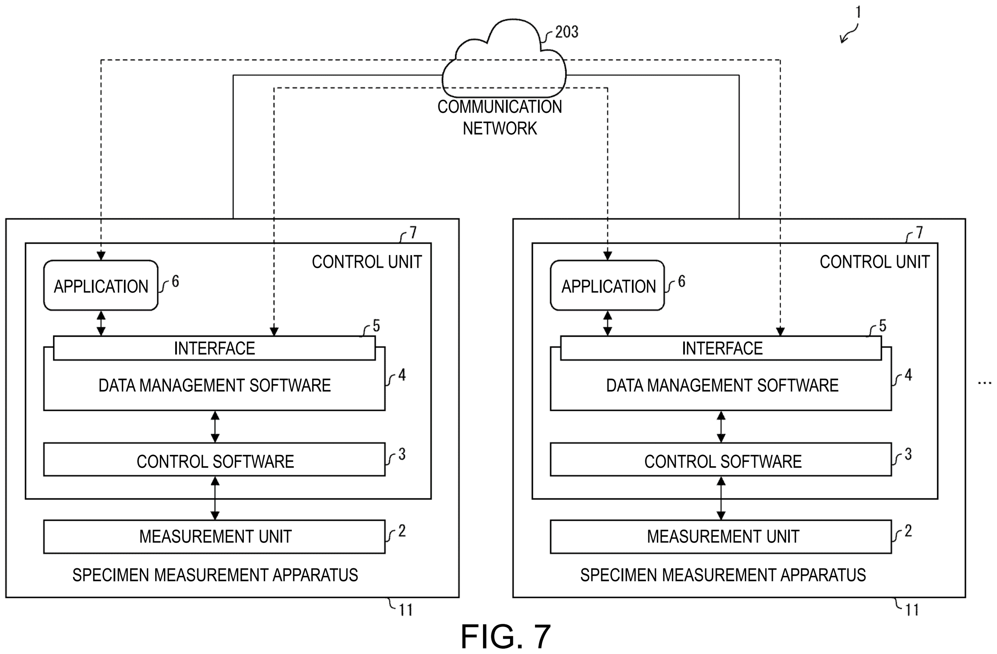

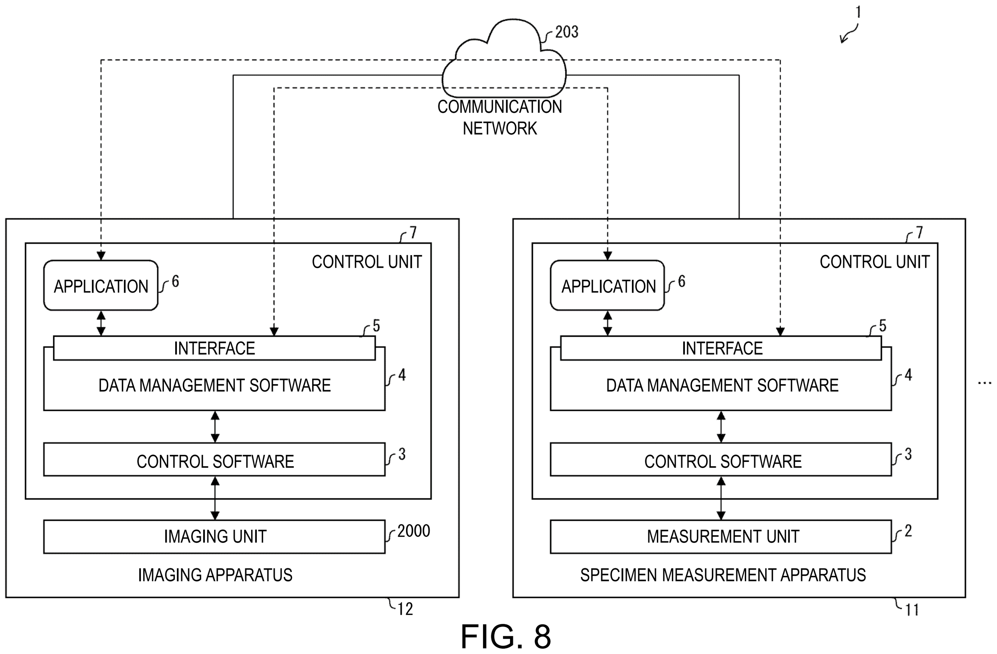

When the specimen measurement apparatus 11 includes the application 6 installed therein, the application 6 itself installed in the specimen measurement apparatus 11 may access another specimen measurement apparatus 11 (hereinafter, it may be called "second specimen measurement apparatus 11") and utilize information managed by the data management software 4 of the other specimen measurement apparatus 11 and the function of the data management software 4 of the second specimen measurement apparatus 11. In the configuration example shown in FIG. 7, the specimen measurement system 1 has a configuration in which the specimen measurement apparatuses 11 that each include: the measurement unit 2; and the control software 3, the data management software 4, the interface 5, and the control unit 7 that executes the application 6 are communicably connected to each other via the communication network 203. The communication network 203 is, for example, an intra network or the Internet. In the configuration example shown in FIG. 8, the specimen measurement system 1 has a configuration in which a specimen measurement apparatus 11 that include: the measurement unit 2; the control software 3; the data management software 4; the interface 5; and a control unit 7 which executes the application 6, and an imaging apparatus 12 that include: the imaging unit 2000; the control software 3; the data management software 4; the interface 5; and a control unit 7 which executes the application 6, are communicably connected to each other via a communication network 203. The communication network 203 is, for example, an intra network or the Internet.

In the case of the present configuration example shown in FIG. 7, the application 6 executed in the specimen measurement apparatus 11 can utilize, via the interface 5 of the specimen measurement apparatus 11, information managed by the data management software 4 of the specimen measurement apparatus 11 and the function of the data management software 4, and can also utilize, by accessing the interface 5 of the second specimen measurement apparatus 11 connected via the communication network 203, information managed by the data management software 4 of the second specimen measurement apparatus 11 and the function of the data management software 4. In the case of this configuration example shown in FIG. 8, for example, the application 6 executed in the imaging apparatus 12 can utilize, via the interface 5 of the imaging apparatus 12, information managed by the data management software 4 of the imaging apparatus 12 and the function of the data management software 4, and can also utilize, by accessing the interface 5 of the specimen measurement apparatus 11 connected via the communication network 203, information managed by the data management software 4 of the specimen measurement apparatus 11 and the function of the data management software 4.

### (Configuration Example 5)

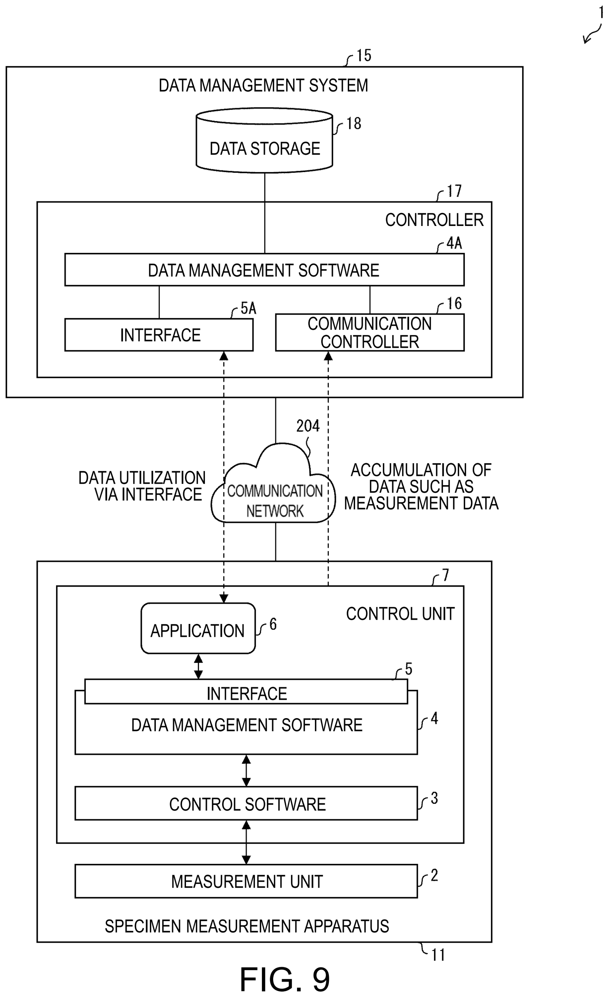

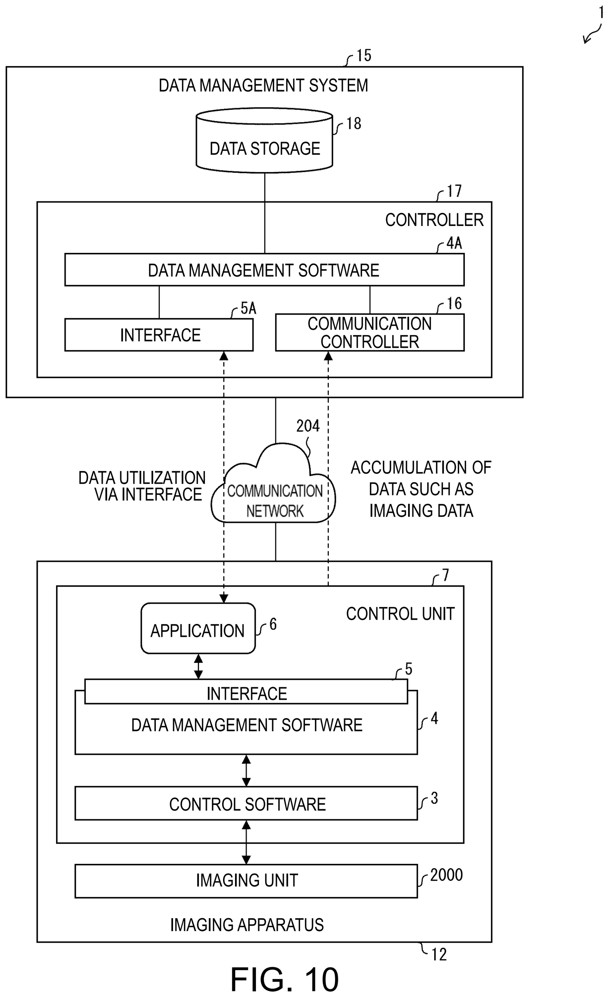

Information (for example, measurement data and the like) managed by the data management software 4 may be managed by an apparatus different from the specimen measurement apparatus 11. In the configuration example shown in FIG. 9, the specimen measurement system 1 includes: the specimen measurement apparatus 11; and the data management system 15 connected to the specimen measurement apparatus 11 via a communication network 204. The communication network 204 is, for example, an intra network or the Internet. Information (for example, measurement data and the like) managed by the data management software 4 may be managed by both the specimen measurement apparatus 11 and the data management system 15. For example, a duplicate of all or a part of the information managed by the data management software 4 of the specimen measurement apparatus 11 may be managed by the data management system 15. In the configuration example shown in FIG. 10, the specimen measurement system 1 includes: the imaging apparatus 12; and a data management system 15 connected to the imaging apparatus 12 via the communication network 204. The communication network 204 is, for example, the intra network or the Internet. Information (for example, imaging data and the like) managed by the data management software 4 may be managed by both the imaging apparatus 12 and the data management system 15. For example, a duplicate of all or a part of the information managed by the data management software 4 of the imaging apparatus 12 may be managed by the data management system 15.

As shown in FIG. 9, the specimen measurement apparatus 11 includes: the measurement unit 2, the control software 3, the data management software 4, the interface 5, and the control unit 7 that executes the application 6. The specimen measurement apparatus 11 includes a function for providing information managed by the data management software 4 to the data management system 15. Although details will be described later, as an example of the function, the interface 5 may provide, in accordance with a predetermined event, information (measurement data and the like) corresponding to the event to the data management system 15 by a PUSH notification via the communication network 204. The PUSH notification may be executed by a component (for example, the data management software 4, another piece of software installed in the control unit 7) other than the interface 5. Instead of the PUSH notification, the specimen measurement apparatus 11 may provide, as a response to an inquiry from the data management system 15, information managed by the data management software 4 to the data management system 15. The process to provide the information to the data management system 15 is the same as configuration examples 6 and 7 described later. As shown in FIG. 10, the imaging apparatus 12 includes: the imaging unit 2000, the control software 3, the data management software 4, the interface 5, and a control unit 7 that executes the application 6. The imaging apparatus 12 includes a function for providing information managed by the data management software 4 to the data management system 15. Although details will be described later, as an example of the function, the interface 5 may provide, in accordance with a predetermined event, information (imaging data and the like) corresponding to the event to the data management system 15 by a PUSH notification via the communication network 204. The PUSH notification may be executed by a component (for example, the data management software 4, another piece of software installed in the control unit 7) other than the interface 5. Instead of the PUSH notification, the imaging apparatus 12 may provide as a response to an inquiry from the data management system 15, information managed by the data management software 4 to the data management system 15, similar to the specimen measurement apparatus 11.

As shown in FIG. 9, the data management system 15 includes, for example, a controller 17 that executes data management software 4A, an interface 5A, and a communication controller 16. The controller 17 is equivalent to the computer 900 shown in FIG. 2. The data management system 15 includes a data storage 18 being a storage device. The data management software 4A manages the information provided by the PUSH notification from the specimen measurement apparatus 11. The information is stored in the data storage 18. The communication controller 16 includes a function of controlling communication regarding the PUSH notification from the specimen measurement apparatus 11. As shown in FIG. 10, the data management software 4A may store and manage, in the data storage 18, the information provided by the PUSH notified from the imaging apparatus 12. The communication controller 16 includes a function of controlling communication regarding the PUSH notification from the imaging apparatus 12.

As shown in FIG. 9, the application 6 is capable of communicating with the data management software 4A via the interface 5A. The data management software 4A acquires, from the data storage 18, data corresponding to a request from the application 6 received by the interface 5A, and, as a response to the request, provides the data to the application 6. The interface 5A includes a function of allowing the application 6 executed in the specimen measurement apparatus 11 to utilize information managed in the data storage 18, via the data management software 4A. The interface 5A may be equivalent to the interface 5. For example, the interface 5A is a software interface, and is an API. The interface 5A allows the application 6 to utilize information managed by the data management software 4A. The application 6 communicates with the interface 5A according to the rule R, and the interface 5A allows the application 6 to utilize the function/information regarding the data management software 4A according to the rule R. As shown in FIG. 10, the interface 5A includes a function of allowing the application 6 executed in the imaging apparatus 12 to utilize information managed in the data storage 18, via the data management software 4A. The interface 5A may be equivalent to the interface 5. For example, the interface 5A is a software interface, and is an API. The interface 5A allows the application 6 to utilize information managed by the data management software 4A. The application 6 communicates with the interface 5A according to the rule R, and the interface 5A allows the application 6 to utilize the function/information regarding the data management software 4A according to the rule R.

In the case of the present configuration example, as shown in FIG. 9, the application 6 executed in the specimen measurement apparatus 11 accesses the interface 5A of the data management system 15 via the communication network 204, and requests utilization of information stored in the data storage 18 of the data management system 15. The data management system 15 may be placed in a laboratory or in a medical facility. In this case, the application 6 of the specimen measurement apparatus 11 accesses the interface 5A via the communication network 204 serving as an intranet, for example. As shown in FIG. 10, the application 6 executed in the imaging apparatus 12 accesses the interface 5A of the data management system 15 via the communication network 204, and requests utilization of information stored in the data storage 18 of the data management system 15. The data management system 15 may be placed in the laboratory or the medical facility. In this case, the application 6 of the imaging apparatus 12 accesses the interface 5A via the communication network 204 serving as an intranet, for example.

### (Configuration Example 6)

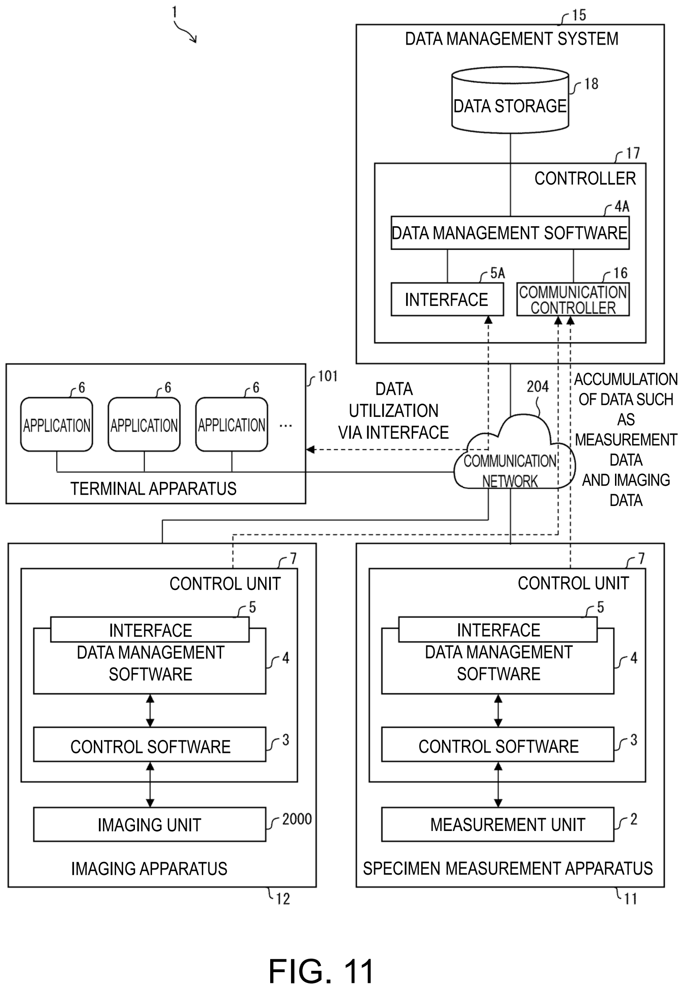

Information stored in the data storage 18 of the data management system 15 may be utilized by the application 6 executed in the terminal apparatus 101. In the configuration example shown in FIG. 11, the specimen measurement system 1 includes a configuration in which the specimen measurement apparatus 11, the imaging apparatus 12, the data management system 15, and the terminal apparatus 101 are connected to each other via the communication network 204.

In the case of the present configuration example, the application 6 executed in the terminal apparatus 101 accesses the interface 5A of the data management system 15 via the communication network 204, and requests utilization of information stored in the data storage 18 of the data management system 15. The application 6 executed in the terminal apparatus 101 communicates with the interface 5A according to the rule R.

### (Configuration Example 7)

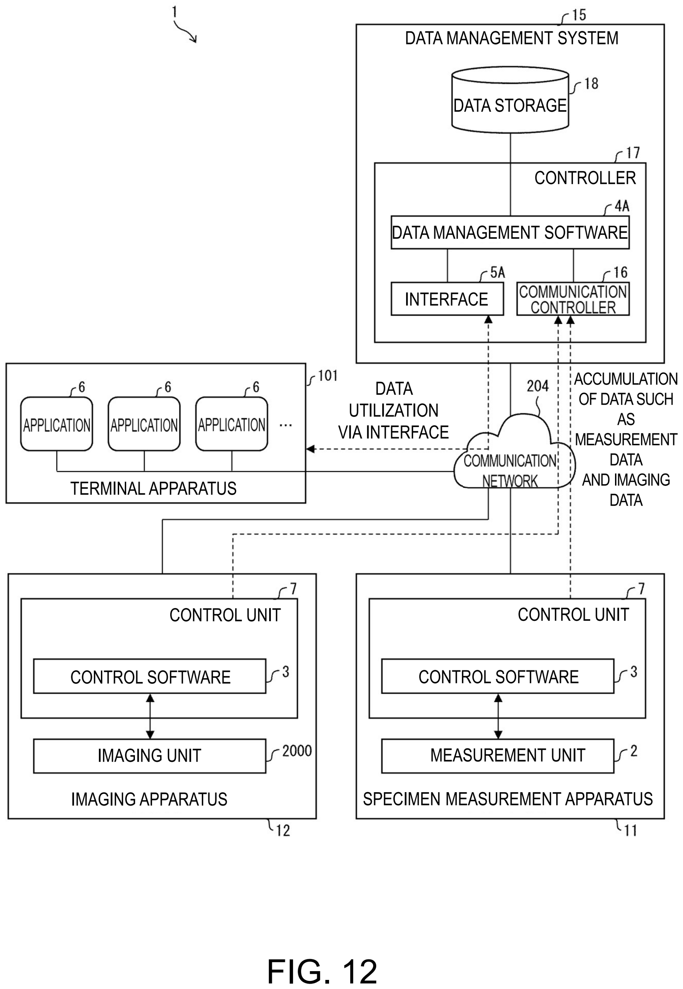

As a modification of the configuration example 6, the specimen measurement apparatus 11 and the imaging apparatus 12 may not include the data management software 4 and the interface 5. The configuration example shown in FIG. 12 is the same as the configuration example 6 except that the specimen measurement apparatus 11 and the imaging apparatus 12 may not include the data management software 4 and the interface 5.

In the case of the present configuration example, information such as measurement data acquired through control of the measurement unit 2 by the control software 3 is transmitted to the data management system 15 via the communication network 204, and is stored into the data storage 18 via the data management software 4A of the data management system 15. Similarly, information such as imaging data acquired through control of the imaging unit 2000 by the control software 3 is transmitted to the data management system 15 via the communication network 204, and is stored into the data storage 18 via the data management software 4A of the data management system 15. Similarly to the configuration example 6, application 6 executed in the terminal apparatus 101 accesses the interface 5A of the data management system 15 via the communication network 204, and requests utilization of information stored in the data storage 18 of the data management system 15.

### (Configuration Example of Specimen Measuring Apparatus/Imaging Apparatus)

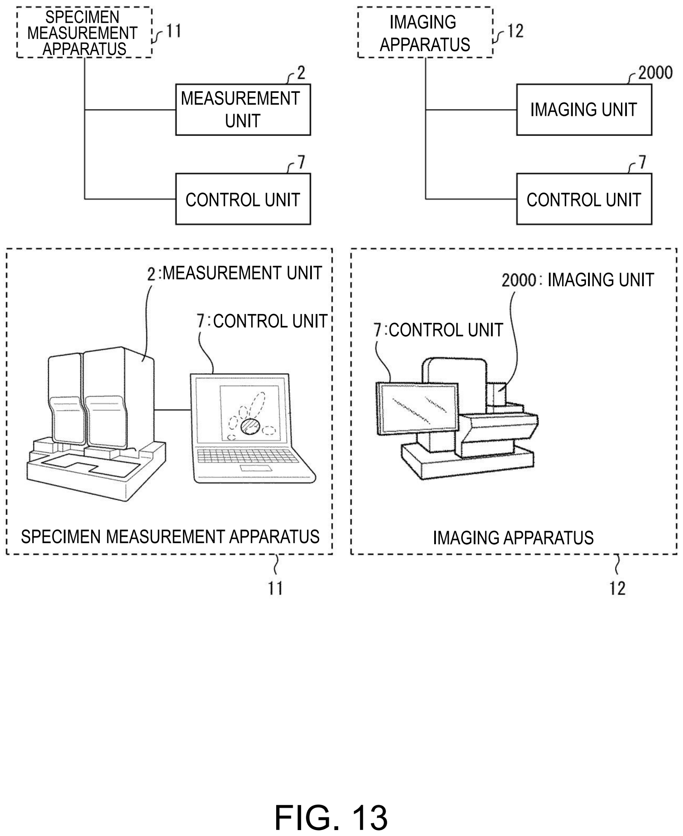

As described above, the specimen measurement apparatus 11 includes, as an example, the control unit 7 and the measurement unit 2 as shown in FIG. 13. The imaging apparatus 12 includes, as an example, the control unit 7 and the imaging unit 2000 as shown in FIG. 13.

### (Control Unit)

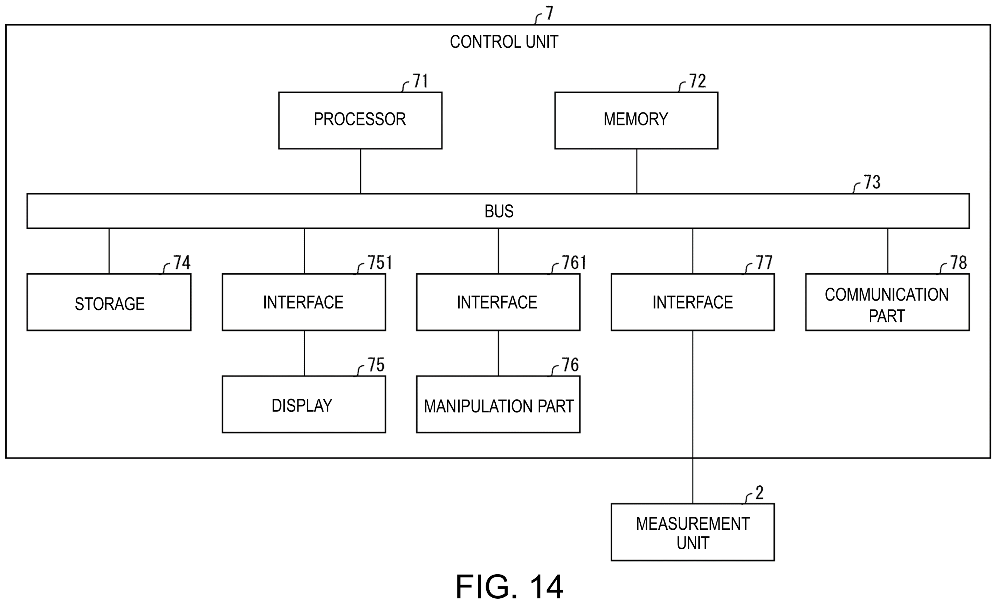

The control unit 7 of the specimen measurement apparatus 11 and the control unit 7 of the imaging apparatus 12 are, for example, a computer. An example of a hardware configuration of the control unit 7 is shown in FIG. 14. The control unit 7 includes, as an example, the processor 71, the memory 72, the bus 73, the storage 74, the display 75, the interface 751, the manipulation part 76, the interface 761, an interface 77, and the communication part 78. FIG. 14 shows an example in which the measurement unit 2 and the control unit 7 are connected via the interface 77. The imaging unit 2000 and the control unit 7 may be connected via the interface 77.

The processor 71 is a controller that comprehensively controls the functions of the control unit 7. The processor 71 is equivalent to the processor 71 shown in FIG. 2. The processor 71 reads out, for example, the control software 3 and the data management software 4 from the storage 74 to be expanded into the memory 72, to execute the control software 3 and the data management software 4. The storage 74 is equivalent to the storage 74 shown in FIG. 2. The memory 72 is equivalent to the memory 72 shown in FIG. 2.

The interface 751 connects the bus 73 and the display 75 such as a display to each other. The interface 761 connects the bus 73 and the manipulation part 76 such as a keyboard and a mouse to each other. The interface 77 connects the bus 73 and the measurement unit 2 to each other. The communication part 78 is responsible for communication with an external apparatus.

### [Configuration Examples of Control Software and Data Management Software]

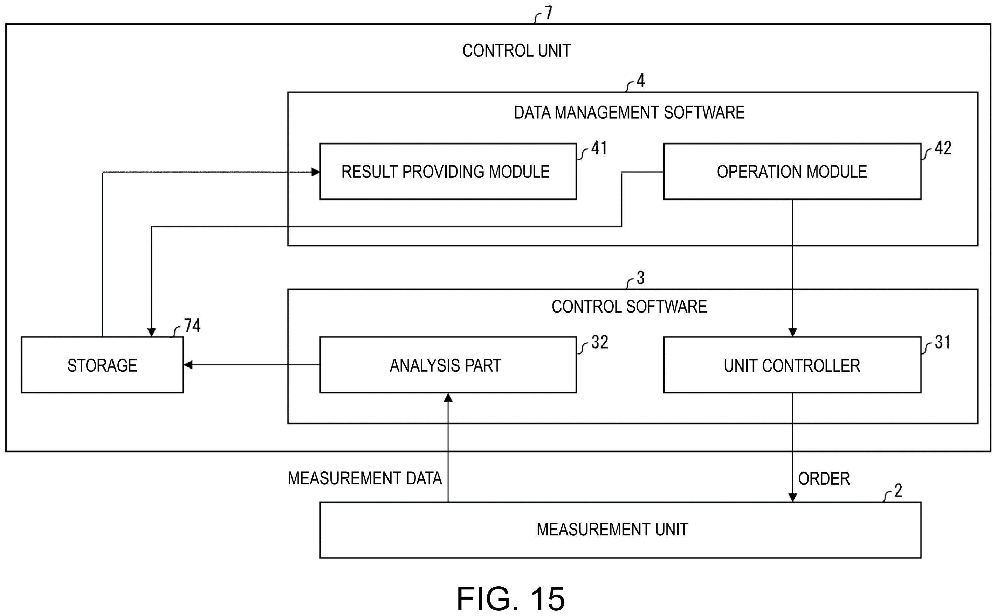

FIG.15 shows configuration examples of the control software 3 and the data management software 4 executed in the control unit 7. The control software 3 includes, for example, a unit controller 31 and an analysis part 32. The unit controller 31, of the control unit 7 connected to the measurement unit 2, orders the measurement unit 2 to execute operation corresponding to a measurement order for a specimen serving as a measurement target. The unit controller 31, of the control unit 7 connected to the imaging unit 2000, orders the imaging unit 2000 to execute operation corresponding to an imaging order for the specimen serving as a capturing target, for example. The analysis part 32, of the control unit 7 connected to the measurement unit 2, analyzes data acquired by the measurement unit 2 having operated in accordance with the order from the unit controller 31, generates measurement data as a measurement result, and stores the generated measurement data in the storage 74, for example. The analysis part 32, of the control unit 7 connected to the imaging unit 2000, analyzes an image captured by the imaging unit 2000, generates a classification result of cells included in the image, and stores the classification result and the image in the storage 74, for example. The control software 3, of the control unit 7 connected to the imaging unit 2000, may not include the analysis part 32. In this case, the control software 3 acquires, from the imaging unit 2000, the image captured by the imaging unit 2000, and stores the acquired image in the storage 74.

The data management software 4 includes, for example, a result providing module 41 and an operation module 42. The result providing unit 41 includes a function (for example, GUI: Graphical User Interface) of acquiring, from the storage 74, the measurement data generated by the analysis part 32, and providing the acquired measurement data to an operator. The operation module 42 includes functions such as a function (UI) of allowing registration of a measurement order/specimen information performed by the operator, a function of acquiring a measurement order and specimen information from a laboratory test information system (LIS: Laboratory Information System) and/or a hospital information system (HIS: Hospital Information System), a function of registering a measurement instruction based on the measurement order, various types of setting functions regarding the apparatus and the system, and an operation function such as a registration of reagent information and consumables information. The information that is set and registered by the operation module 42 is stored into the storage 74. The operation function included in the data management software 4 is, for example, a function (for example, the necessary minimum of functions for measuring a specimen and providing a measurement result according to the intended use) required by the intended use (Intended Use) of the specimen measurement apparatus 11 approved as the medical device. The operation function included in the data management software 4 is, for example, a function required by the intended use (Intended Use) of the specimen measuring apparatus 11 approved as a medical device, and an additional function for enhancing convenience for a user. The operation function of the data management software 4 can be expanded, for example, by addition of the application 6. The operation function included in the data management software 4 may be, for example, a function (for example, necessary minimum of functions for capturing an image of a smear and providing the image according to the intended use) required by the intended use (Intended Use) of the imaging apparatus 12 approved as the medical device, and an additional function for enhancing convenience for a user. The operation function of the data management software 4 can be expanded, for example, by addition of the application 6.

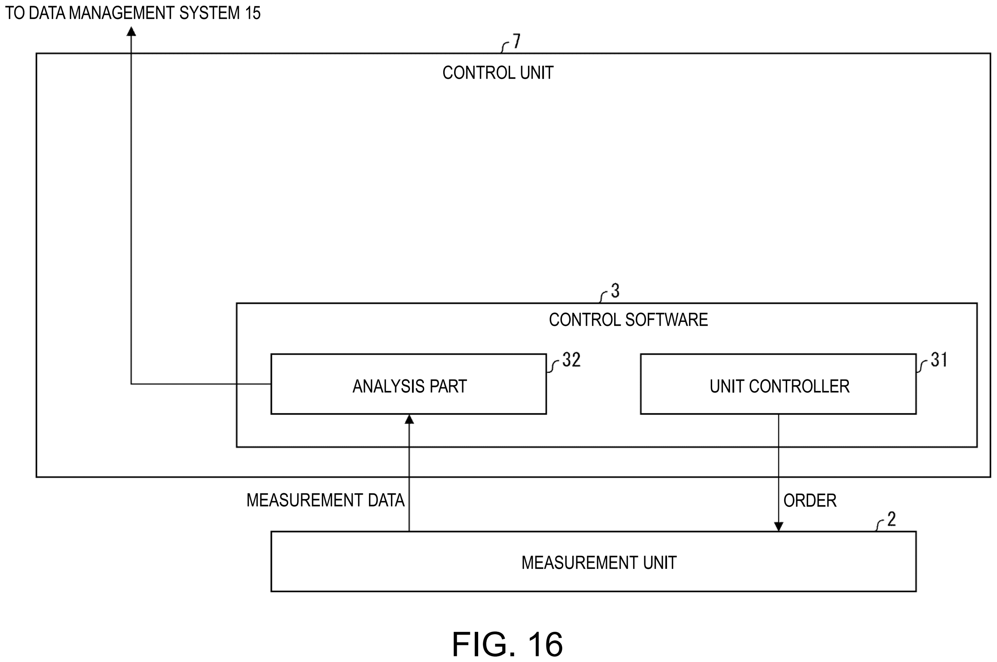

As a modification, the control unit 7 of the specimen measurement apparatus 11 may not include the data management software 4, as shown in FIG. 16. The control unit 7 exemplified in FIG. 16 is used, for example, in the system exemplified in FIG. 12. In this example, the unit controller 31 instructs the measurement unit 2 to execute an operation corresponding to a measurement order acquired from, for example, LIS/HIS. The analysis part 32 analyzes data acquired by the measurement unit 2 operating according to the instruction of the unit controller 31, generates measurement data as a measurement result, and transmits the generated measurement data to the data management system 15 via, for example, the communication part 78. The control unit 7 of the imaging apparatus 12 may not include the data management software 4. In this case, the unit controller 31 instructs the imaging unit 2000 to execute an operation corresponding to an imaging order acquired from, for example, LIS/HIS. The analysis part 32 may analyze data acquired by the imaging unit 2000 operating according to the instruction of the unit controller 31, generate imaging data as an imaging result, and transmit the generated imaging data to the data management system 15.

The specimen measurement apparatus 11, according to the present embodiment, measures a specimen input to the specimen measurement apparatus 11 and acquires data corresponding to a measurement result. The specimen measurement apparatus 11 is, for example, an apparatus that executes measurement for blood cell analysis (for example, CBC: Complete Blood Count). The specimen measurement apparatus 11 may be integrally configured with a plurality of types of apparatuses, and may be, for example, an automatic analysis apparatus integrally configured with an apparatus that executes measurement for blood cell analysis and an apparatus that executes measurement for C-reactive protein (CRP) analysis.

The imaging apparatus 12, according to the present embodiment, captures an image of a smear of a specimen input to the imaging apparatus 12 and acquires data corresponding to the image. The imaging apparatus 12 is, for example, an apparatus that captures an image of blood cells in a smear of the specimen. The imaging apparatus 12 may be integrally configured with a plurality of types of apparatuses, and may be, for example, an apparatus integrally configured with a smear preparation apparatus that prepares a smear by smearing a blood sample on a glass slide and an imaging apparatus.

Each of the specimen measurement apparatus 11 and the imaging apparatus 12 described above may, for example, require certification by a certification authority as a medical device. The medical device requiring certification is, for example, an in vitro diagnostic medical device. The specimen measurement apparatus 11, for example, provides a function corresponding to the intended use of the certified medical device. The intended use of the medical device is, for example, to measure a specimen and to provide a measurement result. When the medical device is a blood cell analyzer, for example, the intended use is to measure a blood specimen of a subject and to provide measurement data regarding blood cells (for example, red blood cell count, white blood cell count, white blood cell classification, and the like). The imaging apparatus 12, for example, provides a function corresponding to the intended use of the certified medical device. The intended use of the medical device is, for example, to capture an image of a smear and to provide the captured image. when the medical device is a blood cell imaging apparatus, for example, the intended use is to capture an image of a smear and to provide morphological data regarding blood cells (for example, classification of band neutrophils, segmented neutrophils, eosinophils, basophils, and the like).

As a system for displaying a hematology test result of a specimen and an image of blood cells in the specimen (an image of a smear of the specimen), for example, each of the hematology test result and the image of the blood cells may be associated with identification information related to the specimen (for example, a specimen ID). In this case, the hematology test result and the image of the blood cells associated with the identification information related to the specimen can be displayed.

Blood tests include, for example, measurement items such as red blood cell count, white blood cell count, platelet count, hemoglobin concentration, hematocrit value. These blood tests are called a hematology test (a blood cell count test), are used for a diagnosis of health conditions, and are a screening test for checking a cured state of a disease. The hematology test is often performed using an automated blood cell analyzer. In such the blood cell analyzer, a flow cytometry method, described later, is employed, and optical information is obtained so that each of cellular components contained in a blood specimen can be classified and counted.

When the hematology test result indicates a possibility of a hematologic disease, in addition to the hematology test, it is necessary to prepare a smear from a blood specimen collected from a subject, to observe morphology of blood cells in the blood specimen to determine whether any morphological abnormalities is present in the blood cells. The smear can be captured by a blood cell imaging apparatus such as the imaging apparatus 12, and each of a plurality of blood cell images in the captured smear can be classified according to each attribute (for example, cell types) of the blood cells, thereby enabling identification of abnormal cells.

The system according to the present embodiment may include each of the specimen measurement apparatus 11 and the imaging apparatus 12, but it is sufficient that at least a result of capturing an image of a smear is acquired from the imaging apparatus 12, and the specimen measurement apparatus 11 need not be included in the system.

### [Configuration Example of GUI]

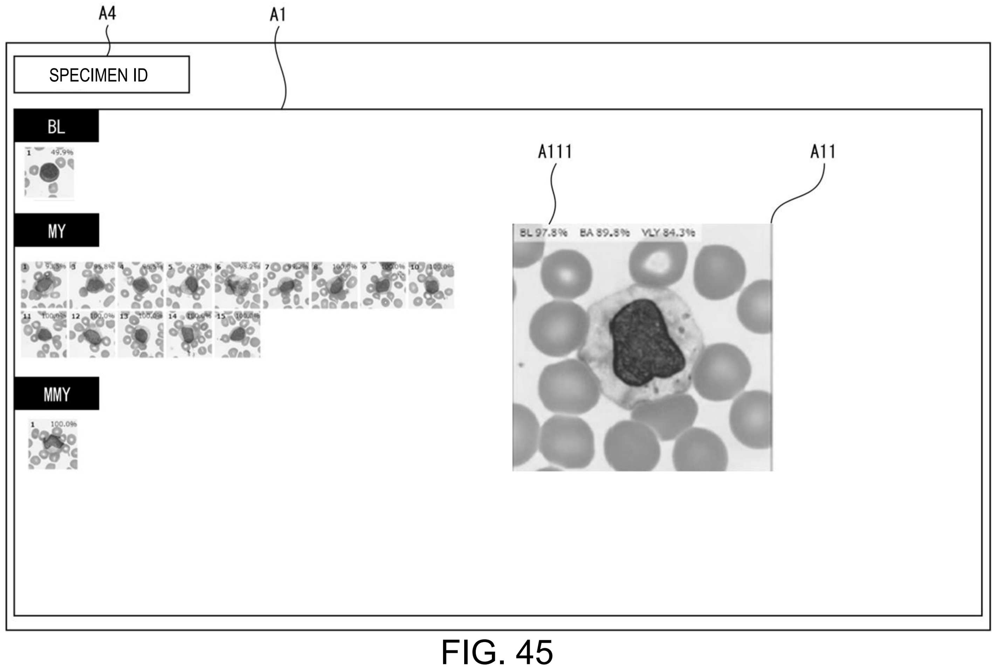

In the configuration shown in FIG. 11, the application software of the terminal apparatus 101 displays the data collected by the data management software of the data management system 15 on a display provided in the terminal apparatus 101. FIG. 45 shows an example of a GUI (Graphical User Interface) that displays data in which each of blood cell images A1 and a plurality of candidates A111 of a specific blood cell in a blood cell image A11 is associated with identification information (specimen ID) A4 related to a specimen. In FIG. 45, display areas for the plurality of blood cell images A1 and the plurality of candidates A111 of the specific blood cell in the blood cell image A11 are provided. For example, the plurality of candidates A111 of the specific blood cell in the blood cell image A11 (for example, BL (blast), BA (basophil), VLY (variant lymphocyte)) are displayed on the GUI. As shown in FIG. 45, by explicitly displaying the plurality of candidates A111 of the specific blood cell in the blood cell image A11 while displaying the plurality of blood cell images A1, it is possible to prevent oversight compared to a case where only a single classification candidate is displayed, and it is possible to evaluate analysis results of the blood cell images in a multi-facetted manner. In the present GUI, the plurality of candidates A111 may be displayed in association with each classification probability of the plurality of candidates A111 (for example, BL: 97.8%, BA: 89.8%, VLY: 84.3%), and various display modes can be applied from the viewpoint of visibility. In the present GUI, a plurality of candidates A111 of specific blood cells in each of the plurality of blood cell images A1 may be displayed. For example, for each of the plurality of blood cell images A1 classified for each specific cell type (for example, BL (blast), BA (basophil), VLY (variant lymphocyte), etc. in FIG. 45), the plurality of candidates may be displayed as an enlarged image in the same manner as the blood cell image A11. In this case, various display modes are also applicable from the viewpoint of visibility. In FIG. 45, the plurality of blood cell images A1 are displayed after being classified according to attributes (for example, BL (blast), MY (myelocyte)). For example, the blood cell images are displayed in a number ranging from several images to several hundred images. The displayed blood cell images may be, for example, individual blood cell images that were captured, or blood cell images generated by stitching together a plurality of blood cell images.

### (Measuring Unit)

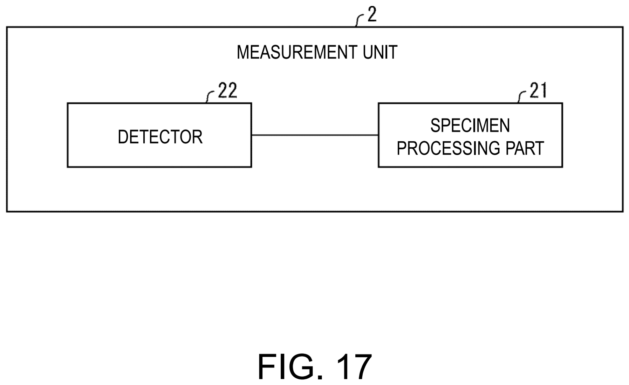

A configuration example of the measurement unit 2 is shown in FIG. 17. The measurement unit 2 includes, as an example, a specimen processing part 21 and a detector 22. The specimen processing part 21 prepares a measurement sample on the basis of a specimen and a reagent. The detector 22 measures the measurement sample prepared by the specimen processing part 21. The unit controller 31 of the control software 3 controls operation of the specimen processing part 21 and the detector 22 linking with (according to an instruction from the operation module 42) the operation module 42 of the data management software 4. The unit controller 31 performs, for example, operation control of a mechanism and a fluid circuit forming the specimen processing part 21 and the detector 22. The operation control may include, for example, at least one of: control of suction operation of a liquid including at least one of a specimen and a reagent; control of mixing the specimen and the reagent to prepare a measurement sample; control of causing the detector 22 to detect the measurement sample; and control of causing the detector 22 to detect at least one of optical information and an electric signal regarding the measurement sample, and analyzing a measurement result on the basis of a detection result having been detected.

### (Blood Cell Analyzer)

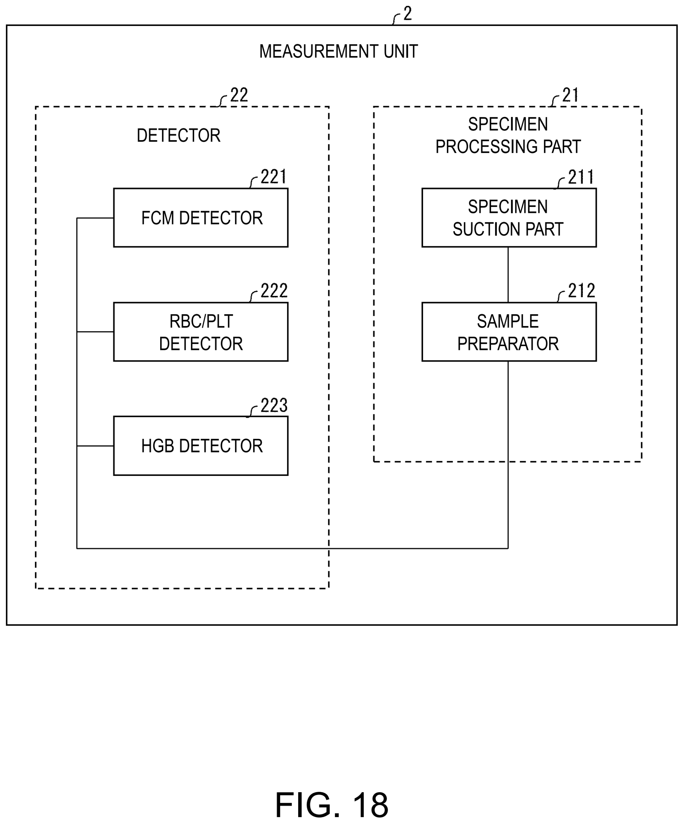

FIG. 18 shows a configuration example when the measurement unit 2 is a blood cell analyzer. In this case, the specimen processing part 21 includes, for example, a specimen suction part 211 and a sample preparator 212, and the detector 22 includes, for example, an FCM detector 221, an RBC/PLT detector 222, and an HGB detector 223.

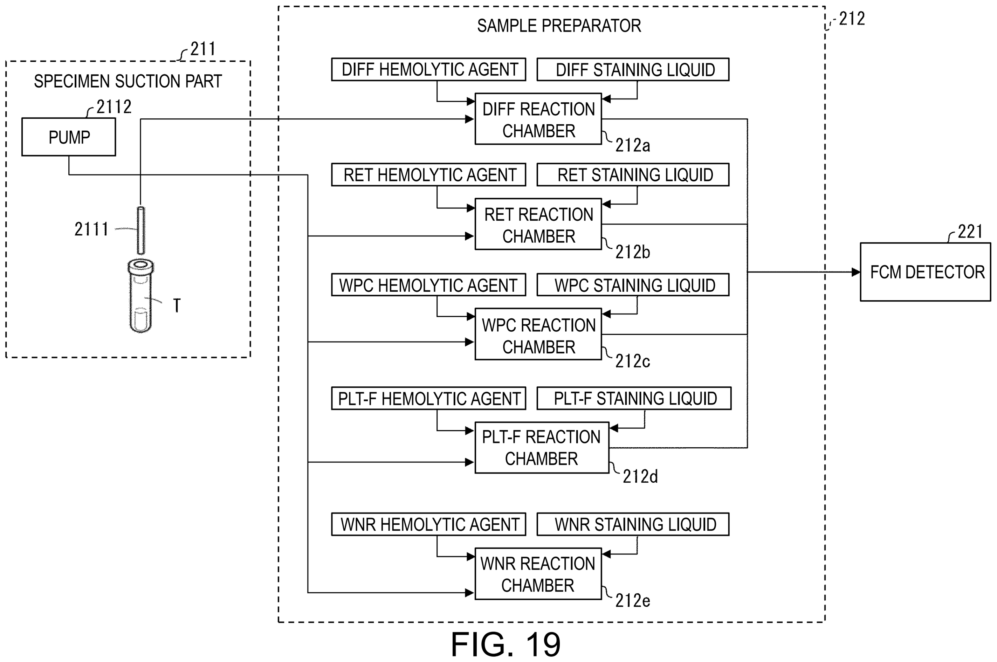

FIG. 19 is a schematic diagram for explaining the specimen suction part 211 and the sample preparator 212 when a measurement sample is supplied to the FCM detector 221. The specimen suction part 211 includes a nozzle 2111 for suctioning a blood specimen (whole blood) from a blood collection tube T, and a pump 2112 for applying a negative pressure/positive pressure to the nozzle 2111. The nozzle 2111 is moved upward and downward by an apparatus mechanism part (not shown), thereby being inserted into the blood collection tube T. When the pump 2112 applies a negative pressure in a state where the nozzle 2111 is inserted in the blood collection tube T, the blood specimen is suctioned via the nozzle 2111. The apparatus mechanism part may include a hand member configured to invert and agitate the blood collection tube T before suctioning blood from the blood collection tube T.

The sample preparator 212 includes five reaction chambers 212a to 212e. The reaction chambers 212a to 212e are respectively used in DIFF, RET, WPC, PLT-F, and WNR measurement channels (also referred to as systems of measurement). Each reaction chamber has connected thereto, via flow paths, a hemolytic agent container containing a hemolytic agent being a reagent and a staining liquid container containing a staining liquid, which correspond to the corresponding measurement channel. One reaction chamber and a reagent (a hemolytic agent and a staining liquid) connected thereto form a measurement channel. For example, the DIFF measurement channel corresponds to a measurement item for classifying (five classification of white blood cells) white blood cells into a plurality of subpopulations, and is composed of a DIFF hemolytic agent and a DIFF staining liquid which are a DIFF measurement reagent, and a DIFF reaction chamber. The RET measurement channel corresponds to a measurement item regarding measurement of reticulocytes, the WPC measurement channel corresponds to a measurement item regarding measurement of abnormal white blood cells, the PLT-F measurement channel corresponds to a measurement item regarding optical measurement of platelets, and the WNR measurement channel corresponds to a measurement item regarding white blood cells and nucleated red blood cells. These measurement channels are also configured similarly to the DIFF measurement channels.

The nozzle 2111 having suctioned the blood specimen accesses, through horizontal/up-down movement by the apparatus mechanism part, a reaction chamber, among the reaction chambers 212a to 212e, that corresponds to the measurement channel corresponding to an order from above, and discharges the suctioned blood specimen. The sample preparator 212 supplies a corresponding hemolytic agent and a corresponding staining liquid to the reaction chamber into which the blood specimen has been discharged, and mixes the blood specimen, the hemolytic agent, and the staining liquid in the reaction chamber, to prepare a measurement sample. The prepared measurement sample is supplied from the reaction chamber to the FCM detector 221 via a flow path, and measurement of cells is performed by a flow cytometry method.

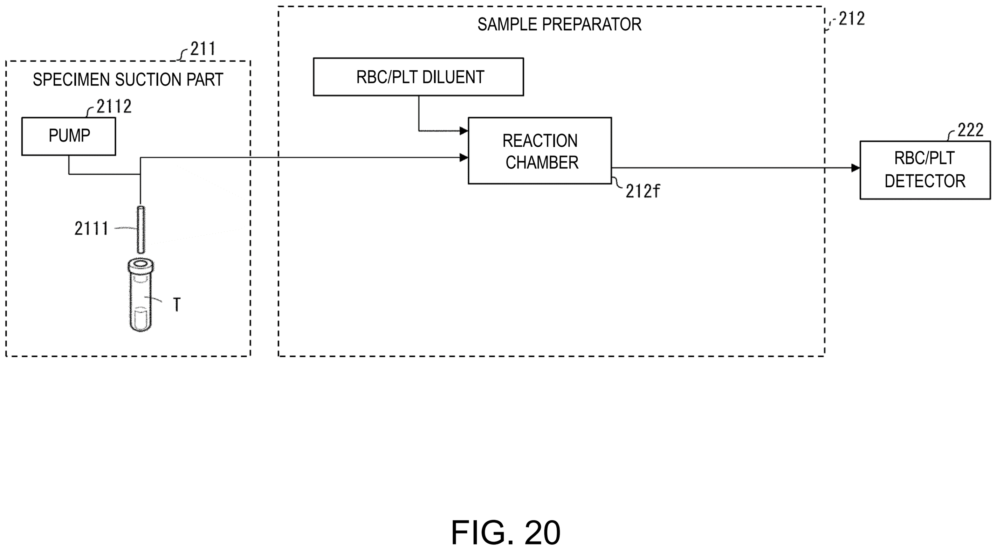

FIG. 20 is a schematic diagram for explaining the specimen suction part 211 and the sample preparator 212 when a measurement sample is supplied to the RBC/PLT detector 222. The specimen suction part 211 is the same as the specimen suction part 211 shown in FIG. 17. The sample preparator 212 includes a reaction chamber 212f. The reaction chamber 212f is connected to a diluent container containing a diluent corresponding to RBC/PLT via a flow path. The measurement sample is prepared by mixing the blood specimen and the diluent in the reaction chamber 212f. The prepared measurement sample is supplied from the reaction chamber to the RBC/PLT detector 222 via a path.

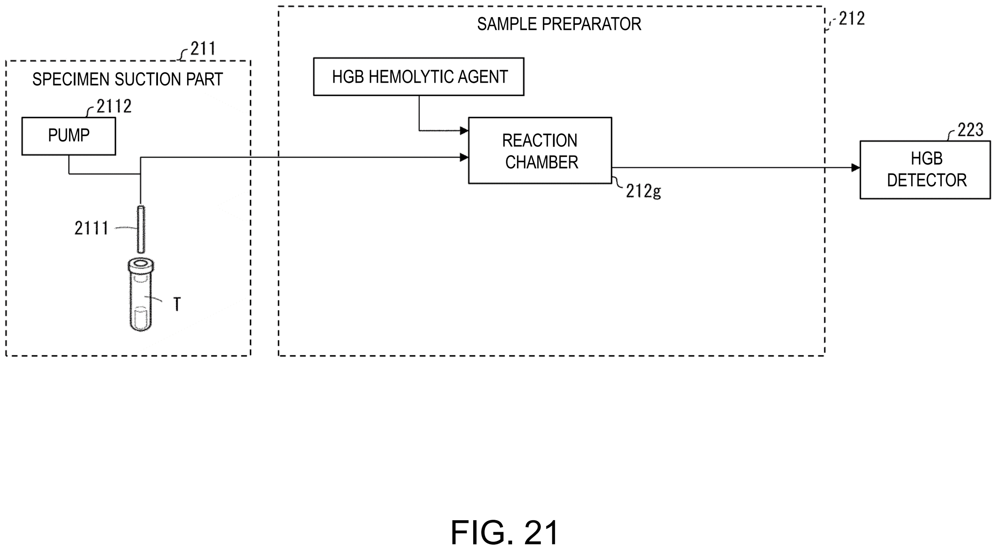

FIG. 21 is a schematic diagram for explaining the specimen suction part 211 and the sample preparator 212 when a measurement sample is supplied to the HGB detector 223. The specimen suction part 211 is the same as the specimen suction part 211 shown in FIG. 17. The sample preparator 212 includes a reaction chamber 212g. The reaction chamber 212g is connected to a hemolytic agent container containing a hemolytic agent being a reagent corresponding to HGB via a flow path. The measurement sample is prepared by mixing the blood specimen and the hemolytic agent in the reaction chamber 212g. The prepared measurement sample is supplied from the reaction chamber to the HGB detector 223 via a flow path.

### [Imaging Apparatus]

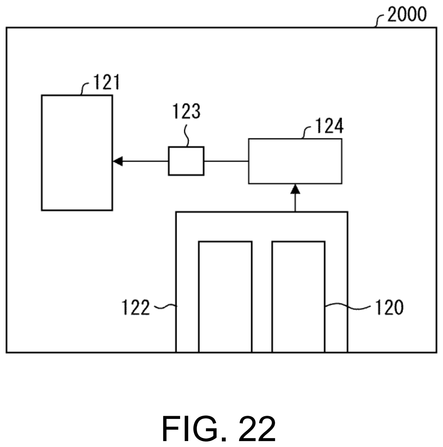

A configuration example of the imaging unit 2000 of the imaging apparatus 12 shown in FIG. 13 is shown in FIG. 22. As shown in FIG. 22, the imaging unit 2000 includes, for example, an imaging part 121, a transfer part 122, a conveyor 123, and an oil applicator 124. The imaging unit 2000 supplies immersion oil to a smear surface of a smear 120 conveyed from the conveyor 123 and images the smear 120 using an objective lens provided in the imaging part 121. The imaging unit 2000 captures blood cell images of the smear 120 by, for example, detecting positions of respective ones of a plurality of blood cells in the smear 120, adding immersion oil to the smear 120, and enlarging and capturing the plurality of cells in the smear. The imaging unit 2000 may capture an image of a predetermined area to acquire a wide-area image of the smear. For example, the imaging unit 2000 may acquire metadata from the blood cell images and classify the blood cells included in the blood cell images based on the metadata. The metadata includes, for example, flag information indicating that the blood cells included in the blood cell images are abnormal cells, and information indicating an attribute (for example, blood cell type) of the blood cells included in the blood cell images. A classification of the blood cell images may be performed automatically by the imaging apparatus 12 shown in FIG. 13, or may be performed automatically or manually by the application. The imaging apparatus 12 includes the control unit 7, and the control unit 7 may, for example, acquire data and perform communication with the data management system. The imaging apparatus 12 may be connected to a smear preparation apparatus and automatically perform a series of processes ranging from preparing a smear to classifying a plurality of blood cell images.

The smear is a specimen that has been stained for bright-field microscopy, for example, by May-Giemsa staining. The specimen is not limited as long as the morphology of each of the plurality of blood cells can be observed, and may be, for example, a smear prepared from peripheral blood.

### (Configuration Example of Interface)

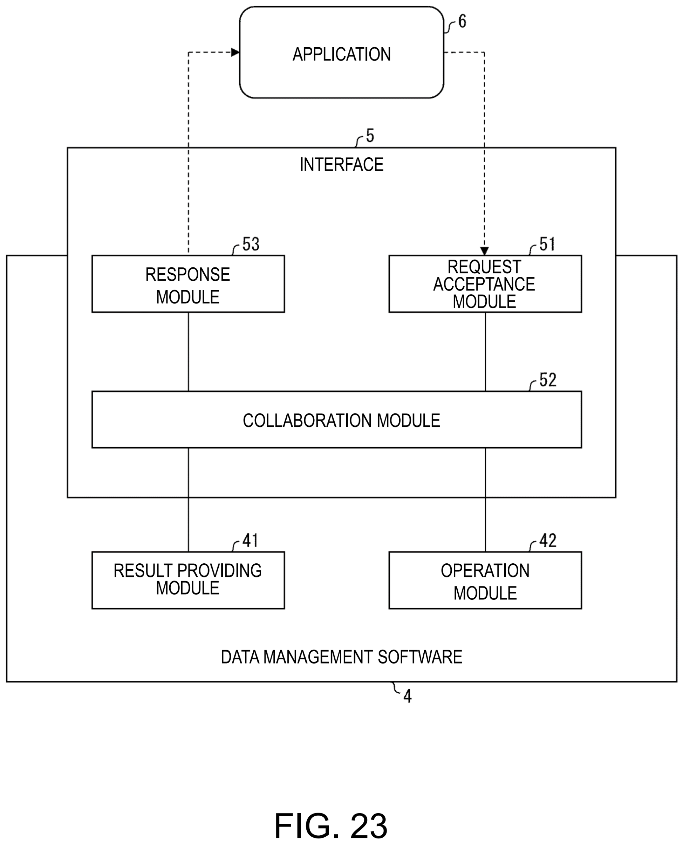

The interface 5 receives a request from the application 6, communicates with the data management software 4 (the result providing module 41 and the operation module 42), and performs a response corresponding to the request to the application 6. FIG. 23 shows a configuration example of the interface 5. The interface 5 includes, as an example, a request acceptance module 51, a collaboration module 52, and a response module 53. The request acceptance module 51 accepts a request from the application 6. The request acceptance module 51 analyzes the content of the request according to the rule R, and instructs the collaboration module 52 to communicate with the data management software 4 in order to acquire a response to the request. The collaboration module 52 communicates with the data management software 4 in accordance with the instruction, and acquires a response corresponding to the request. The response module 53 provides the response acquired by the collaboration module 52, to the application 6.

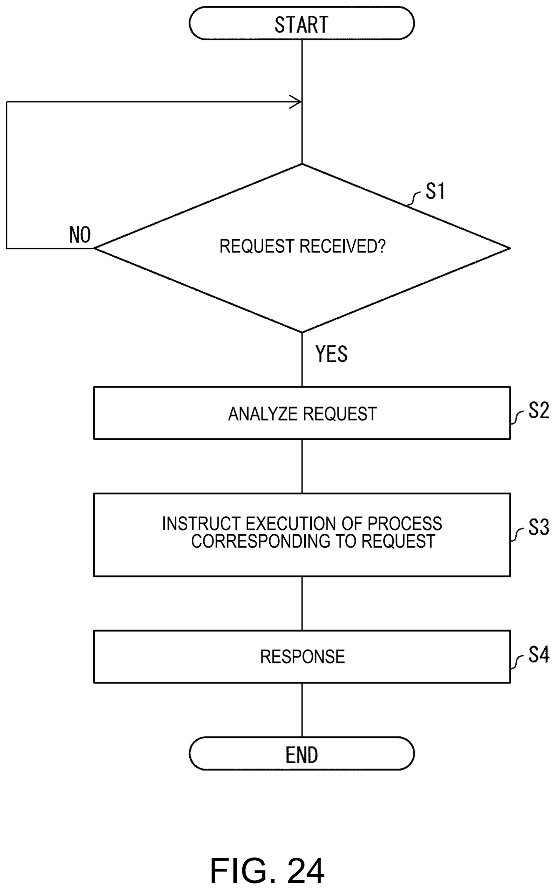

FIG. 24 is a flowchart showing an example of the flow (method of communication between the application and the interface) of the process performed by the interface 5 of the specimen measurement apparatus 11. First, the interface 5 waits for a request from the application 6 (S1). In response to receiving the request from the application 6 (Yes in S1), the interface 5 analyzes the content of the request according to the rule R (S2). The interface 5 instructs the data management software 4 to perform a process corresponding to the analyzed request (S3). The data management software 4 executes the process according to the instruction. For example, if the request instructs to acquire information regarding a measurement result, the result providing module 41 of the data management software 4 identifies the requested information and provides the identified information to the application 6 via the interface 5. The measurement result, for example, may be measurement data measured by the measurement unit 2 of a specific specimen measurement apparatus 11, a measurement results of a quality control substance in a time period (for example, the time period may be per day, per week, per month) designated by the request. The operation module 42 of the data management software 4 registers, for example, a measurement order in accordance with the request for registration of the measurement order from the application 6, and provides a response indicating that the registration has been completed, to the application 6 via the interface 5. In response to executing the process by the data management software 4, the interface 5 returns a response to the application 6 (S4). Thus, the interface 5 allows the application 6 to utilize the data and/or function corresponding to the request.

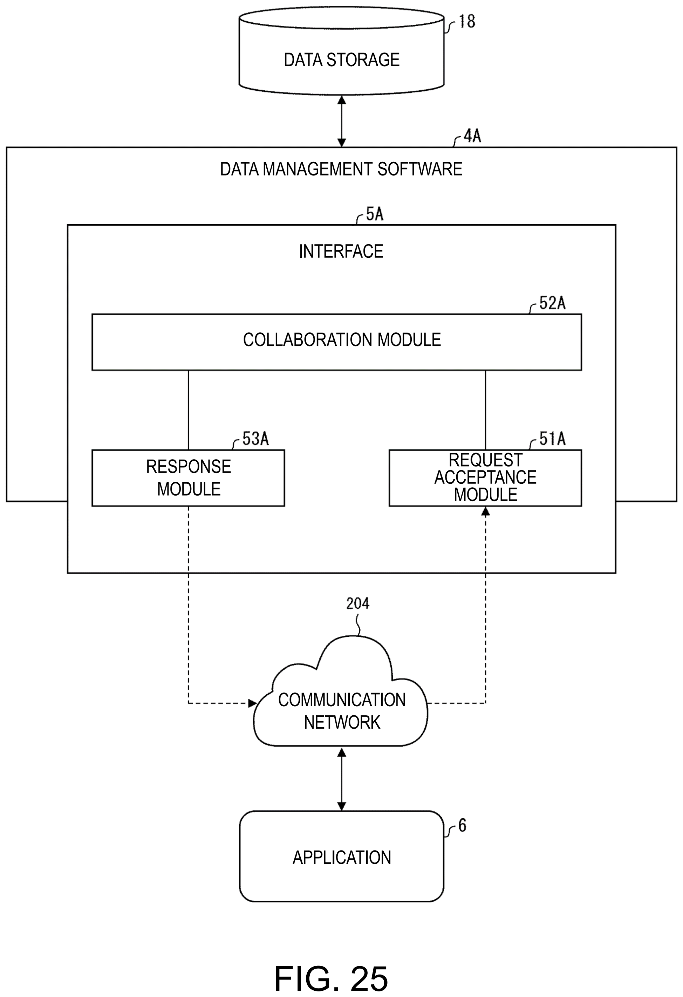

FIG. 25 shows a configuration example of the interface 5A of the data management system 15. The interface 5A includes, as an example, a request acceptance module 51A, a collaboration module 52A, and a response module 53A. The request acceptance module 51A accepts a request from the application 6 via the communication network 204. The request acceptance module 51A analyzes the content of the request, and instructs, on the basis of the analyzed request, the collaboration module 52A to link with the data management software 4A in order to acquire a response to the request. The collaboration module 52A communicates with the data management software 4A in accordance with the instruction and acquires a response corresponding to the request. The response module 53A provides the response acquired by the collaboration module 52A, to the application 6 via the communication network 204.

When the interface 5, 5A is configured as a Web API, the application 6 accesses the interface 5, 5A by using a URI defined according to the rule R. An example of the syntax of the URI defined according to the rule R is shown in Formula 1.$http : / / \left[server address\right] / \left[interface ID\right] / \left[apparatus ID\right] \left\{/\left[resource\right]/\left[query parameter\right]\right\}$

The "Server Address" in Formula 1 is a communication address corresponding to the interface 5, 5A. The server address is designated by, for example, an IP address and a port number.

A plurality of types of the interface 5/5A may be configured according to the type of the specimen measurement apparatus 11 and the imaging apparatus 12, for example. For example, the type of the interface 5/5A may correspond to the type of the specimen measurement apparatus 11. In this case, the "interface ID" in Formula 1 is designated in order to identify the interface 5, 5A corresponding to a certain type of the specimen measurement apparatus 11 and the imaging apparatus 12. The interface ID is, for example, an ID corresponding to the type of the specimen measurement apparatus 11. The designation of the "interface ID" in Formula 1 may not be mandatory, and if the "interface ID" is not designated, for example, a wildcard (blank, *, etc.) may be used. The interface ID may correspond to, for example, a type of the rule R. For example, there are a plurality of types of the rules R according to the types of the specimen measurement apparatuses 11 and the imaging apparatus 12. In other words, the interface ID corresponds to, for example, each the type of the specimen measurement apparatus 11 and the imaging apparatus 12. For example, a format of information management of the data management software 4 and the data management system 15 may differ depending on the type of the specimen measurement apparatus 11. In such a case, for example, by collaborating the interface 5/5A and the application 6 with each other according to a plurality of types of the rules R each corresponding to the formats of information management (e.g., each format corresponds to the type of the specimen measurement apparatus 11), the application 6 can utilize a plurality of types of information for which the formats of information management are respectively different.

The "apparatus ID" in Formula 1 is the ID of the specimen measurement apparatus 11 whose information and function are to be used by the application 6. The apparatus ID is, for example, the serial number of the specimen measurement apparatus 11 and imaging apparatus 12. Designation of "apparatus ID" in Formula 1 may not be mandatory. For example, if the "apparatus ID" is not designated, a wildcard (blank, *, etc.) is used.

The "resource" in Formula 1 is the resource of the API endpoint. The resource is, for example, the directory on a database corresponding to the information to be used by the application 6.

The "query parameter" in Formula 1 is a query, when a plurality of data and control subjects are present on the resource designated by the "resource" parameter, for uniquely identifying a certain data and a certain subject. For example, the "query parameter" is used for identifying a certain measurement data among a plurality of measurement data. To identify the certain measurement data, at least one condition (for example, specific time period, specific specimen, measurement data in which a measurement abnormality has occurred) may be designated as the query parameter. Designation of the "resource" in Formula 1 may not be mandatory, and if the "resource" is not designated, for example, a wildcard (blank, *, etc.) may be used. The designation formats of the "resource" and the "query parameter" may differ depending on, for example, the type of the specimen measurement apparatus 11. For example, with respect to acquisition of measurement data, the designation format of the "resource" and/or the "query parameter" for acquisition of the measurement data of the blood cell analysis apparatus, and the designation format of the "resource" and/or the "query parameter" for acquisition of the measurement data of an immunoassay apparatus may be different from each other. With respect to the measurement data, in accordance with the type of the specimen measurement apparatus 11, for example, the resource as the API endpoint may be different, or a specific query parameter may be required for each type of the specimen measurement apparatus 11 and the imaging apparatus 12. In such a case, the designation formats of the "resource" and the "query parameter" may be different depending on, for example, the type of the specimen measurement apparatus 11. The designation formats of the "resource" and the "query parameter" are a part of the rule R. Therefore, in other words, the "resource" and/or the "query parameter" may be designated on the basis of a plurality of types of the rules R according to the type of the specimen measurement apparatus 11.

Manipulation with respect to the resource designated by the URI is defined by, for example, an HTTP method. Examples of the HTTP method include methods such as "GET", "PUT", "DELETE", and "POST". Information designated by the "resource" and the "query parameter" of the URI is acquired by using the "GET" method.

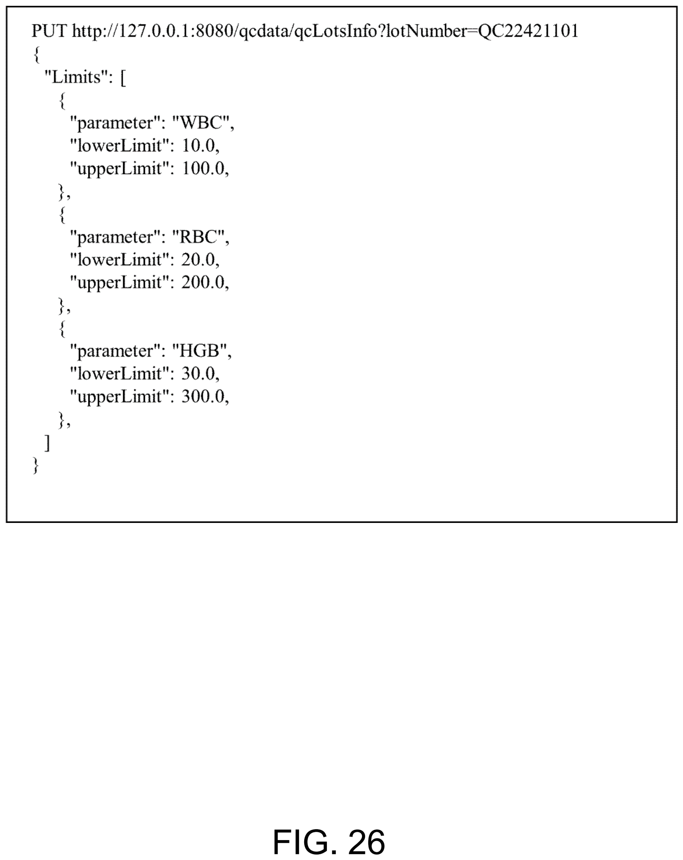

By use of the "PUT" method, information designated by the "resource" and the "query parameter" of the URI is updated or edited. In this case, new data (for example, the content of the resource to be updated or edited) is caused to be included in the HTTP request body. FIG. 26 shows an example of the data structure of the HTTP request body when lot information on quality control is updated. This example is described in the JSON (JavaScript Object Notation) format (JavaScript is a registered trademark). For example, the URI is designated as "http://127.0.0.1:8080/qcdata/qcLotsInfo?lotNumber=QC22421101". The "server address" of the control unit 7 to serve as the target of the "PUT" method is designated as "127.0.0.1". The "resource" for the QC (quality control) data held by the control unit 7 is designated as the "qcdata". A QC lot information is designated by the "query parameter". In this example, the "query parameter" is designated as "QC22421101" which represents a QC lot number. In this example, the URI is modified by the "PUT" method in order to update the QC data corresponding to the lot number "QC22421101". The contents (i.e., the QC data) to be updated is designated by HTTP request body. In this example, the HTTP request body is defined as "{"Limits":[{ "parameter": "WBC", "lowerLimit":10.0, "upperLimit":100.0},{"parameter": "RBC", "lowerLimit":20.0, "upperLimit":200.0},{"parameter": "HGB", "lowerLimit":30.0, "upperLimit":300.0},]}". With such a combination of the URI and the HTTP request body, update is performed with respect to the designated QC lot information, such that: as for WBC (white blood cell count), lower limit value is updated as 10.0, upper limit value is updated as 100.0; as for RBC (red blood cell count), lower limit value is updated as 20.0, upper limit value is updated as 200.0; and as for HGB (hemoglobin concentration), lower limit value is updated as 30.0, upper limit value is updated as 300.0. Here, since the lower limit value and the upper limit value of each of WBC (white blood cell count), RBC (red blood cell count), and HGB (hemoglobin concentration) are updated, QC can be performed by using the updated lower limit value and upper limit value. The lower limit value and the upper limit value as above are also referred to as management values. The management values are set for guaranteeing that the measurement result is accurate and for proving the validity of the measurement method and the management method. In addition, when the measurement result is outside the lower limit value or the upper limit value, the lower limit value or the upper limit value is also used for indicating that the measurement result is abnormal or that the measurement failed to be correctly performed.

By use of the "DELETE" method, information designated by the "resource" and the "query parameter" of the URI is deleted.

When new information is to be registered or created, the "POST" method is used. When the "POST" method is used, the "resource" and the "query parameter" may not be designated. In this case, new data (the content of the resource to be registered or created) is caused to be included in the HTTP request body.

The request acceptance module 51 of the interface 5 accepts, for example, the URI and the HTTP method from the application 6. The collaboration module 52 of the interface 5 communicates with the data management software 4 according to the URI and the HTTP method accepted by the request acceptance module 51, for example. For example, the collaboration module 52 communicates with the result providing module 41 in accordance with an acquisition request ("GET" of the HTTP method) for the measurement result in the specimen measurement apparatus 11 designated by the "apparatus ID" of the URI, and acquires corresponding measurement result. The response module 53 of the interface 5 transmits a response according to the received URI and HTTP method, to the application 6.

Similarly, the request acceptance module 51A of the interface 5A accepts, for example, the URI and the HTTP method from the application 6. The collaboration module 52A of the interface 5A communicates with the data management software 4A according to the URI and the HTTP method accepted by the request acceptance unit 51A, for example. For example, the collaboration module 52A communicates with the data management software 4A in accordance with an acquisition request ("GET" of the HTTP method) for the measurement result in the specimen measurement apparatus 11 designated by the "apparatus ID" of the URI, and acquires corresponding measurement result. The response module 53A of the interface 5A transmits a response according to the received URI and HTTP method, to the application 6.

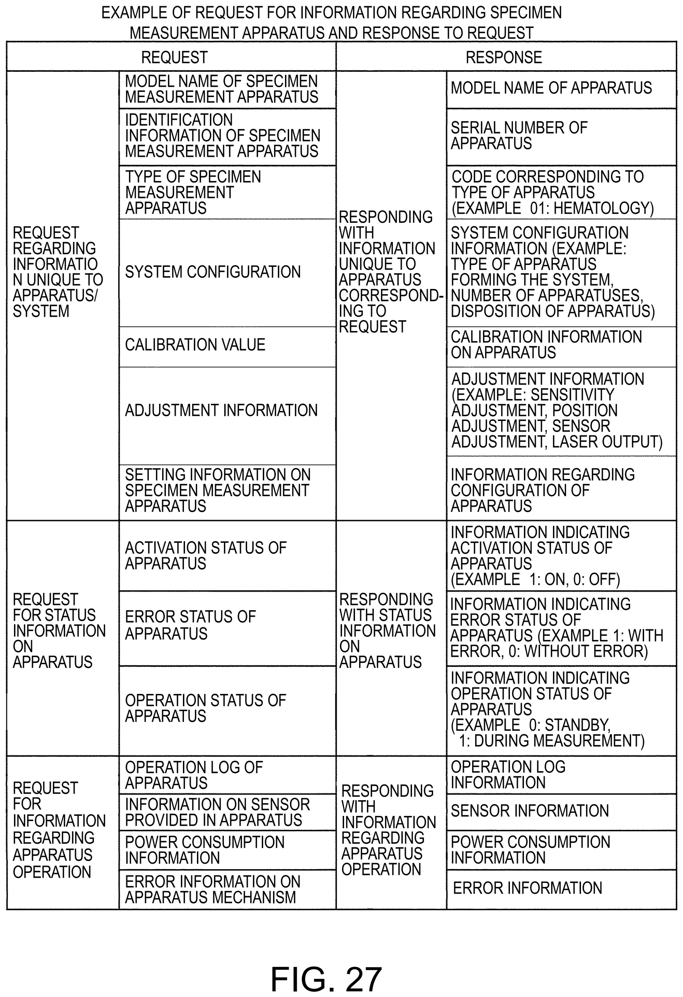

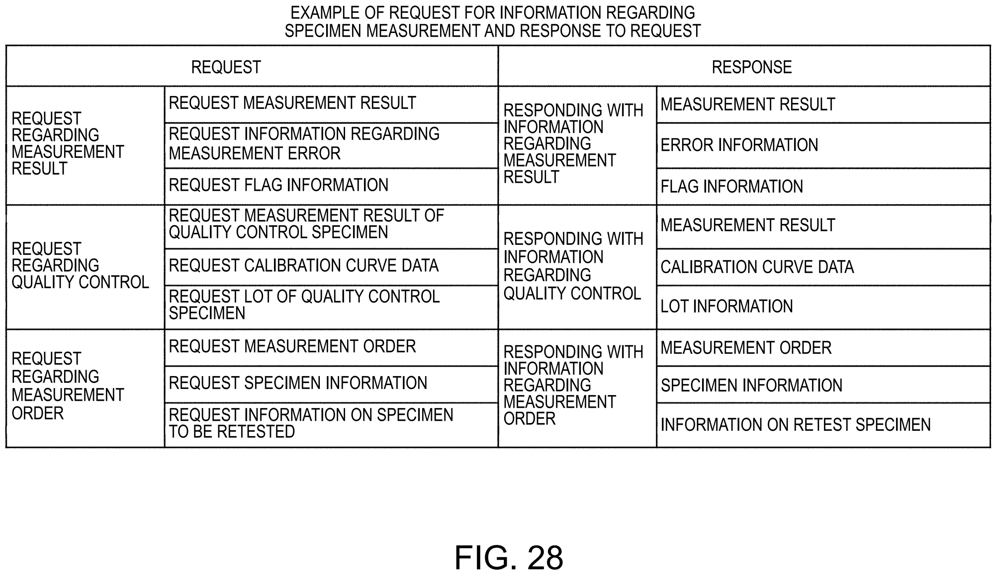

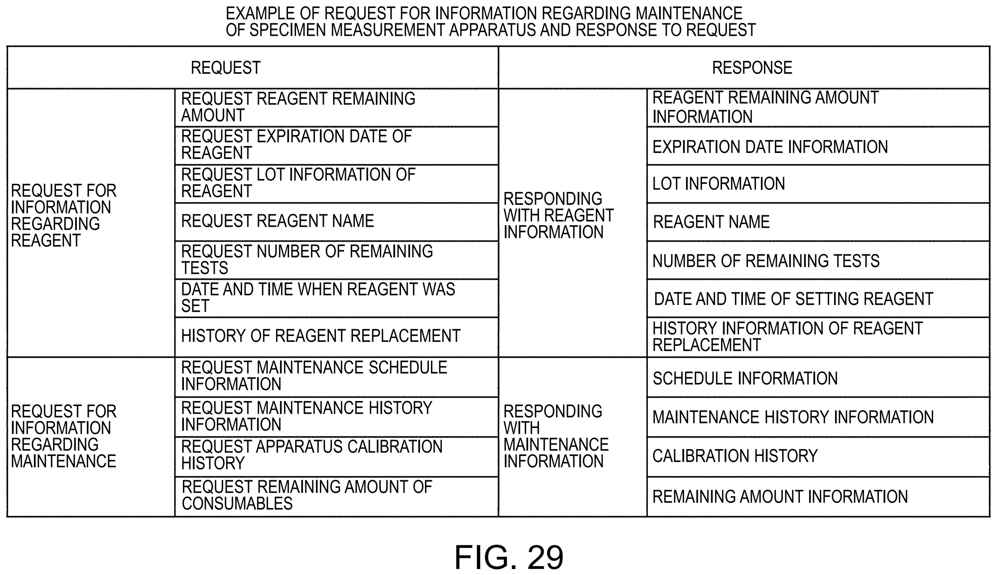

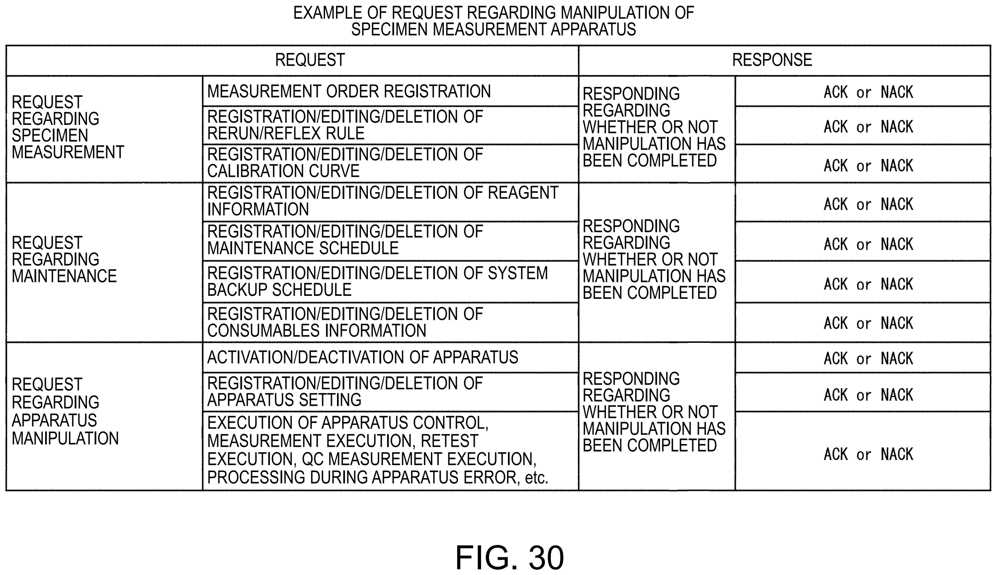

Examples of requests and responses to the interface 5, 5A of the specimen measurement apparatus 11 are shown in FIGS. 27 to 30. FIG. 27 shows examples of the request for information regarding the specimen measurement apparatus 11 / the response to the request. For example, FIG. 27 shows examples of: (1) the request for information unique to the specimen measurement apparatus 11 and the system including the specimen measurement apparatus 11 and a response thereto; (2) the request for information regarding the status of the specimen measurement apparatus 11 and the response thereto; and (3) the request for information regarding operation of the specimen measurement apparatus 11 and the response thereto. Examples of various types of information corresponding to the request and the response are shown in the table of FIG. 27. However, the various types of information corresponding to the request and the response are not limited to the examples shown in the table. The interface 5 can collectively provide, for example, information regarding a plurality of the specimen measurement apparatuses 11 (for example, serial numbers of a plurality of the specimen measurement apparatuses 11 placed in a laboratory, error statuses of the plurality of the specimen measurement apparatuses 11 placed in the laboratory, and the like), to the application 6. FIG. 28 shows examples of the request for information regarding specimen measurement / the response to the request. For example, FIG. 28 shows examples of: (1) the request for information regarding the measurement result and the response thereto; (2) the request regarding a quality control measurement and the response thereto; and (3) the request for information regarding the measurement order and the response thereto. Examples of various types of information corresponding to the request and the response are shown in the table of FIG. 28. However, the various types of information corresponding to the request and the response are not limited to the examples shown in the table. The interface 5 can collectively provide, for example, information regarding a plurality of the specimen measurement apparatuses 11 (for example, results of measurement of specimens performed in a predetermined period by a plurality of the specimen measurement apparatuses 11 placed in a laboratory, results of measurement of quality control specimens performed in a predetermined period by the plurality of the specimen measurement apparatuses 11 placed in the laboratory, and the like), to the application 6. FIG. 29 shows examples of the request for information regarding maintenance of the specimen measurement apparatus 11 / the response to the request. For example, FIG. 29 shows examples of: (1) the request for information regarding a reagent and the response thereto; and (2) the request for information regarding maintenance and the response thereto. Examples of various types of information corresponding to the request and the response are shown in the table of FIG. 29. However, the various types of information corresponding to the request and the response are not limited to the examples shown in the table. The consumables described in FIG. 29 are, for example, cuvettes to be used in measurement, washing liquids for washing flow paths in the apparatus, pipette tips, and the like. The interface 5 can collectively provide, for example, information regarding a plurality of the specimen measurement apparatuses 11 (for example, reagent remaining amount information for a plurality of the specimen measurement apparatuses 11 placed in a laboratory, maintenance schedule information for the plurality of the specimen measurement apparatuses 11 placed in the laboratory, and the like), to the application 6. FIG. 30 shows examples of the request regarding manipulation of the specimen measurement apparatus 11. For example, FIG. 30 shows examples of: (1) the request for manipulation regarding the specimen measurement and the response thereto; (2) the request for manipulation regarding maintenance and the response thereto; and (3) the request regarding manipulation of the specimen measurement apparatus 11 and the response thereto. Examples of various types of information corresponding to the request and the response are shown in the table of FIG. 30. However, the various types of information corresponding to the request and the response are not limited to the examples shown in the table. The interface 5 can cause, for example, the application 6 to collectively execute manipulation with respect to a plurality of the specimen measurement apparatuses 11 (for example, registration of a Rerun/Reflex rule with respect to a plurality of the specimen measurement apparatuses 11 placed in a laboratory, an activation/deactivation request for the plurality of the specimen measurement apparatuses 11 placed in the laboratory, and the like). In the example of FIG. 30, as the response to a manipulation request, for example, information (for example, ACK corresponding to completion of manipulation and NACK corresponding to incompletion manipulation (for example, error)) indicating whether or not manipulation has been completed is shown.

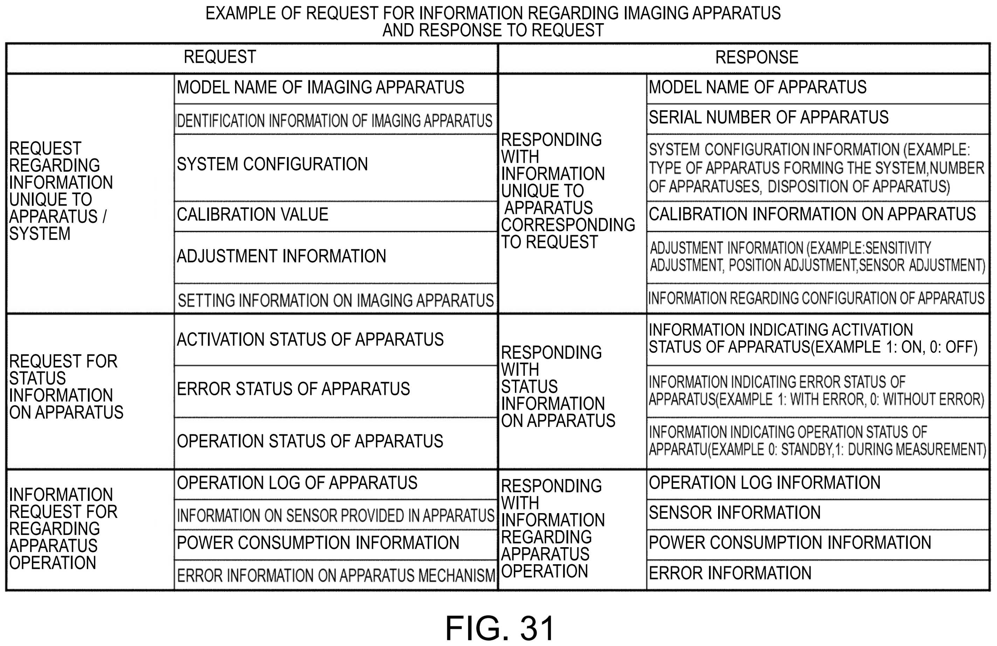

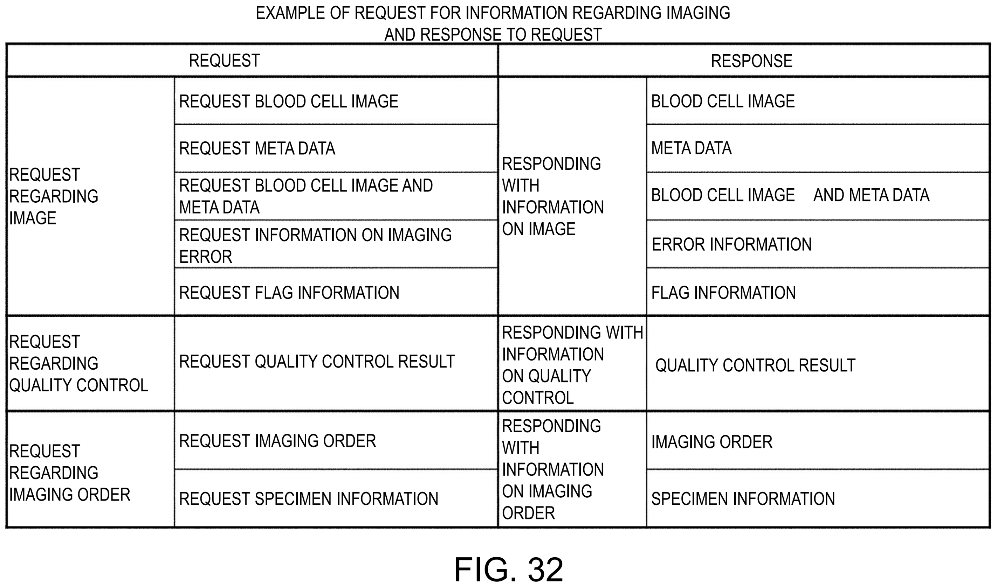

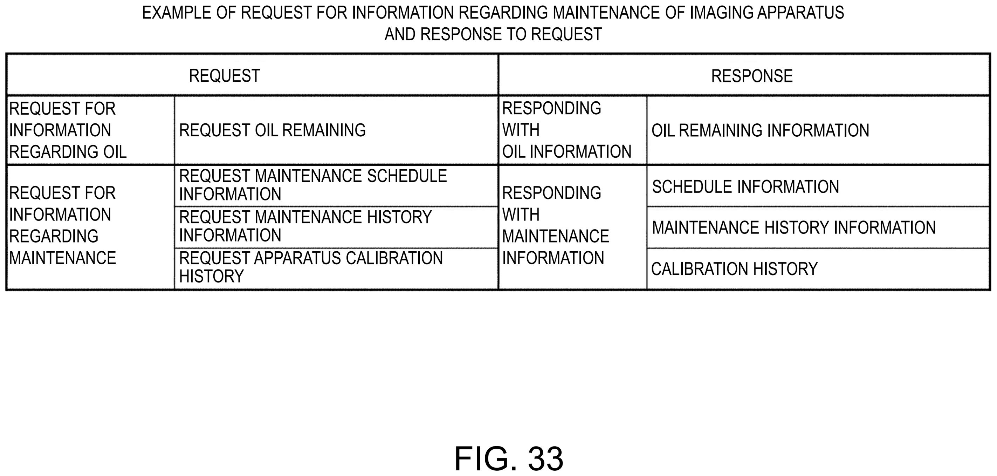

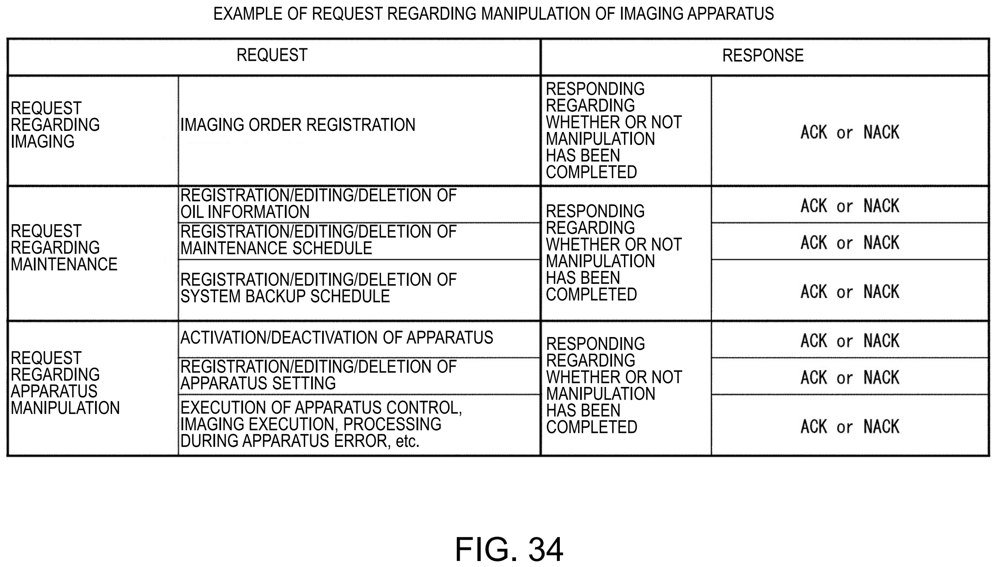

Examples of requests and responses to the interface 5, 5A of the imaging apparatus 12 are shown in FIGS. 31 to 34. FIG. 31 shows examples of the request for information regarding the imaging apparatus 12 / the response to the request. FIG. 31 shows examples of: (1) the request for information unique to the imaging apparatus 12 and the system including the imaging apparatus 12 and a response thereto; (2) the request for information regarding the status of the imaging apparatus 12 and a response thereto; and (3) the request for information regarding operation of the imaging apparatus 12 and the response thereto. Examples of various of information corresponding to the request and the response are shown in the table of FIG. 31. However, the various types of information corresponding to the request and the response are not limited to the examples shown in the table. The interface 5 can collectively provide, for example, information regarding a plurality of imaging apparatuses 12 (for example, serial numbers of a plurality of the imaging apparatuses 12 placed in a laboratory, error statuses of the plurality of imaging apparatuses 12 placed in the laboratory, and the like), to the application 6. FIG. 32 shows an example of the request for information regarding imaging and the response to the request. FIG. 32 shows examples of: (1) the request for information regarding the image and a response thereto; (2) the request regarding quality control and the response thereto; and (3) the request for information regarding an imaging order and the response thereto. Examples of various types of information corresponding to the request and the response are shown in the table of FIG. 32. However, the various types of information corresponding to the request and the response are not limited to the examples shown in the table. The interface 5 can collectively provide, for example, information regarding the image (for example, blood cell images, metadata associated with blood cell images, and the like), to the application 6. The information regarding the image is, for example, associated with identification information related to a specimen (for example, specimen ID). For example, based on the identification information related to the specimen, the application 6 can request information regarding the image from the interface 5. The interface 5 can provide the application 6 with information regarding the image including blood cell images and metadata. The metadata includes, for example, flag information indicating that a blood cell in a blood cell image is likely an abnormal blood cell, and information indicating an attribute (for example, blood cell type) of the blood cell in the blood cell image. For example, based on the metadata, the application 6 can generate, for each specimen, classification result (summary) in which the blood cell images are classified according to attributes of the blood cells. For example, the interface 5 can provide the application 6 with information regarding imaging orders (for example, a list of imaging orders, whether an imaging order exists, a list of specimen IDs associated with the imaging orders, etc.). For example, based on the information regarding the imaging order, the interface 5 can extract identification information related to the specimen for which an imaging order has been issued, and collectively provide the application 6 with information regarding the imaging data associated with the extracted identification information related to the specimen. For example, the interface 5 can collectively provide the application 6 with information regarding a plurality of imaging apparatuses 12 (for example, imaging results of specimens acquired by the plurality of imaging apparatuses 12 placed in a laboratory during a predetermined period, quality control results acquired by the plurality of imaging apparatuses 12 placed in the laboratory during a predetermined period, etc.). Quality control in the imaging apparatus 12 is performed to confirm whether the imaging apparatus 12 can correctly detect cells in the smear of the specimen. For example, a predetermined number of images of the smear prepared by smearing a blood specimen on a glass slide may be captured by the imaging apparatus 12, and whether the blood cells in the smear are correctly detected can be used as an index for quality control. FIG. 33 shows an example of the request for information regarding maintenance of the imaging apparatus 12 / the response to the request. FIG. 33 shows examples of: (1) the request for information regarding oil and the response thereto; and (2) the request for information regarding maintenance and the response thereto. Examples of various information corresponding to the request and the response are shown in the table of. FIG. 33. However, the various types of information corresponding to the request and the response are not limited to the examples shown in the table. For example, the interface 5 can collectively provide the application 6 with information regarding the plurality of imaging apparatuses 12 (for example, information on the remaining oil levels of the plurality of imaging apparatuses 12 placed in a laboratory, maintenance-schedule information of the plurality of imaging apparatuses 12 placed in the laboratory, etc.). FIG. 34 shows an example of the request regarding manipulation of the imaging apparatus 12. FIG. 34 shows examples of: (1) the request for manipulation regarding capturing and the response thereto; (2) the request for manipulation regarding maintenance and the response thereto; and (3) the request regarding manipulation of the imaging apparatus 12 and the response thereto. Examples of various types of information corresponding to the request and the response are shown in the table of FIG. 34. However, the various types of information corresponding to the request and the response are not limited to the examples shown in the table. The interface 5 can cause, for example, the application 6 to collectively execute manipulation with respect to a plurality of the imaging apparatuses 12 (e.g., start/end requests for the plurality of imaging apparatuses 12 placed in a laboratory, etc.). In the example of FIG. 34, as the response to a manipulation request, for example, information (for example, ACK corresponding to completion of manipulation and NACK corresponding to incompletion manipulation (for example, error)) indicating whether or not manipulation has been completed is shown.

### (PUSH notification)

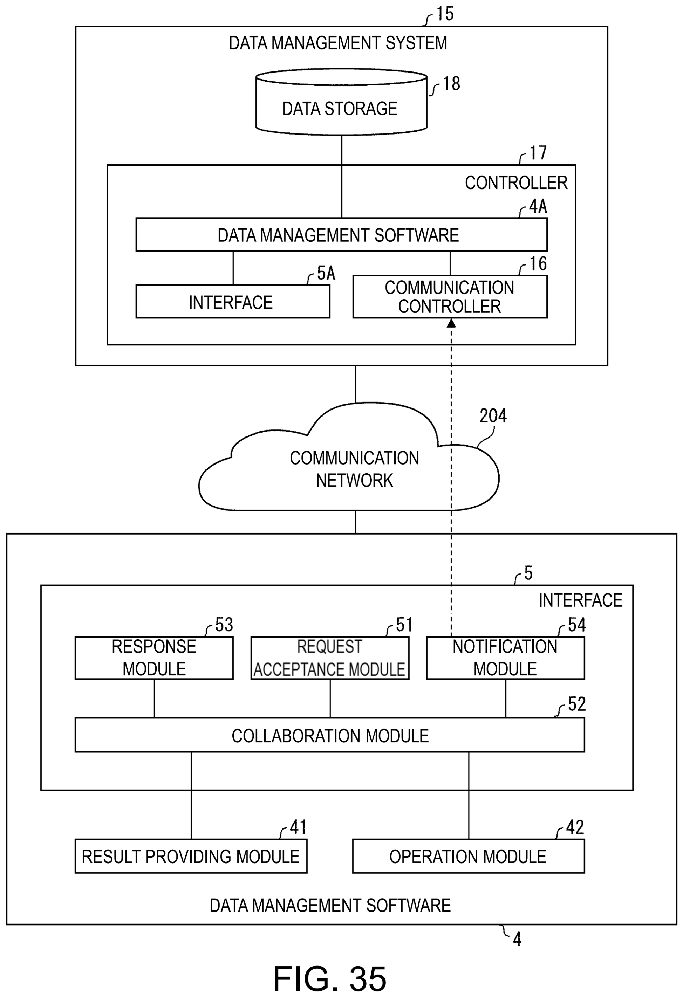

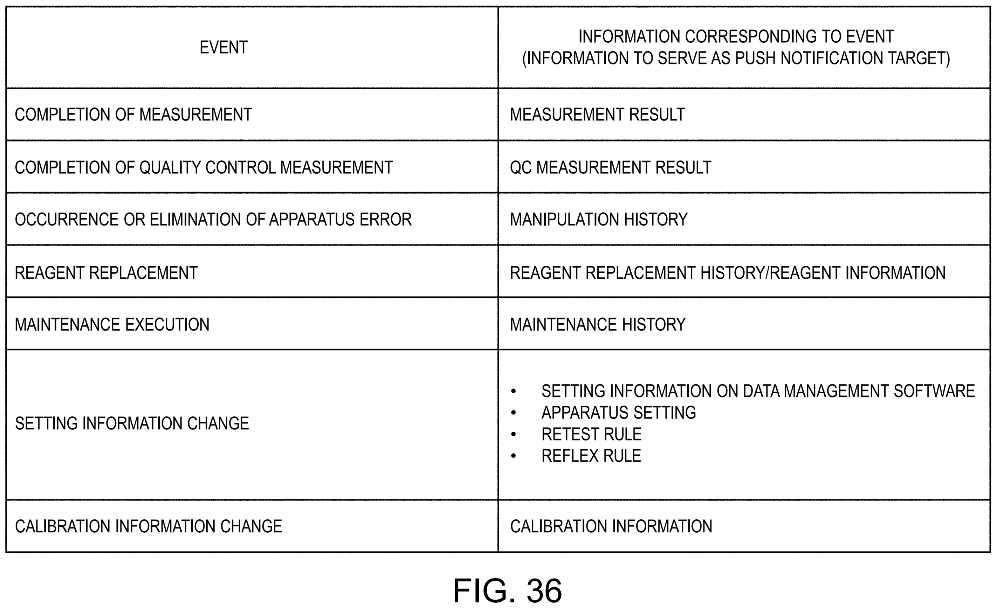

FIG. 35 shows a configuration example of the PUSH notification from the data management software 4 of the specimen measurement apparatus 11 and the imaging apparatus 12 to the data management system 15. The interface 5 may include a notification module 54 including a function of performing the PUSH notification. For example, at the time of occurrence of a predetermined event, the notification module 54 provides information corresponding to the event, to the data management system 15 by using the PUSH notification via the communication network 204. FIG. 36 shows examples of events and examples of information (information to serve as the subject of the PUSH notification) corresponding to the event. For example, upon acquisition of the measurement result of the specimen by the specimen measurement apparatus 11, the notification module 54 provides the measurement result to the data management system 15. For example, upon execution of predetermined manipulation (for example, reagent replacement, maintenance, information setting, and the like) on the specimen measurement apparatus 11, the notification module 54 provides information related to the manipulation, to the data management system 15. For example, in accordance with change (occurrence of an error, elimination of the error, and the like) in the status of the specimen measurement apparatus 11, the notification module 54 provides information (for example, error information, manipulation history regarding the error, and the like) related to the change in the status, to the data management system 15. As another example, upon acquisition of the image of the blood cells in the specimen captured by the imaging apparatus 12, the notification module 54 provides the image to the data management system 15. For example, upon execution of predetermined manipulation (for example, oil replacement, maintenance, information setting, and the like) on the imaging apparatus 12, the notification module 54 provides information related to the manipulation, to the data management system 15. For example, in accordance with change (occurrence of an error, elimination of the error, and the like) in the status of the imaging apparatus 12, the notification module 54 provides information (for example, error information, manipulation history regarding the error, and the like) related to the change in the status, to the data management system 15.

The notification module 54 may be provided to the data management software 4 instead of the interface 5. Alternatively, the notification module 54 may be provided to the control unit 7 as software independent of the interface 5 and the data management software 4.

### (Application)

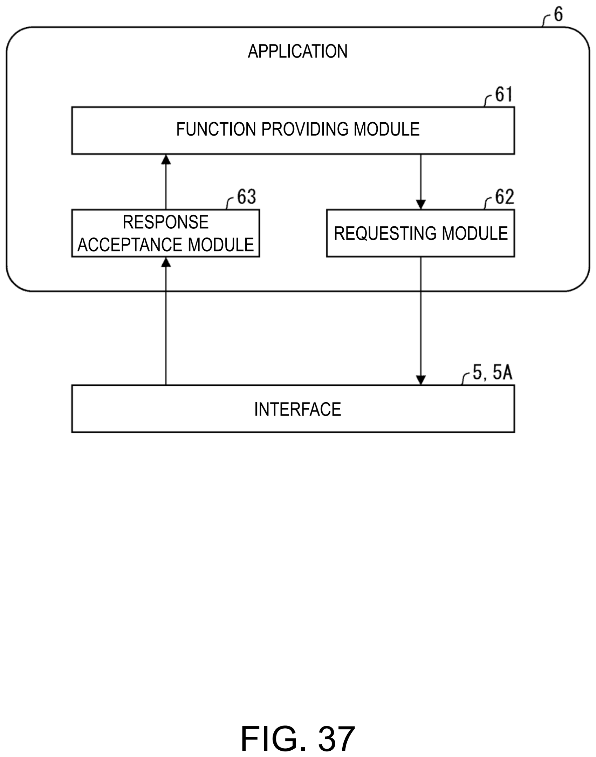

FIG. 37 shows a configuration example of the application 6. The application 6 includes, for example, a function providing module 61, a requesting module 62, and a response acceptance module 63.

The function providing module 61 provides a function for expanding the specimen measurement system 1. The function providing module 61 provides a function according to the type of the application 6. For example, according to the type of the application 6, the function providing module 61 provides a function of providing a QC result (QC chart and the like), a function of managing a reagent placed in the specimen measurement apparatus 11, a function regarding managing of an error having occurred in the specimen measurement apparatus 11, a function regarding maintenance of the specimen measurement apparatus 11, a function regarding operation management of the specimen measurement apparatus 11, and the like. For example, according to the type of the application 6, the function providing module 61 provides a function displaying an image of a blood cell, a function displaying the plurality of candidates of the specific blood cell, a function displaying a measurement result by the specimen measurement apparatus 11, and the like.

The function providing module 61 may have a function of allowing the user to log-in by using an account of a service provider of the function provided by the application 6. In this case, only when log-in has been established using the account of the service provider, the application 6 provides the function by the function providing module 61.

For example, when information regarding the specimen measurement apparatus 11 and the imaging apparatus 12 is necessary, the function providing module 61 requests the requesting module 62 to acquire the information. The requesting module 62 requests information to the interface 5, 5A in accordance with the request from the function providing module 61. For example, as described above, the requesting module 62 requests the information by a command C defined by a combination of the URI and the HTTP method. In accordance with operation of the function providing module 61, for example, when control of the apparatus 11 (for example, registration/change of configuration of the specimen measurement apparatus 11) become necessary, the function providing module 61 makes the request (for example, the request for registration/change of the configuration) regarding the control of the apparatus 11 to the requesting module 62. Furthermore, when control of the imaging apparatus 12 (for example, registration/change of configuration of the imaging apparatus 12, etc.) is needed according to the operation of the function providing module 61, the function providing module 61 makes a request regarding the control (for example, the request for registration/change of the configuration) to the requesting module 62. The requesting module 62 makes the request regarding the control of the apparatus 11 to the interface 5, 5A. For example, as described above, the requesting module 62 makes the request regarding the control of the apparatus 11 by the command C defined by the combination of the URI and the HTTP method. For example, the requesting module 62 generates the command C including the URI on the basis of information provided by the function providing module 61, and transmits the request to the interface 5/5A. For example, when requesting information regarding the specimen measurement apparatus 11, the function providing module 61 notifies the requesting module 62 of an instruction (for example, result of measurement in a predetermined period performed by a certain specimen measurement apparatus 11 with the apparatus ID) regarding the information is required. For example, when requesting information regarding the imaging apparatus 12, the function providing module 61 notifies the requesting module 62 of an instruction regarding the requested information (for example, an image in a predetermined period captured by a certain imaging apparatus 12 with the apparatus ID). The requesting module 62 generates the command C according to the notified instruction. The instruction from the function providing module 61 to the requesting module 62 is notified, for example, according to manipulation of the application 6 by the user. For example, when the user has requested the measurement result acquired on the specific day by a certain specimen measurement apparatus 11, the function providing module 61 notifies the requesting module 62 of the instruction corresponding to the request. For example, when a user requests an image captured by a certain imaging apparatus 12 with the apparatus ID on a specific date, the function providing module 61 notifies the requesting module 62 of an instruction corresponding to the request. When generating the command C, the requesting module 62 generates the command C with reference to, for example, configuration information (for example, IP address of the request destination server) regarding creation of the URI. As the configuration information, the IP address itself of the request destination server may be set, or the URL (Uniform Resource Locator) of the server may be set. The IP address of the request destination server is, for example, the IP address of the control unit 7 of the specimen measurement apparatus 11 and the imaging apparatus 12 set in the laboratory, or the IP address of the data management system 15. When the address of the request destination server is set by the URL, the requesting module 62 inquires the DNS (Domain Name System) about the IP address on the basis of the domain name included in the URL, for example. The requesting module 62 generates the URI on the basis of the IP address notified from the DNS. The IP address or the URL may be input by the user via a GUI of the application 6. The process of generation of the URI or the command C by the requesting module 62 is the same in the examples of the application described later.

The response acceptance module 63 accepts, from the interface 5, 5A, the response corresponding to the request by the requesting module 62, and passes the response content to the function providing module 61.

### [Example of Application]

An example is described in which the application 6 is the application that displays a plurality of candidates indicating classification for a specific blood cell in blood cell images that visually demonstrate morphological features of blood cells, and the blood cell images. The application provides, for example, a function of collecting the blood cell images of a specimen and metadata including information indicating a cell type of the blood cell in the blood cell images from the imaging apparatus 12, and displaying, based on the metadata, the plurality of candidates for the specific blood cell. The application provides, for example, a function of displaying the blood cell image A11 and the plurality of candidates A111 for the specific blood cell in the blood cell image A11 as shown in FIG. 45.

Referring again to FIG. 37, each part of the application is described. The function providing module 61 requests the requesting module 62 to acquire information regarding the imaging apparatus 12 that is a management target for the data in order to display the blood cell image and the plurality of candidates of the specific blood cell in the blood cell image. For example, the function providing module 61 requests the requesting module 62 to acquire metadata including information indicating a cell type in a blood cell image of a specimen acquired by the imaging apparatus 12, information regarding the plurality of candidates of the blood specific cell based on the metadata, the blood cell image of the specimen, attribute information regarding a subject, and the like. When there are a plurality of imaging apparatuses 12, the function providing module 61 may request the requesting module 62 to acquire, for example, the apparatus IDs and the names of the plurality of imaging apparatuses 12.

The requesting module 62, for example, generates a URI based on the request content from the function providing module 61, and requests information acquisition to the interface 5, 5A with a command C generated by combining the generated URI and "GET" of the HTTP method. The URI generated by the requesting module 62 is, for example, "http://128.0.0.1:8080/DI//YYYY" when acquiring information regarding the imaging apparatus 12. In this example of the URI, the "server address" is designated as "128.0.0.1:8080", the "interface ID" is designated as "DI", the "apparatus ID" is not designated, the "resource" is designated as "YYYY", and the "query parameter" is not designated. "YYYY" is an API endpoint corresponding to information (for example, information regarding the type of the imaging apparatus 12, imaging data of a patient captured by the imaging apparatus 12, and the like) regarding the imaging apparatus 12. "128.0.0.1:8080" of the "server address" is, for example, an address corresponding to the interface of the data management system that manages information regarding the imaging apparatus 12. When there are a plurality of "server addresses", a plurality of URIs (each of the plurality of URIs corresponds to each of a plurality of addresses) may be generated. "DI" of the "interface ID" is set, for example, according to the type of the imaging apparatus 12 which is the target of information acquisition. URIs may be generated in a plural number according to the kind of information that is requested. The response acceptance module 63 accepts a response corresponding to the request from the requesting module 62, and passes the accepted information to the function providing module 61. For example, the requesting module 62 generates a URI in response to the request, and requests information acquisition to the interface 5, 5A and the interface of the data management software that manages information regarding the imaging apparatus 12 with a command C generated by combining the generated URI and "GET" of the HTTP method. When the imaging apparatus 12 is not provided with an interface, the data is transmitted from the imaging apparatus 12 not provided with an interface to the data storage 18 of the data management system 15 via the interface 5 of the data management system 15. For example, the data is transmitted from the imaging apparatus 12 to the data storage 18 of the data management system 15 via the interface 5 of the data management system 15. In this case, an application regarding data transmission is added to the imaging apparatus 12. The added application generates a URI and transmits the data to the interface 5 of the data management system 15 with a command C generated by combining the generated URI and "POST" or "PUT" of the HTTP method. The data transmitted to the data management system 15 can be acquired by the application. Therefore, the requesting module 62 generates a URI based on the request content from the function providing module 61, and requests transmission of the data to the interface 5 of the data management system 15 with a command C generated by combining the generated URI and "GET" of the HTTP method.

The function providing module 61, for example, compares a rule regarding the plurality of candidates of the specific blood cell with metadata to determine whether to generate the plurality of candidates of the specific blood cell. For example, the function providing module 61 generates the plurality of candidates of the specific blood cell upon determining that the meta data satisfies the rule regarding the plurality of candidates. The metadata includes information indicating a cell type in a blood cell image of a specimen. The function providing unit 61 determines that the rule regarding the plurality of candidates is satisfied, for example, when the metadata indicates that there are the plurality of candidates of the specific blood cell (for example, BL (blast), BA (basophil), VLY (variant lymphocyte)) in the blood cell image of the specimen exists. The metadata including the information indicating that there are the plurality of candidates of the specific blood cell may be, for example, a summary such as a classification result or a classification probability classified by image analysis on the blood cell image, or may be information calculated from a predetermined classification criterion based on past image data classified by image analysis on the blood cell image. Further, the metadata including the information indicating that there are the plurality of candidates of the blood cell may be, for example, information generated by processing using an artificial intelligence algorithm on the blood cell image. The function providing module 61 displays the blood cell image and the plurality of candidates of the specific blood cell according to a determination result.

A function to display the blood cell image and the plurality of candidates of the specific blood cell in the blood cell image can be extended by the application 6. In order to distinguish and utilize data for each specimen by the application 6, for example, metadata may be associated with identification information (for example, specimen ID) related to the specimen.

FIG. 45 shows an example 1 of providing a GUI for displaying the blood cell image and the plurality of candidates of the specific blood cell in the blood cell image by the function providing module 61. The function providing module 61 acquires, for example, a plurality of blood cell images associated with the identification information A4 of the specimen. The function providing module 61 displays, for example, a list A1 of the blood cell images associated with the identification information A4 of the specimen. The function providing module 61 groups and displays the blood cell images, for example, according to the cell classification type (BL (blast), MY (myelocyte), MMY (metamyelocyte)) in the blood cell images. The function providing module 61 recognizes the classification type based on the metadata of each of the blood cell images, and groups the blood cell images, for example. The function providing module 61 displays the plurality of candidates A111 of the specific blood cell, for example, based on the metadata corresponding to a specific blood cell image associated with the identification information A4 of the specimen.

### [Modification of Application]

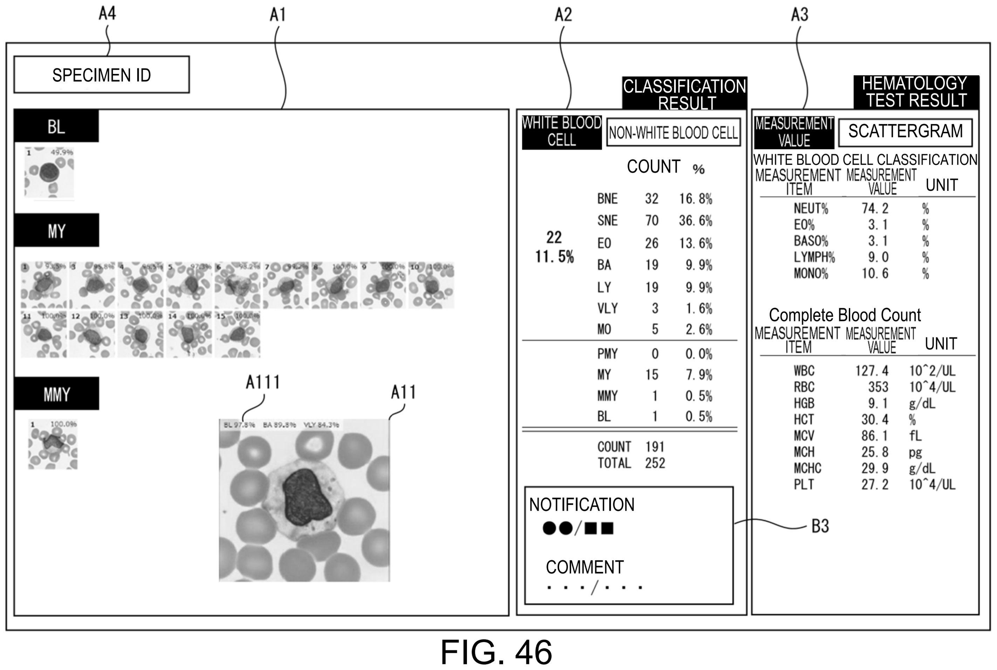

A modification is described in which the application 6 is an application that further displays a summary based on metadata including information representing numerically and/or linguistically morphological features of the blood cells in the blood cell images of the specimen, a notification indicating, based on the metadata, a possibility of presence of a specific blood cell in the blood cell images, and a hematology test result. The application of the modification provides, for example, a function of displaying the summary A2 and the notification B3 in addition to the blood cell images A1 and A11 and the plurality of candidates A111 of the specific blood cell in the blood cell image, and further displaying the hematology test result A3 acquired by the specimen measurement apparatus 11 as shown in FIG. 46.

Referring again to FIG. 37, each part of the application that further displays the summary, the notification, and the hematology test result is be described. The function providing module 61 requests the requesting module 62 to acquire information regarding each of the specimen measurement apparatus 11 and the imaging apparatus 12 that are management targets for the data to be integratedly displayed. For example, the function providing module 61 requests the requesting module 62 to acquire the hematology test result acquired by the specimen measurement apparatus 11, the result of capturing the image of the blood cells acquired by the imaging apparatus 12 (for example, a plurality of blood cell images and metadata which is additional information associated with each of the plurality of blood cell images), attribute information of the subject, and the like. When there are a plurality of specimen measurement apparatuses 11 and imaging apparatuses 12, the function providing module 61 may request the requesting module 62 to acquire, for example, the apparatus IDs of each of the plurality of specimen measurement apparatuses 11 and imaging apparatuses 12, and the names of each of the plurality of specimen measurement apparatuses 11 and imaging apparatuses 12.

The requesting module 62, for example, generates a URI based on the request content from the function providing module 61, and requests information acquisition to the interface 5, 5A with a command C generated by combining the generated URI and "GET" of the HTTP method. The URI generated by the requesting module 62 is, for example, "http://127.0.0.1:8080/XR//XXXX" when acquiring information regarding the specimen measurement apparatus 11. In this example of the URI, the "server address" is designated as "127.0.0.1:8080", the "interface ID" is designated as "XR", the "apparatus ID" is not designated, the "resource" is designated as "XXXX", and the "query parameter" is not designated. "XXXX" is an API endpoint corresponding to information (for example, information regarding the type of the specimen measurement apparatus 11, measurement data of a patient acquired by the specimen measurement apparatus 11, and the like) regarding the specimen measurement apparatus 11. "127.0.0.1:8080" of the "server address" is, for example, an address corresponding to the interface 5A of the data management system 15 that manages information regarding the specimen measurement apparatus 11. When there are a plurality of "server addresses", a plurality of URIs (each of the plurality of URIs corresponds to each of a plurality of addresses) may be generated. "XR" of the "interface ID" is set, for example, according to the type of the specimen measurement apparatus 11 which is the target of information acquisition. URIs may be generated in a plural number according to the kind of information that is requested. Similarly, the URI generated by the requesting module 62 is, for example, "http://128.0.0.1:8080/DI//YYYY" when acquiring information regarding the imaging apparatus 12. In this example of the URI, the "server address" is designated as "128.0.0.1:8080", the "interface ID" is designated as "DI", the "apparatus ID" is not designated, the "resource" is designated as "YYYY", and the "query parameter" is not designated. "YYYY" is an API endpoint corresponding to information (for example, information regarding the type of the imaging apparatus 12, imaging data of a patient captured by the imaging apparatus 12, and the like) regarding the imaging apparatus 12. "128.0.0.1:8080" of the "server address" is, for example, an address corresponding to the interface of the data management system that manages information regarding the imaging apparatus 12. When there are a plurality of "server addresses", a plurality of URIs (each of the plurality of URIs corresponds to each of a plurality of addresses) may be generated. "DI" of the "interface ID" is set, for example, according to the type of the imaging apparatus 12 which is the target of information acquisition. URIs may be generated in a plural number according to the kind of information that is requested. The response acceptance module 63 accepts a response corresponding to the request from the requesting module 62, and passes the accepted information to the function providing module 61. For example, the requesting module 62 generates a URI in response to the request, and requests information acquisition to the interface 5 and/or 5A with a command C generated by combining the generated URI and "GET" of the HTTP method.

The function providing module 61 provides a function of displaying the blood cell image, the plurality of candidates of the specific blood cell in the blood cell image, the summary, and the notification, and further displaying the hematology test result.

FIG. 46 shows an example 2 of providing a GUI by the function providing module 61. The example 2 of providing the GUI is an example of integratedly displaying the summary, the notification, and the hematology test result. The function providing module 61 acquires, for example, a plurality of blood cell images associated with the identification information A4 of the specimen. The function providing module 61 displays, for example, a list A1 of the blood cell images associated with the identification information A4 of the specimen. The function providing module 61 groups and displays the blood cell images, for example, according to the cell classification type (BL (Blast), MY (Myelocyte), MMY (Metamyelocyte)) in the blood cell images. The function providing module 61 recognizes the classification type based on the metadata of each of the blood cell images, and groups the blood cell images, for example. The function providing module 61 displays the summary A2, for example, based on the metadata corresponding to each of the plurality of blood cell images associated with the identification information A4 of the specimen. The function providing module 61 displays, for example, the count and ratio of the blood cell images classified into each of the plurality of classification types as the summary A2, based on the classification types included in the metadata. The function providing module 61 displays the notification flag B3 in addition to the summary A2. The function providing module 61 acquires, for example, the hematology test result A3 associated with the identification information A4 of the specimen, and displays, in an integrated manner, the hematology test result A3, the list A1 of the blood cell images, the summary A2, and the notification B3. The hematology test result A3 includes, for example, the result of CBC (Complete Blood Count) and the result regarding white blood cell classification.

A function of the system can be extended by adding the application 6 to the system, to display the GUI exemplified in FIG. 46. In order to utilize data in this system, it is necessary to distinguish and utilize the data for each specimen, and for example, each of the metadata of the blood cell images and the hematology test result may be associated with the identification information (for example, specimen ID) of the specimen. In this case, the blood cell image, the plurality of candidates of the specific blood cell in the blood cell image, the summary, the notification, and the hematology test result that are associated with the identification information of the same specimen can be displayed.

### (Example of Data Structure in Data Storage)

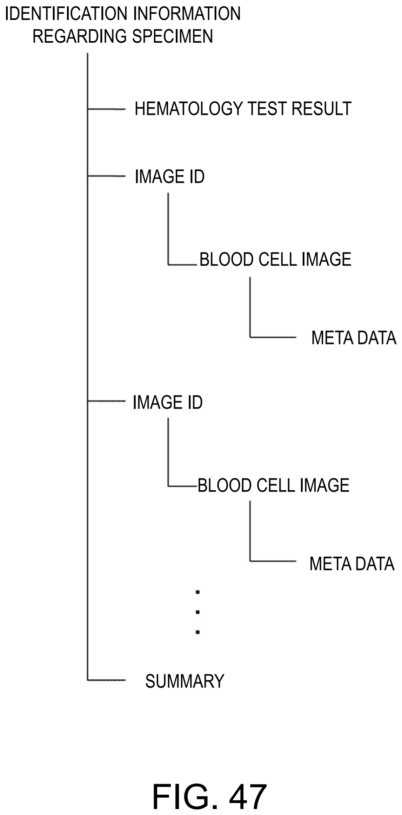

FIG. 47 is a diagram showing an example of a data structure when the data storage 18 or the storage 74 is a relational database. As shown in the FIG. 47, the information managed by the data storage 18 or the storage 74 includes, for example, data in which each of the ""hematology test result"" acquired by the specimen measurement apparatus 11 and the ""blood cell images"" captured by the imaging apparatus 12 is associated with ""identification information of the specimen"". The ""blood cell images"" are associated, for example, with the ""image IDs"" to identify the ""blood cell images"" and with the ""metadata"". The ""metadata"" include, for example, the classification type of the blood cells included in the blood cell images, a flag regarding the blood cells (for example, a flag indicating that the blood cell is an abnormal blood cell), a comment from a medical technologist who classified the blood cells, the timestamp of the blood cell images, and information regarding a scale/size (for example, the scale/size preset according to the classification type) of the blood cells included in the blood cell images. The interface 5 and 5A designate the blood cell images and metadata to be utilized, for example, based on the identification information of the specimen and/or the image IDs. The application 6 generates a summary, for example, based on the metadata of the blood cell images associated with the specimen. The summary may be stored in the data storage 18 or the storage 74, as shown in the example of FIG. 47. For example, the data management software 4 of the imaging apparatus 12 may generate the summary based on the blood cell images and the metadata of the blood cell images captured for the specimen, and store the summary in the data storage 18 or the storage 74. In this case, the application 6 may acquire the summary stored in the data storage 18 or the storage 74, and display the acquired summary. The application 6 may provide a function of modifying the metadata of the blood cell images. For example, the application 6 provides a function of changing the classification type of the blood cells included in the blood cell images via the GUI shown in the example of FIG. 46. In response to changing the classification type of the blood cells via the GUI, the application 6 updates, via the interface 5 or 5A, the metadata of the blood cell images in the data storage 18 and/or the storage 74, for example. The application 6 can provide a function of displaying the blood cell images acquired via the interface 5 or 5A, together with a reference image, for example. The application 6 refers to the metadata corresponding to each of the acquired blood cell images, and displays the blood cell images grouped according to the classification type of the blood cells, for example. The application 6 displays the reference image corresponding to the classification type of the group in a display area of the group, for example. The application 6 has reference images corresponding to classification types, for example. The reference images are, for example, images that serve as a reference for the blood cells corresponding to the classification types (for example, when the classification type is ""Neutrophil"", the reference image is a blood cell image in which a typical neutrophil is captured). The application 6 may display a ruler indicating an index of the size of the blood cells included in the blood cell images, for example. The application 6 displays the ruler by referring to the metadata of the blood cell images, for example. The metadata of the blood cell images may include, for example, information indicating the scale/size preset according to the classification type of the blood cells in the blood cell images. The application 6 displays the ruler, for example, based on the information indicating the scale/size included in the metadata of the blood cell images. The application 6 may provide a function for an operator to register information indicating that the displayed hematology test result and/or blood cell image have been validated, for example, in the GUI shown in FIG. 46. The hematology test result include, for example, the count/ratio of blood cells (for example, count/ratio of white blood cells, etc.), the classification of blood cells and the count/ratio for each classification of the blood cells, histograms, and scattergrams."

The "attribute information of the subject" may be associated with the identification information of the specimen. The "attribute information of the subject" can include, for example, the sex, age, clinical department, diagnosis, and medical record information regarding medical history of the subject.

The data storage 18 or the storage 74 may store a unique "metadata ID" associated with the metadata of the blood cell images. The application 6 may request the interface 5 and/or 5A to acquire desired metadata of the blood cell images based on the metadata ID, for example, when acquiring the metadata of the blood cell images. By making a request based on the metadata ID, for example, the application 6 can designate and request arbitrary metadata of the blood cell images among the plurality of metadata of the blood cell images related to the identification information of the specimen.

The hematology test result can include, for example, a value related to the count of at least one type of blood cells selected from among red blood cells, nucleated red blood cells, small red blood cells, platelets, reticulocytes, immature granulocytes, neutrophils, eosinophils, basophils, lymphocytes, and monocytes, and a value related to the ratio of at least one type of blood cells selected from among red blood cells, nucleated red blood cells, small red blood cells, platelets, reticulocytes, immature granulocytes, neutrophils, eosinophils, basophils, lymphocytes, and monocytes, and a value related to at least one selected from among hemoglobin (Hb), hematocrit (Hct), mean corpuscular volume (MCV), mean corpuscular hemoglobin (MCH), mean corpuscular hemoglobin concentration (MCHC), and mean platelet volume (MPV). The hematology test result may include scattergrams and histograms (for example, frequency distribution chart regarding red blood cells, frequency scattergram regarding white blood cells, and the like).

The image of the blood cells includes a flag indicating a possibility that abnormal blood cells are included among the blood cells included in each of the blood cell images. As the flag indicating an abnormal blood cell, for example, a value related to at least one selected from among nuclear morphological abnormality, granule abnormality, abnormal cell size, cell dysmorphia, cell destruction, vacuoles, immature cells, presence of inclusion bodies, Döhle bodies, rosetting phenomenon, abnormal nuclear chromatin pattern, petaloid nucleus, high nuclear-to-N/C ratio, blebbing morphology, smudge cells, and hairy cell-like morphology can be given. Nuclear morphological abnormality can include at least one selected from among hypersegmentation, hypo segmentation, Pseudo-Pelger-Huët anomaly, ring-shaped nucleus, spherical nucleus, oval nucleus, apoptosis, multinucleation, karyorrhexis, enucleation, naked nucleus, irregular nuclear contour, nuclear fragmentation, internuclear bridging, multiple nuclei, notched nucleus, karyokinesis, and nucleolar abnormality. Granule abnormality can include at least one selected from among degranulation, abnormal granule distribution, toxic granules, Auer rods, faggot cells, and Pseudo-Chediak-Higashi-like granules. A flag indicating that the blood cells of each blood cell image included in the image are abnormal cells may be added as additional metadata associated with the blood cell image.

The classification result, in which the plurality of blood cell images included in the image of the blood cells are classified by each attribute of the blood cells, may be classification result regarding blood cell type or morphology. A morphological classification of the blood cells can include a value related to the count of at least one type of blood cells selected from among neutrophils, eosinophils, platelets, lymphocytes, monocytes, basophils, metamyelocytes, myelocytes, promyelocytes, blast cells, plasma cells, atypical lymphocytes, immature eosinophils, immature basophils, erythroblasts, and megakaryocytes, and at least one value related to the ratio of at least one type of blood cells selected from among neutrophils, eosinophils, platelets, lymphocytes, monocytes, basophils, metamyelocytes, myelocytes, promyelocytes, blast cells, plasma cells, atypical lymphocytes, immature eosinophils, immature basophils, erythroblasts, and megakaryocytes. A classification of each of the plurality of blood cell images may be performed manually by an operator on the application, or automatically using machine classification and artificial intelligence algorithms. The operator may reclassify a result initially classified by the imaging apparatus 12 via the application.

The classification result (summary) may be corrected, for example, according to a manual or automatic modification of the metadata.

### [Flow of process performed by Application Software]

A description is given using the configuration example 1 (see FIG. 4) as an example. First, the data management software of the data management system 15 collects data including a result of an image captured by the imaging apparatus 12. For example, the data management software of the data management system 15 collects, from the imaging apparatus 12, data in which a result of an image of the blood cells is associated with the identification information related to the specimen. In addition, the blood cell image and the plurality of candidates of the specific blood cell in the blood cell image are associated with the identification information of the specimen may be collected. Furthermore, the data management software of the data management system 15 may collect data in which the summary and the notification are associated with the identification information related to the specimen, and may collect data including the hematology test result acquired by the specimen measurement apparatus 11. For example, the data management software of the data management system 15 may collect, from the specimen measurement apparatus 11, data in which the hematology test result is associated with the identification information.

Next, the application software 6 of the terminal apparatus 101 displays, on a display provided in the terminal apparatus 101, the data collected by the data management software of the data management system 15. The data displayed on the display provided in the terminal apparatus 101 includes, for example, the result of the image associated with the identification information. FIG. 45 shows an example of a GUI (Graphical User Interface) displaying the data in which each of the plurality of the blood cell images and the plurality of candidates of the specific blood cell in the blood cell image is associated with the identification information (specimen ID). In FIG. 45, the blood cell image are displayed grouped by classification type (for example, BL (blast), MY (myelocyte), MMY (metamyelocyte)), and for the specific blood cell image, the plurality of candidates (for example, BL (blast), BA (basophil), VLY (variant lymphocyte)) are displayed. The blood cell images are displayed in a number ranging from several to several hundred, and may be individual blood cell images captured, or a single blood cell image generated by concatenating a plurality of blood cell images.

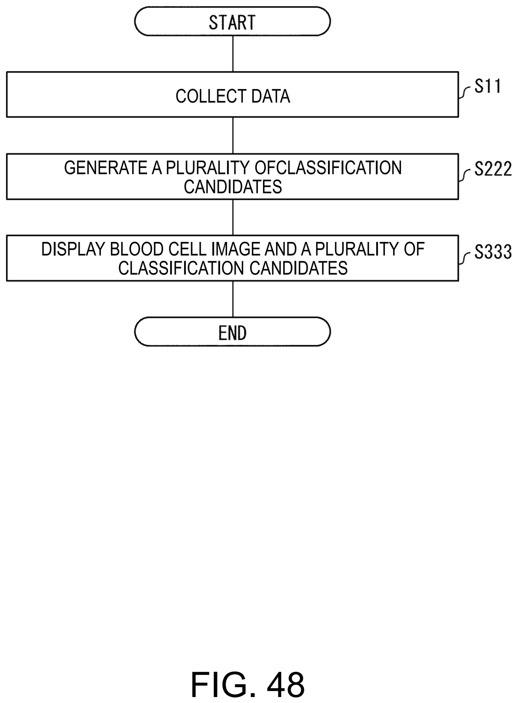

FIG. 48 is a flowchart showing an example of a flow of process performed by the application software 6 of the terminal apparatus 101. In FIG. 48, the application software 6 of the terminal apparatus 101 collects data including a result of images of a smear captured by the imaging apparatus 12 (S11). The collected data may include a hematology test result. The application software 6 collects the data from, for example, the data management system 15. The application software 6 generates the plurality of candidates of the specific blood cell in the blood cell image based on the collected data (S222). For example, the application software 6 may collect the plurality of candidates generated by the data management system 15. The application software 6 of the terminal apparatus 101 displays the blood cell image and the plurality of candidates for each specimen ID (S333). The application software 6 may display the summary, the notification, and the hematology test result. As exemplified in FIG. 45, the application software 6 may display the blood cell image and the plurality of candidates. In addition, as exemplified in FIG. 46, the application software 6 may display the blood cell image, the plurality of candidates, the summary, the notification, and the hematology test result.

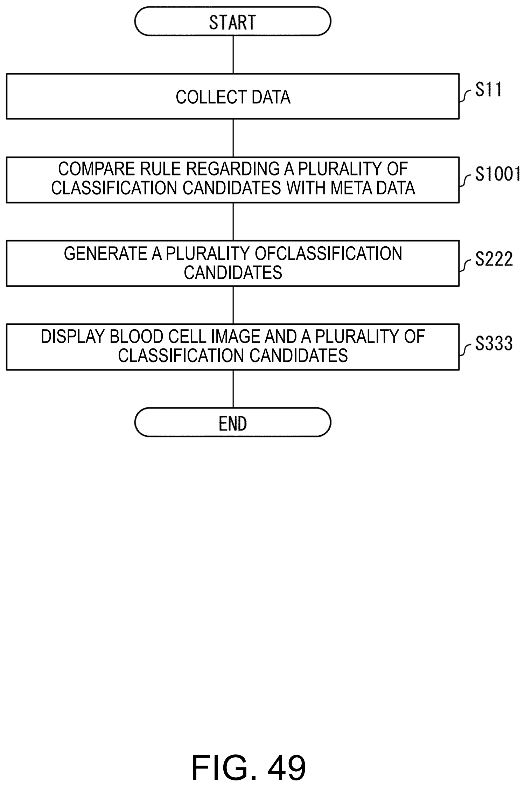

FIG. 49 is a flowchart showing details of the flow of process in FIG. 48. The application software 6 of the terminal apparatus 101 collects data including the result of the image of the smear (S111). The collected data may include the hematology test result. The application software 6 collects the data from, for example, the data management system 15. The application software 6 compares metadata included in the collected data with a rule regarding the plurality of candidates (S1001). The rule regarding the plurality of candidates is preset in the application software 6, for example. The rule regarding the plurality of candidates may be a plurality of rules set by statistical information such as classification results or classification probabilities based on past image data classified by image analysis on blood cell images, or may be a rule set by a predetermined classification criterion based on past image data classified by image analysis on blood cell images. Further, the rule regarding the plurality of candidates may be, for example, a rule using an artificial intelligence algorithm preset in the application software 6. The rule regarding the plurality of candidates may be arbitrarily settable by a user of the application software 6 based on past data regarding microscopic examination of a smear. Based on the determination by the rule, the application software 6 of the terminal apparatus 101 generates the plurality of candidates (S222), and displays the blood cell image and the plurality of candidate for each specimen ID (S333). The blood cell image and the plurality of candidates are displayed, for example, as shown in the aforementioned FIG. 45. Alternatively, when the rule regarding the plurality of candidates is compared with the metadata in S1001 and it is determined that the rule regarding the plurality of candidates is not satisfied, the plurality of candidates are not generated, and for example, only the blood cell images classified into a single cell type are displayed on the GUI.

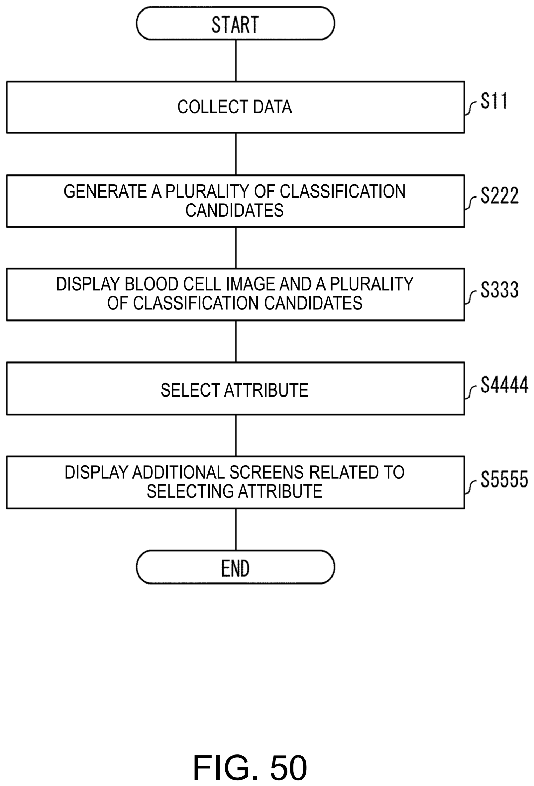

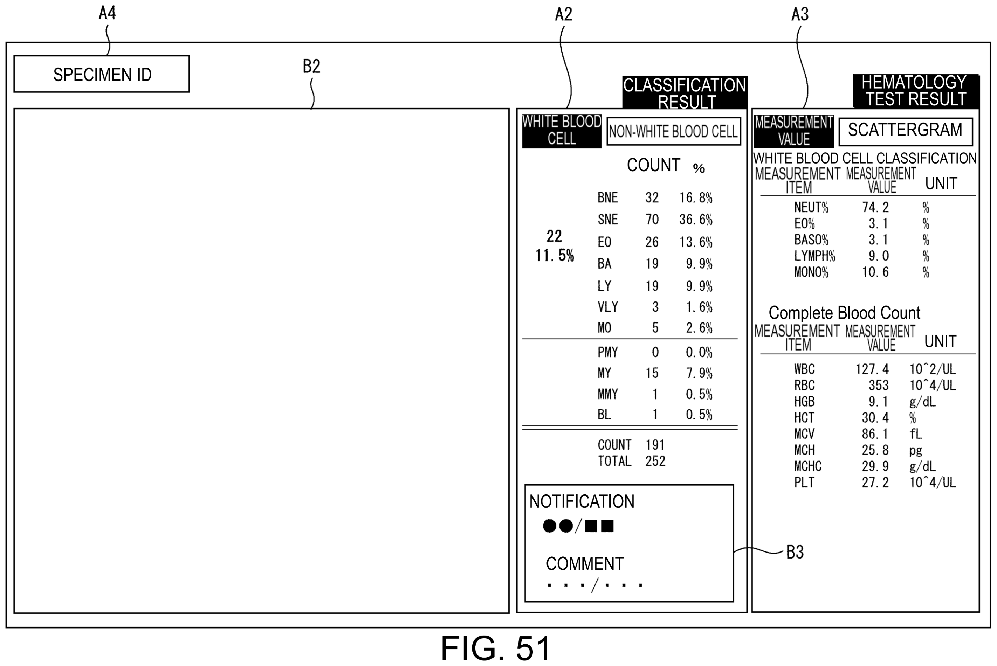

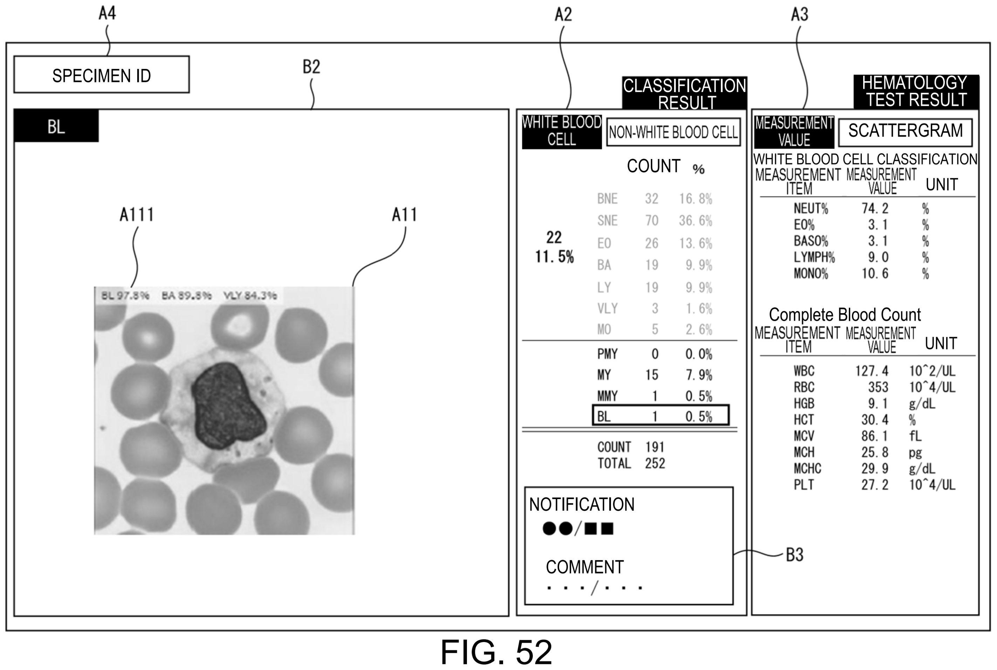

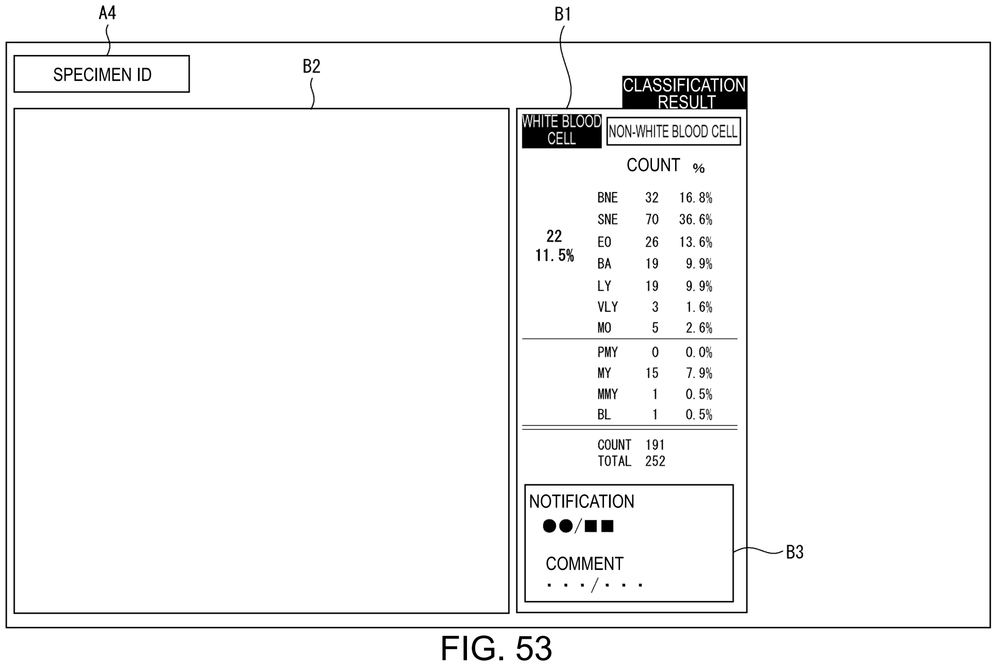

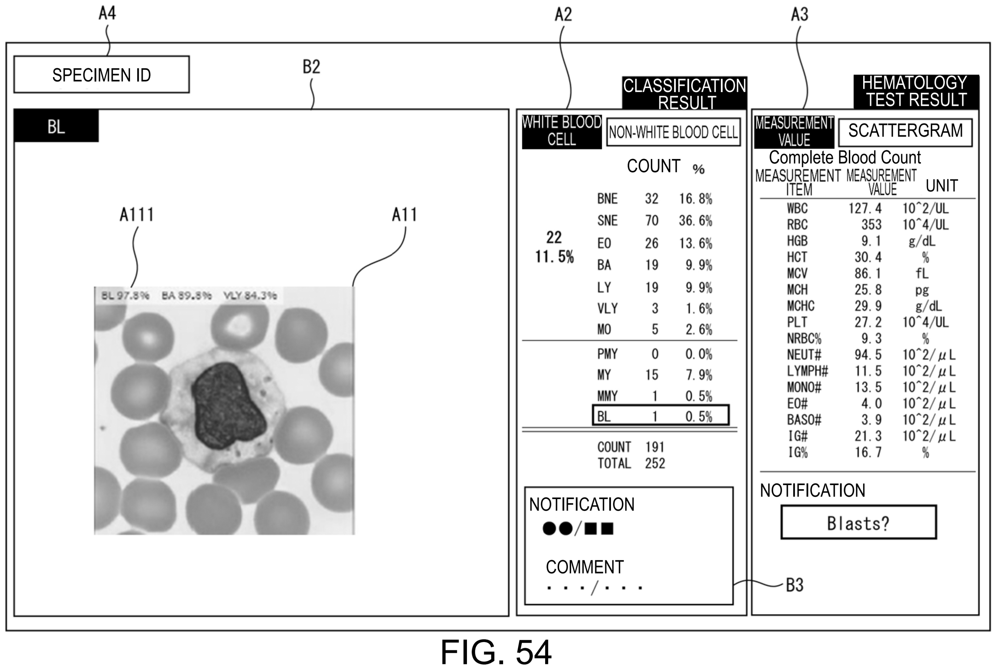

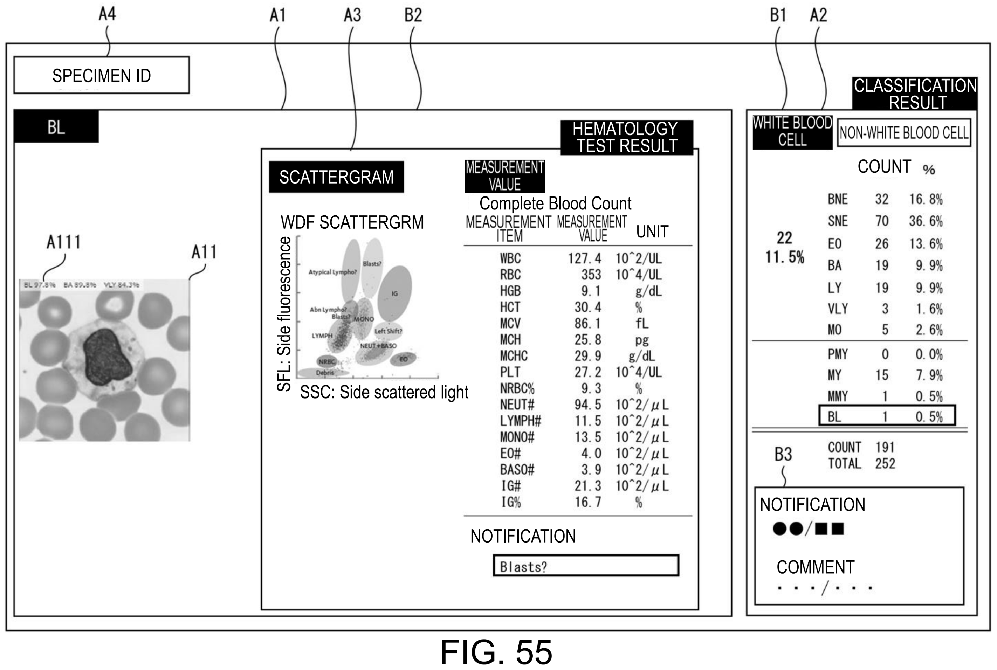

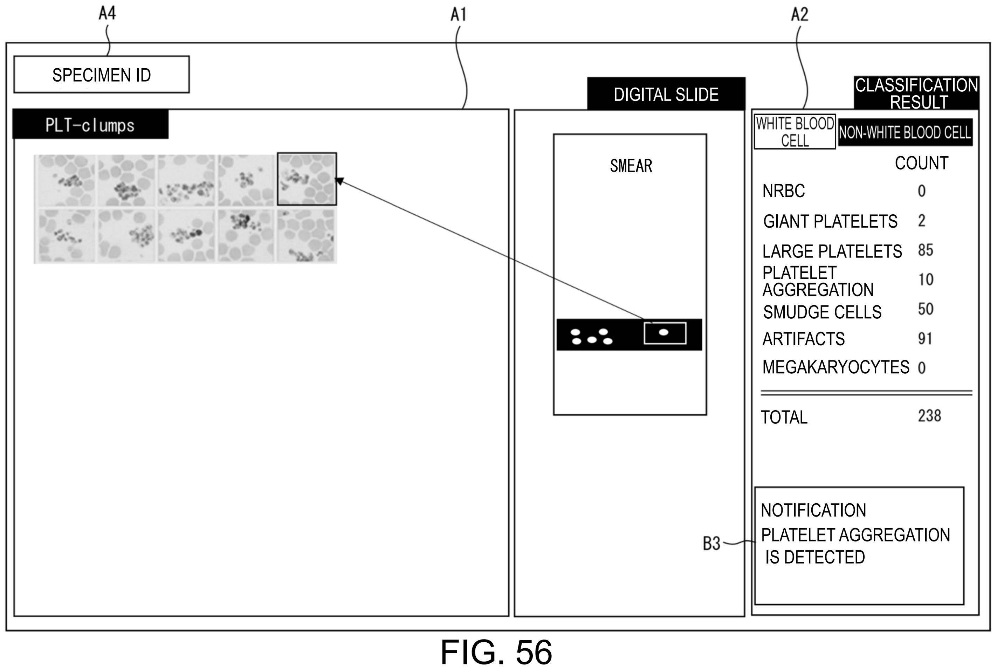

FIG. 50 is a flowchart showing a modification of FIG. 48. In an example of FIG. 50, the application software 6 is capable of change the display content in response to an operation of the GUI. In FIG. 50, after S333, the application software 6 displays an additional screen associated with a selection of the attribute of the blood cell images (S5555) in response to the selection of the attribute (S4444). The attribute of the blood cell images is, for example, the classification type of the blood cells. The application software 6 can display, for example, the plurality of candidates of the specific blood cell in a related blood cell image and the hematology test result as the additional screen according to the selected classification type. FIGS. 51 and 52 show an example 3 of providing the GUI corresponding to the flowchart of FIG. 50. In the screen shown in FIG. 51 in which the summary A2, the notification B3, and the hematology test result A3 are displayed, when the selection of the attribute (classification type indicated by the bold frame: BL (blast)) included in the summary A2 is received as shown in FIG. 52 (S4444), the blood cell image A11 related to the selection of the attribute (BL (blast)) and the plurality of candidates A111 of the specific blood cell in the blood cell image A11 are displayed in an area B2 as the additional screen (S5555). The application software 6 may, for example, highlight the display content of the selected attribute (for example, classification type of the blood cells) compared to the display content of the unselected attribute. As an example of highlighting the display content, for example, the selected classification type may be displayed within a frame such as a rectangle, or may be displayed in a color distinguishable from the display content of the unselected classification type. The operation for selecting the attribute includes at least one of a mouse operation, a touch panel operation, and a voice operation. FIGS. 53 and 54 show an example 4 of providing the GUI. As shown in FIGS. 53 and 54, the summary is displayed for each attribute (for example, for each classification type), an area B1 displaying the notification B3 is displayed, and the blood cell image A11 related to the selection of the attribute, the plurality of candidates A111 of the specific blood cell in the blood cell image A11, and the hematology test result A3 may be displayed as the additional screen according to the selection of the attribute (the portion indicated by the bold frame). FIG. 55 shows an example 5 of providing the GUI. As shown in FIG. 55, for example, a first area B1 displaying the summary A2 and the notification B3 and a second area B2 displaying the blood cell image A1 are displayed, and the blood cell image A11 related to the selection of the attribute, the plurality of candidates A111 of the specific blood cell in the blood cell image A11, and a scattergram (for example, a scattergram in which a cell cluster indicating blasts has appeared) included in the hematology test result A3 may be displayed according to the selection of the attribute (for example, the classification type indicated by the bold frame: BL (blast)). FIG. 56 shows an example 6 of providing the GUI. In the GUI of the example 6, the blood cell image A1 in which platelet aggregation is detected, the notification B3 (for example, "Platelet aggregation is detected"), and the summary A2 are displayed. In the GUI of the example 6, a digital slide virtually indicating positions on the smear 120 at which the blood cell images were captured by the imaging apparatus 12 may be displayed together with the blood cell image. The digital slide shown in FIG. 56 indicates the platelet aggregation (for example, aggregates of 5 or more platelets) in the blood cell images captured by the imaging apparatus 12 with white dots. By reviewing the digital slide, the operator can determine, for example, whether the platelet aggregation in the blood cell images is present on the upper, lower, left, of right region of the smear 120.

Conventionally, in the imaging apparatus 12, the operator has validated whether the acquired results may be output for use in diagnosis. A flag indicating whether the operator has validated the acquired result of capturing the image of the blood cells may also be displayed and used as reference information when the results are reviewed with the application.

### (Configuration Example of Providing Application Function as Web service)

The function of the application 6 need not necessarily be provided as stand-alone software to be executed in the terminal apparatus 101 and the specimen measurement apparatus 11, and may be provided as a Web service.

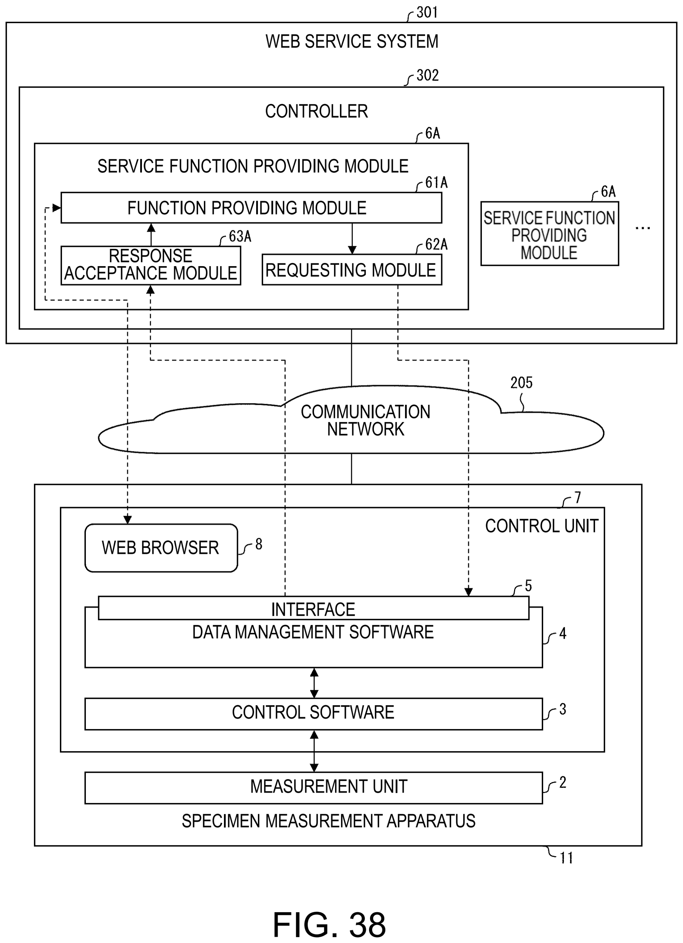

FIG. 38 shows a configuration example of providing the function of the application 6 as a Web service. In the configuration example shown in FIG. 38, the specimen measurement system 1 includes: a specimen measurement apparatus 11 having a Web browser 8 installed therein; and a Web service system 301 connected to the specimen measurement apparatus 11 via a communication network 205. The communication network 205 is, for example, the Internet. The specimen measurement apparatus 11 includes: the measurement unit 2; and the control unit 7 having the processor 71 that executes the control software 3, the data management software 4, the interface 5, and the Web browser 8.

The Web service system 301 includes a controller 302 having a processor that executes a service function providing module 6A functioning as a Web service. The service function providing module 6A has a function equivalent to that of the application 6. The service function providing module 6A includes, for example, a function providing module 61A, a requesting module 62A, and a response acceptance module 63A. The function providing module 61A, the requesting module 62A, and the response acceptance module 63A respectively have the functions equivalent to those of the function providing module 61, the requesting module 62, and the response acceptance module 63.

In the case of the present configuration example, when the service function providing module 6A functioning as a Web service is accessed by the Web browser 8 of the specimen measurement apparatus 11, information and function can be utilized via the interface 5 of the specimen measurement apparatus 11.

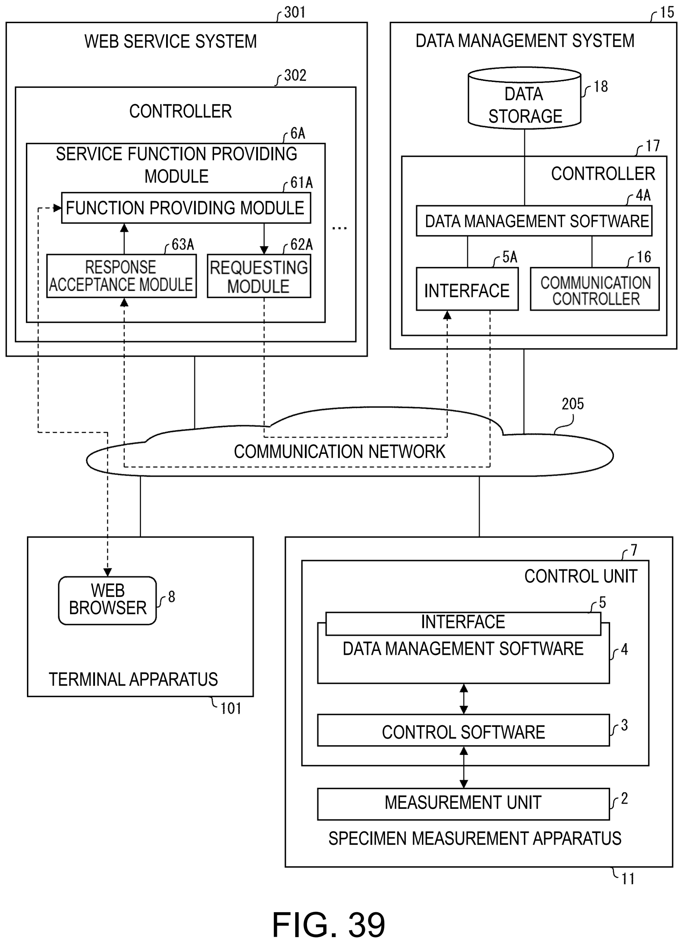

FIG. 39 shows another configuration example for providing the function of the application 6 as a Web service. In the configuration example shown in FIG. 39, the specimen measurement system 1 has a configuration in which the specimen measurement apparatus 11, the Web service system 301, the data management system 15, and the terminal apparatus 101 having the Web browser 8 installed therein are connected to each other via the communication network 205.

In the case of the present configuration example, when the service function providing module 6A functioning as a Web service is accessed by the Web browser 8 of the terminal apparatus 101, information managed in the data storage 18 can be utilized via the interface 5A of the data management system 15.

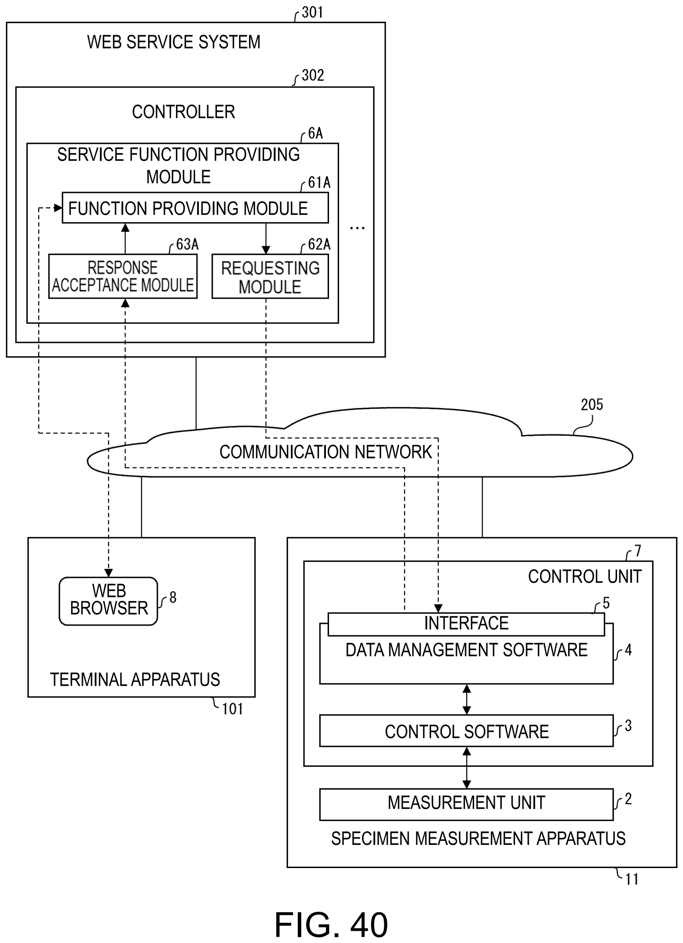

FIG. 40 shows still another configuration example for providing the function of the application 6 as a Web service. In the configuration example shown in FIG. 40, the specimen measurement system 1 has a configuration in which the specimen measurement apparatus 11, the Web service system 301, and the terminal apparatus 101 having the Web browser 8 installed therein are connected to each other via the communication network 205.

In the case of the present configuration example, when the service function providing module 6A functioning as a Web service is accessed by the Web browser 8 of the terminal apparatus 101, information and function can be utilized via the interface 5 of the specimen measurement apparatus 11.

### (Provision Form of Application)

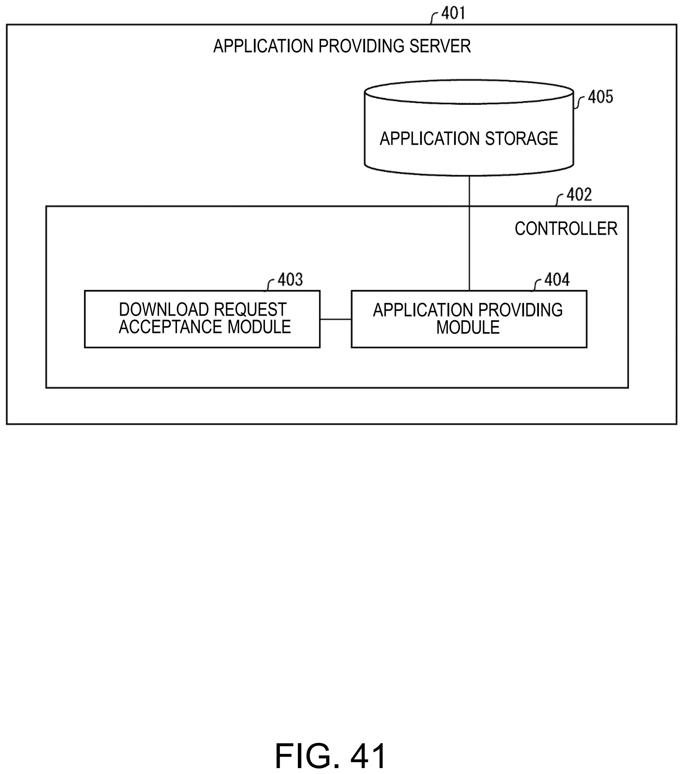

A provision form of the application 6 will be described. FIG. 41 shows a configuration example of an application providing server 401 that provides the application 6. The application providing server 401 is arranged on, for example, the cloud, and is communicably connected to the terminal apparatus 101 and the control unit 7 via a communication network. The terminal apparatus 101 and the control unit 7 are each, for example, a smartphone (iPhone, Android terminal), a tablet (iPad, Android tablet, Windows tablet), a Windows PC having Windows OS installed therein, or a Mac having Mac OS installed therein. The application providing server 401 includes: a controller 402 having a processor that executes a download request acceptance module 403 and an application providing module 404; and an application storage 405 having the application 6 stored therein.

The operator of the terminal apparatus 101 and the control unit 7 logs into a predetermined download application (for example, App Store, Google Play Store, Windows Store) using an account ID (for example, Apple ID, Google account, Microsoft account, or the like) managed by an application provider, for example, and requests download of a desired application 6 to the application providing server 401 via the download application.

For example, the download request acceptance module 403 notifies the application providing module 404 of information regarding the requested application 6. The application providing module 404 searches and acquires the application 6 notified from the application storage 405, and transmits the application 6 to the request source (the terminal apparatus 101 or the control unit 7). The application providing module 404 may transmit, for example, an installer of the application 6 to the request source (the terminal apparatus 101 or the control unit 7). The terminal apparatus 101 or the control unit 7 having received the installer installs the application 6 on the basis of the installer.

The application providing server 401 may be compatible with a multi-platform (e.g., Windows, MacOS, iOS, Android). A plurality of the application providing servers 401 may be present according to the kind of the platform. For example, the application providing server 401 (for example, App Store) that provides the application 6 for iOS, the application providing server 401 (for example, Google Play) that provides the application 6 for Android, the application providing server 401 (for example, Microsoft Store) that provides the application 6 for Windows, and the like. In this case, the terminal apparatus 101 and the control unit 7 request provision of the application 6 by a method according to the platform.

The terminal apparatus 101 and the control unit 7 having iOS installed therein request, for example, to the application providing server 401 that provides the application 6 for iOS, download of the desired application 6 searched for by a predetermined application (for example, an App Store). The terminal apparatus 101 and the control unit 7 having Android OS installed therein request, for example, to the application providing server 401 that provides the application 6 for Android, download of the desired application 6 searched for by a predetermined application (for example, Google Play store). The terminal apparatus 101 and the control unit 7 having Windows OS installed therein request, for example, to the application providing server 401 that provides the application 6 for Windows, download of the desired application 6 searched for by a predetermined application (for example, Microsoft Store).

Depending on the medical facility or the laboratory, it is assumable that the terminal apparatus 101 and the control unit 7 are not connected to the Internet or the like. In such cases, for example, from a medium (for example, CD/DVD/USB memory/external HDD/SDD) having stored therein an installer for the application 6, a maintenance person may copy the installer into the terminal apparatus 101 and the control unit 7, and may install the application 6 into the terminal apparatus 101 and the control unit 7.

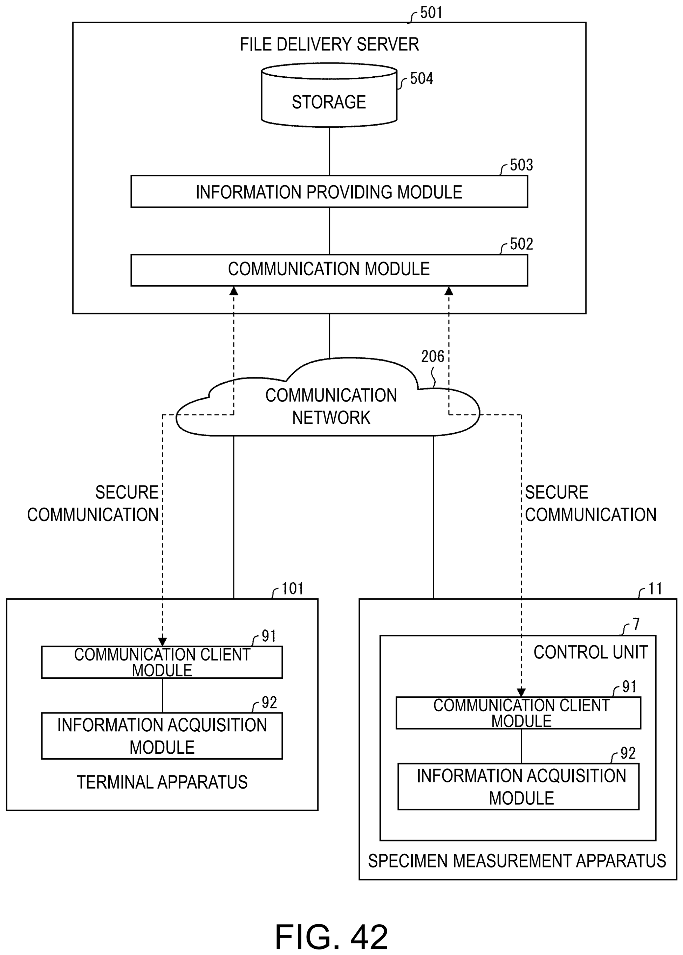

Depending on the medical facility or the laboratory, the terminal apparatus 101 and the control unit 7 may be allowed to perform communication with the outside only in a communication network (for example, VPN: Virtual Private Network) in which security is ensured. In this case, for example, from a server for which a secure communication network with respect to the terminal apparatus 101 and the control unit 7 can be set, the application 6 may be delivered. FIG. 42 shows a configuration example of providing the application 6 in a secure communication environment. The secure communication environment includes an environment in which a file delivery server 501 is placed in an intranet in a facility and can be accessed via the intranet from the terminal apparatus 101 and the control unit 7.

The file delivery server 501 includes: a processor that executes a communication module 502 and an information providing module 503; and a storage 504 having stored therein data (for example, installers and setting files of the applications 6) necessary for installing the application 6. The communication module 502 has, for example, a VPN server function. The information providing module 503 has, for example, a Web server function.

The terminal apparatus 101 and the control unit 7 each have a processor that executes a communication client module 91 and an information acquisition module 92. The communication client module 91 has, for example, a VPN client function, and sets a secure communication network 206 with the communication module 502. The information acquisition module 92 is, for example, a Web browser.

The information acquisition module 92 of the terminal apparatus 101 and the control unit 7 accesses the information providing module 503 of the file delivery server 501 via the secure communication network 206. The information providing module 503 provides, for example, data that can be downloaded by the terminal apparatus 101 and the control unit 7, to the information acquisition module 92. The information acquisition module 92 presents the data in a Web page. When the operator of the terminal apparatus 101 and the control unit 7 selects data to be downloaded from the Web page, the information providing module 503 acquires corresponding data from the storage 504 and transmits the data to the information acquisition module 92. The information acquisition module 92 installs the application 6 on the basis of the acquired data.

The terminal apparatus 101 and the control unit 7 may download an installer of the application 6 via the Internet and install the application 6 using the installer.

### (Web Clip)

The function of the application 6 may be provided to the terminal apparatus 101 and the control unit 7 as a Web clip (a short-cut to a Web site). The service provider notifies, for example, the terminal apparatus 101 and the control unit 7 of a URL (Uniform Resource Locator) to serve as the access determination of the Web clip. The operator of the terminal apparatus 101 and the control unit 7 bookmarks the URL notified of, as a Web clip onto the home screen or the desktop of the terminal apparatus 101 and the control unit 7, for example. The operator of the terminal apparatus 101 and the control unit 7 may access a Website of the service provider by the Web browser installed in the terminal apparatus 101 and the control unit 7, access a menu providing a desired service from the Website, and bookmark the site of the menu as a Web clip, for example.

The system configuration example for utilizing the function of the application 6 by using a Web clip is similar to those of FIGS. 38 to 40. In the configuration example shown in FIG. 38, when the service function providing module 6A functioning as the Web service corresponding to the URL designated by the Web clip is accessed by the Web browser 8 of the control unit 7, information and functions can be utilized via the interface 5 of the specimen measurement apparatus 11. Similarly, in the configuration example shown in FIG. 39, when the service function providing module 6A functioning as the Web service corresponding to the URL designated by the Web clip is accessed by the Web browser 8 of the terminal apparatus 101, information managed in the data storage 18 can be utilized via the interface 5A of the data management system 15. Similarly, in the configuration example shown in FIG. 40, when the service function providing module 6A functioning as the Web service corresponding to the URL designated by the Web clip is accessed by the Web browser 8 of the terminal apparatus 101, information and functions can be utilized via the interface 5 of the specimen measurement apparatus 11.

### (Provision of Application by MDM/MAM)

The application 6 may be provided not via a predetermined application provider (For example, Apple Store, Google Play Store, Microsoft Store). For example, the application 6 may be provided to the terminal apparatus 101 and the control unit 7, by utilizing the mechanism of MDM (Mobile Device Management) or MAM (Mobile Application Management) , and not via a predetermined application provider.

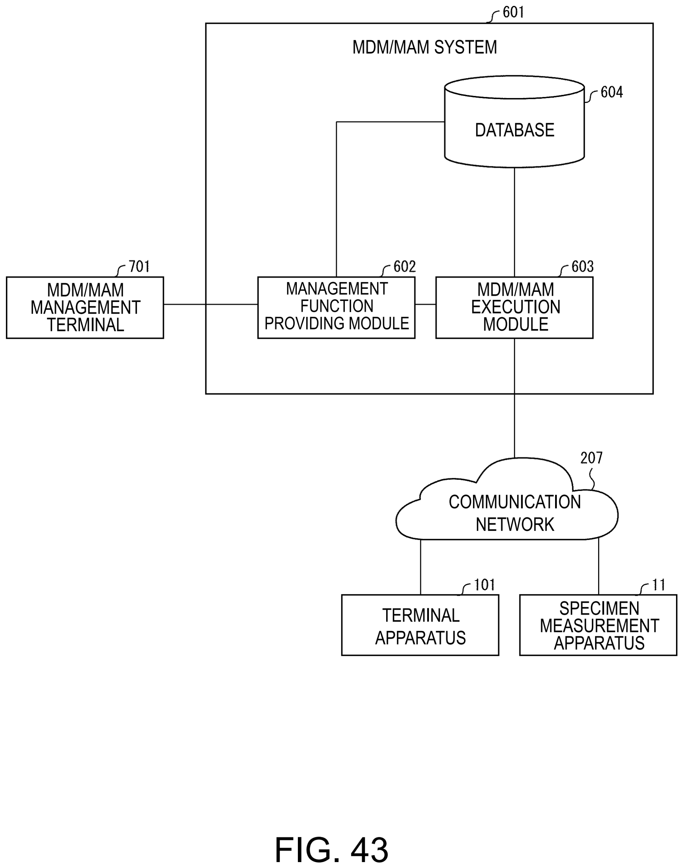

FIG. 43 shows a configuration example for providing the application 6 by utilizing MDM or MAM. The terminal apparatus 101 and the control unit 7 are communicably connected to an MDM/MAM system 601 via a communication network 207. The terminal apparatus 101 and the control unit 7 are registered in the MDM/MAM system 601 and managed by the MDM/MAM system 601 in accordance with a management policy. In order to manage the terminal apparatus 101 and the control unit 7 in the MDM/MAM system 601, for example, a management profile is installed into the terminal apparatus 101 and the control unit 7. For example, information (for example, a URL for downloading an installer) for installing the management profile is notified of (for example, by an electronic mail or the like) to the terminal apparatus 101 and the control unit 7.

When the management profile has been installed in the terminal apparatus 101 and the control unit 7, the terminal apparatus 101 and the control unit 7 are registered into the MDM/MAM system 601, and information regarding the terminal apparatus 101 and the control unit 7 is registered into a database 604 of the MDM/MAM system 601.

An MDM/MAM execution module 603 acquires information on the application 6 installed in the terminal apparatus 101 and the control unit 7, and registers the information into an application list of the database 604. The MDM/MAM execution module 603 can deliver the application 6 to the terminal apparatus 101 and the control unit 7. The terminal apparatus 101 and the control unit 7 install the delivered application 6. Accordingly, the application 6 can be provided not via a predetermined application provider (App Store or the like).

A management function providing module 602 provides a management function of the terminal apparatus 101 and the control unit 7 to an MDM/MAM management terminal 701. For example, the management function providing module 602 provides, to the MDM/MAM management terminal 701, a Website for managing the terminal apparatus 101 and the control unit 7. Via the Web site, the operator of the MDM/MAM management terminal 701 executes: registration and change of the management policy to the terminal apparatus 101 and the control unit 7; delivery of the application 6 to the terminal apparatus 101 and the control unit 7; and management of the application 6 installed in the terminal apparatus 101 and the control unit 7, for example.

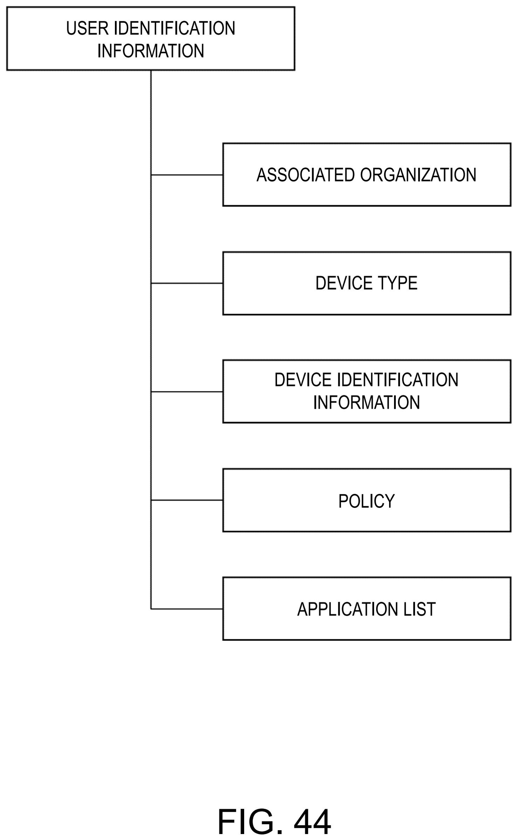

FIG. 44 shows an example of the data structure of the database 604 when the database 604 is a relational database. As shown in FIG.44, the information managed by the database 604 includes, for example, user identification information, associated organization, device type, device identification information, policy, and application list.

The user identification information is information for identifying the user of the terminal apparatus 101 and the control unit 7. For example, an ID assigned to the user from the MDM/MAM system 601, the name of the user, a combination thereof, and the like are included.

The associated organization is identification information of the organization to which the terminal apparatus 101 and the control unit 7 are associated. For example, an ID assigned to the organization from the MDM/MAM system 601, the organization name, a combination thereof, and the like are included.

The device type is information for identifying the platform of the device. For example, iOS, Android, Windows, Mac, and the like are included.

The device identification information is identification information unique to the terminal apparatus 101 and the control unit 7 to serve as the management target in the MDM/MAM system 601. Examples include IMEI (International Mobile Equipment Identifier), serial numbers, SIM (Subscriber Identity Module) card number, telephone numbers, MAC (Media Access Control) addresses, combinations thereof, and the like.

The policy is the management policy by the MDM/MAM system 601. For example, setting information of the management policy is included.

The application list is information regarding the application 6 installed in the terminal apparatus 101 and the control unit 7 corresponding to the user identification information. For example, a list identification information (application name, application ID) of the application 6 and the version of the application 6 are included.

The present invention is not limited to the embodiments described above, and various modifications can be made within the scope of the claims. Embodiments obtained by combining, as appropriate, the technological means disclosed in different embodiments are also included in the technological scope of the present invention.

### (Supplementary Note)

The inventions according to the following items can be extracted from the embodiments described above.

Item 1 A system including a measurement unit configured to measure a specimen and an imaging unit configured to capture an image of a blood cell in the specimen, the system comprising: a first control software configured to control the measurement unit; a first data management software configured to manage first data regarding the measurement unit that operates under control by the first control software; a second control software configured to control the imaging unit; a second data management software configured to manage second data including a blood cell image of the blood cell in the specimen captured by the imaging unit that operates under control by the second control software, and metadata of the blood cell image; and an interface configured to allow application software capable of being added to the system, to utilize the first data and the second data, wherein the interface is configured to allow the application software to utilize the second data including the metadata indicating a classification of a cell type of the blood cell, and a function of the system is extended, by the application software added to the system, to display a plurality of classification candidates of the blood cell based on the metadata including the plurality of classification candidates of the blood cell.

Item 2 The system according to Item 1, wherein the interface allows the application software to utilize the second data including the metadata indicating the plurality of classification candidates classified by image analysis on the blood cell images.

Item 3 The system according to Item 1, wherein the interface allows the application software to utilize the second data including the metadata, and the function of the system is expanded by the application software, to display the plurality of classification candidates associated with classification probabilities of each of the plurality of classification candidates, based on the metadata.

Item 4 The system according to Item 1, wherein the interface allows the application software to utilize the second data in which a plurality of the metadata are associated with identification information related to the specimen.

Item 5 The system according to Item 1, wherein the interface allows the application software to utilize the second data in which each of a plurality of the metadata and a plurality of the blood cell images is associated with identification information related to the specimen.

Item 6 The system according to Item 1, wherein the interface allows the application software to utilize the second data in which each of a plurality of the metadata, a plurality of the blood cell images, and a plurality of image IDs distinguishing respective one of the plurality of blood cell images is associated with identification information related to the specimen.

Item 7 The system according to Item 1, wherein the interface allows the application software to utilize the first data and the second data, in which each of a plurality of the metadata, a plurality of the blood cell images, and a hematology test result of the specimen is associated with identification information related to the specimen.

Item 8 The system according to Item 1, wherein the interface allows the application software to utilize the first data and the second data, in which each of a plurality of the metadata, a plurality of the blood cell images, a hematology test result of the specimen, and attribute information of a subject is associated with identification information related to the specimen.

Item 9 The system according to Item 1, wherein the interface allows the application software to utilize the first data and the second data in which each of a plurality of the metadata, a plurality of the blood cell images, and information related to platelets based on the metadata is associated with identification information related to the specimen.

Item 10 The system according to Item 1, wherein the interface allows the application software to utilize the second data in which each of the metadata and the blood cell image is associated with identification information related to the specimen, and the function of the system is expanded by the application software to display the plurality of classification candidates associated with the blood cell image.

Item 11 The system according to Item 1, wherein the interface allows the application software to utilize the second data in which each of the metadata and the blood cell images is associated with identification information related to the specimen, and the function of the system is expanded by the application software, to display the blood cell image associated with the plurality of classification candidates in an enlarged manner.

Item 12 The system according to Item 1, wherein the interface allows the application software to utilize the second data in which each of the metadata and the blood cell images is associated with identification information related to the specimen, and the function of the system is expanded by the application software, to display the plurality of classification candidates based on a result of comparing the metadata with a preset rule.

Item 13 The system according to Item 1, wherein the interface allows the application software to utilize the second data in which each of the metadata and the blood cell images is associated with identification information related to the specimen, and the function of the system is expanded by the application software, to display the plurality of classification candidates based on the metadata compared with a plurality of preset rules.

Item 14 The system according to Item 1, wherein the interface allows the application software to utilize the second data in which each of the metadata and the blood cell images is associated with identification information related to the specimen, and the function of the system is expanded by the application software, to receive a setting of a rule related to the plurality of classification candidates.

Item 15 The system according to Item 1, wherein the interface allows the application software to utilize the second data in which each of the metadata and the blood cell image is associated with identification information related to the specimen, and the function of the system is expanded by the application software, to display a reference image to be compared with a cell included in the blood cell image.

Item 16 The system according to Item 1, wherein the interface allows the application software to utilize the second data in which each of the metadata and the blood cell image is associated with identification information related to the specimen, and the function of the system is expanded by the application software, to display a ruler indicating an index of a size of a cell contained in the blood cell image.

Item 17 The system according to Item 1, wherein the interface allows the application software to utilize the second data in which each of the metadata and the blood cell image is associated with identification information related to the specimen, and the function of the system is expanded by the application software, to display the plurality of classification candidates based on a predetermined classification criterion.

Item 18 The system according to Item 1, wherein the interface allows the application software to utilize the second data which includes a plurality of image IDs distinguishing respective ones of a plurality of the blood cell images and each of the plurality of blood cell images associated with each of the plurality of image IDs, and the function of the system is expanded by the application software, to specify each of a plurality of the metadata utilized via the interface, based on the image IDs.

Item 19 The system according to Item 1, wherein the interface allows the application software to utilize the second data including metadata of each of a plurality of the blood cell images, and the function of the system is expanded by the application software, to display statistical information regarding cells included in each of the plurality of blood cell images for each attribute of the cells, and to modify the statistical information in response to modification of the metadata.

Item 20 The system according to Item 1, wherein the interface allows the application software to utilize the data based on a predetermined rule.

Item 21 The system according to Item 1, wherein the interface allows a plurality of types of the application software to utilize the data based on a predetermined rule that is common to the plurality of types of application software offering different functions.

Item 22 The system according to Item 1, wherein the interface provides a response corresponding to a request from the application software to the application software in response to the request.

Item 23 The system according to Item 1, wherein the interface allows the application software to utilize the data based on a command created by the application software according to a predetermined rule.

Item 24 The system according to Item 1, wherein the interface allows the application software to utilize at least one of the data regarding the measurement unit, the data regarding a measurement operation by the measurement unit, the data regarding maintenance of the measurement unit, and the data regarding an operation of the measurement unit, based on a command created by the application software according to a predetermined rule.

Item 25 The system according to Item 1, wherein the interface causes the application software to execute at least one of acquisition of the data, registration of the data, update of the data, and deletion of the data, based on a command created by the application software according to a predetermined rule.

Item 26 The system according to Item 1, wherein the interface allows a plurality of types of the application software to utilize the data based on a predetermined rule that is common to the plurality of types of application software each operating on a plurality of types of operating systems.

Item 27 The data management software manages the data based on a classification corresponding to a type of the data.

Item 28 The system according to Item 1, wherein the interface allows the application software to utilize the data based on a predetermined rule, and the data management software manages the data based on a classification corresponding to the predetermined rule.

Item 29 The system comprises a specimen measurement apparatus including the measurement unit, the control software, and the data management software, and the interface allows the application software to utilize the data based on a plurality of predetermined rules each corresponding to a type of the specimen measurement apparatus.

Item 30 The system comprises a specimen measurement apparatus including the measurement unit, the control software, and the data management software, and the specimen measurement apparatus is certified as a medical device.

Item 31 The system according to Item 1, wherein the interface allows the application software, which is a non-medical device, to utilize the data.

Item 32 The control software and the data management software are independent software from the application software.

Item 33 The measurement unit includes a specimen processing part for preparing a measurement sample based on the specimen and a reagent, and a detector for measuring the prepared measurement sample, the control software controls operations of the specimen processing part and the detector in collaboration with the data management software, and the data management software manages the data including a measurement result obtained by the operations of the specimen processing part and the detector.

Item 34 The measurement unit includes a specimen processing part for preparing a measurement sample based on the specimen and a reagent, and a detector for measuring the prepared measurement sample, the control software controls operations of the specimen processing part and the detector in collaboration with the data management software, the data management software manages the data including a measurement result obtained by the operations of the specimen processing part and the detector, and the control software and the data management software are independent software from the application software.

Item 35 The measurement unit includes a specimen processing part for preparing a measurement sample based on the specimen and a reagent, and a detector for measuring the prepared measurement sample, the control software controls operations of the specimen processing part and the detector in collaboration with the data management software, the data management software manages the data including a measurement result obtained by the operations of the specimen processing part and the detector, the control software and the data management software are independent software from the application software, and the data management software can collaborate with the application software via the interface.

Item 36 The system comprises a specimen measurement apparatus including the measurement unit, the control software, and the data management software, the measurement unit includes a specimen processing part for preparing a measurement sample based on the specimen and a reagent, and a detector for measuring the prepared measurement sample, the control software controls operations of the specimen processing part and the detector in collaboration with the data management software, the data management software manages the data including a measurement result obtained by the operations of the specimen processing part and the detector, the data management software can collaborate with the application software via the interface, the measurement unit, the control software, and the data management software are components of a certified medical device, and the control software and the data management software are independent software from the application software.

Item 37 The system comprises a specimen measurement apparatus including the measurement unit, the control software, and the data management software, the measurement unit includes a specimen processing part for preparing a measurement sample based on the specimen and a reagent, and a detector for measuring the prepared measurement sample, the control software controls operations of the specimen processing part and the detector in collaboration with the data management software, the data management software manages the data including a measurement result obtained by the operations of the specimen processing part and the detector, the data management software can collaborate with the application software via the interface, the measurement unit, the control software, and at least a part of the data management software provide functions corresponding to an intended use of the specimen measurement apparatus certified as a medical device, and the function of the system is expanded by the application software capable of providing a function different from the functions corresponding to the intended use.

Item 38 Operation control of the specimen processing part and the detector by the control software includes at least one of: control of a suction operation of a liquid including at least one of the specimen and the reagent; control for mixing the specimen and the reagent to prepare a measurement sample; control for causing the detector to detect the measurement sample; and control for causing the detector to detect at least one of optical information and an electric signal regarding the measurement sample, and analyzing the measurement result based on a detected detection result.

Item 39 An application software used in a system including a measurement unit configured to measure a specimen, a first control software configured to control the measurement unit, a first data management software configured to manage first data regarding the measurement unit that operates based on control by the first control software, an imaging unit configured to image blood cells in the specimen, a second control software configured to control the imaging unit, and a second data management software configured to manage second data regarding the imaging unit that operates based on control by the second control software, the application software comprising: a requesting module for requesting an interface to utilize the first data and the second data including the blood cell images in which the blood cells in the specimen are imaged and the metadata indicating classification of cell types of the blood cells; a response acceptance module for accepting a response corresponding to the request; and a function providing module for providing, as a function for extending the system based on utilization of the first data and the second data, a function to display the plurality of classification candidates based on the metadata including the plurality of classification candidates of the blood cells.

Item 40 The application software according to Item 39, wherein the requesting module requests the interface to utilize the second data including the metadata indicating the plurality of classification candidates classified by image analysis on the blood cell images.

Item 41 The application software according to Item 39, wherein the requesting module requests the interface to utilize the second data including the metadata, and the function providing module provides a function to display the plurality of classification candidates in association with classification probabilities of each of the plurality of classification candidates.

Item 42 The application software according to Item 39, wherein the requesting module requests the interface to utilize the second data in which a plurality of the metadata is associated with identification information related to the specimen.

Item 43 The application software according to Item 39, wherein the requesting module requests the interface to utilize the second data in which each of a plurality of the metadata and a plurality of the blood cell images is associated with identification information related to the specimen.

Item 44 The application software according to Item 39, wherein the requesting module requests the interface to utilize the second data in which each of a plurality of the metadata, a plurality of the blood cell images, and a plurality of image IDs distinguishing each of the plurality of blood cell images is associated with identification information related to the specimen.

Item 45 The application software according to Item 39, wherein the requesting module requests the interface to utilize the first data and the second data in which each of a plurality of the metadata, a plurality of the blood cell images, and a hematology test result of the specimen is associated with identification information related to the specimen.

Item 46 The application software according to Item 39, wherein the requesting module requests the interface to utilize the first data and the second data in which each of a plurality of the metadata, a plurality of the blood cell images, a hematology test result of the specimen, and attribute information of a subject is associated with identification information related to the specimen.

Item 47 The application software according to Item 39, wherein the requesting module requests the interface to utilize the first data and the second data in which each of a plurality of the metadata, a plurality of the blood cell images, and information on platelets based on the metadata is associated with identification information related to the specimen.

Item 48 The application software according to Item 39, wherein the requesting module requests the interface to utilize the second data in which each of the metadata and the blood cell images is associated with identification information related to the specimen, and the function providing module provides a function to display the plurality of classification candidates associated with the blood cell image.

Item 49 The application software according to Item 39, wherein the requesting module requests the interface to utilize the second data in which each of the metadata and the blood cell images is associated with identification information related to the specimen, and the function providing module provides a function to enlarge and display the blood cell image associated with the plurality of classification candidates.

Item 50 The application software according to Item 39, wherein the requesting module requests the interface to utilize the second data in which each of the metadata and the blood cell images is associated with identification information related to the specimen, and the function providing module provides a function to display the plurality of classification candidates based on a result of comparing the metadata with a predetermined rule.

Item 51 The application software according to Item 39, wherein the requesting module requests the interface to utilize the second data in which each of the metadata and the blood cell images is associated with identification information related to the specimen, and the function providing module provides a function to display the plurality of classification candidates based on the metadata compared with a plurality of predetermined rules.

Item 52 The application software according to Item 39, wherein the requesting module requests the interface to utilize the second data in which each of the metadata and the blood cell images is associated with identification information related to the specimen, and the function providing module provides a function to accept setting of a rule regarding the plurality of classification candidates.

Item 53 The application software according to Item 39, wherein the requesting module requests the interface to utilize the second data in which each of the metadata and the blood cell images is associated with identification information related to the specimen, and the function providing module provides a function to display a reference image to be compared with a cell contained in the blood cell image.

Item 54 The application software according to Item 39, wherein the requesting module requests the interface to utilize the second data in which each of the metadata and the blood cell images is associated with identification information related to the specimen, and the function providing module provides a function to display a ruler indicating an index of a size of a cell contained in the blood cell image.

Item 55 The application software according to Item 39, wherein the requesting module requests the interface to utilize the second data in which each of the metadata and the blood cell images is associated with identification information related to the specimen, and the function providing module provides a function to display the plurality of classification candidates based on a predetermined classification criterion.

Item 56 The application software according to Item 39, wherein the requesting module requests the interface to utilize the second data in which each of the metadata and the blood cell images is associated with identification information related to the specimen, and the function providing module provides a function to specify each of a plurality of the metadata utilized via the interface based on the image ID.

Item 57 The application software according to Item 39, wherein the requesting module requests the interface to utilize the second data including metadata of each of a plurality of the blood cell images, and the function providing module provides a function to display statistical information regarding a cell contained in each of the plurality of blood cell images for each attribute of the cell, and to modify the statistical information according to modification of the metadata.

Item 58 A specimen measurement system operable to optically perform a measurement on a specimen, the specimen measurement system comprising: a specimen analyzer configured to optically perform the measurement on the specimen, the specimen analyzer including: (i) a specimen suction part configured to suction the specimen, the specimen suction part comprising a nozzle to suction the specimen in a specimen container, a movement mechanism configured to move the nozzle into the specimen container and a pump configured to apply a negative pressure so that the nozzle suctions the specimen; (ii) a sample preparator configured to prepare a measurement sample by mixing the suctioned specimen and a reagent in a container; (iii) an optical detector configured to detect light from the prepared measurement sample; and (iv) a controller configured to control, according to control software, the specimen suction part, the sample preparator and the optical detector so that the specimen analyzer optically performs the measurement on the specimen, wherein the specimen analyzer analyzes, in order to obtain a measurement result, optical information which is derived from the detected light, and manages, according to management software, data relating to the specimen analyzer, wherein the data includes the measurement result; an imaging apparatus configured to capture images of blood cells in a smear of the specimen; and a computer capable of running an interface which interacts with application software programmed to utilize the data and the image, wherein the application software is independent of the specimen analyzer, wherein the interface executes operations including: (a) receiving a request from the application software; (b) retrieving, according to a predetermined rule, the images corresponding to the request, wherein the retrieved images are associated with metadata including morphological features of the blood cells; and (c) providing the retrieved images to the application software so that the application software displays, based on the metadata, a plurality of candidates indicating classification for a specific blood cell in the retrieved images that visually demonstrate the morphological features of the blood cells.

Item 59 The specimen measurement system of Item 58, wherein the interface interacts with the application software to utilize the metadata indicating the plurality of candidates classified by an image analysis of the images.

Item 60 The specimen measurement system of Item 58, wherein the interface interacts with the application software to utilize the metadata, and a function of the specimen measurement system is extended by the application software, to display, based on the metadata, the plurality of candidates associated with each classification probability of the plurality of candidates.

Item 61 The specimen measurement system of Item 58, wherein the interface interacts with the application software to utilize the metadata associated with identification information regarding the specimen.

Item 62 The specimen measurement system of Item 58, wherein the interface interacts with the application software to utilize the metadata and the images, in which each of the metadata and the image is associated with identification information regarding the specimen.

Item 63 The specimen measurement system of Item 58, wherein the interface interacts with the application software to utilize the metadata, the images, and image IDs distinguishing respective ones of the images, in which each of the metadata, the images and the image IDs is associated with identification information regarding the specimen.

Item 64 The specimen measurement system of Item 58, wherein the interface interacts with the application software to utilize the metadata, the images and a hematology test result included in the data, in which each of the metadata, the images and the hematology test result is associated with identification information regarding the specimen.

Item 65 The specimen measurement system of Item 58, wherein the interface interacts with the application software to utilize the images, the metadata, a hematology test result included in the data and attribute information of a subject, in which each of the images, the metadata, the hematology test result and the attribute information is associated with identification information regarding the specimen.

Item 66 The specimen measurement system of Item 58, wherein the interface interacts with the application software to utilize the metadata, the images and information regarding platelets based on the metadata, in which each of the metadata, the images and the information regarding platelets is associated with identification information regarding the specimen.

Item 67 The specimen measurement system of Item 58, wherein the interface interacts with the application software to utilize the metadata and the images, in which each of the metadata and the images is associated with identification information regarding the specimen, and a function of the specimen measurement system is extended by the application software, to display the plurality of candidates associated with the images.

Item 68 The specimen measurement system of Item 58, wherein the interface interacts with the application software to utilize the metadata and the images, in which each of the metadata and the images is associated with identification information regarding the specimen, and a function of the specimen measurement system is extended by the application software, to display the images associated with the plurality of candidates in an enlarged view.

Item 69 The specimen measurement system of Item 58, wherein the interface interacts with the application software to utilize the metadata and the images, in which each of the metadata and the images is associated with identification information regarding the specimen, and a function of the specimen measurement system is extended by the application software, to display the plurality of candidates based on a result obtained by comparing the metadata with a preset rule.

Item 70 The specimen measurement system of Item 58, wherein the interface interacts with the application software to utilize the metadata and the images, in which each of the metadata and the images is associated with identification information regarding the specimen, and a function of the specimen measurement system is extended by the application software, to display the plurality of candidates based on the metadata compared with a plurality of preset rules.

Item 71 The specimen measurement system of Item 58, wherein the interface interacts with the application software to utilize the metadata and the images, in which each of the metadata and the images is associated with identification information regarding the specimen, and a function of the specimen measurement system is extended by the application software, to accept rule settings related to the plurality of candidates.

Item 72 The specimen measurement system of Item 58, wherein the interface interacts with the application software to utilize the metadata and the images, in which each of the metadata and the images is associated with identification information regarding the specimen, and a function of the specimen measurement system is extended by the application software, to display a reference image to be compared with the blood cells included in the images.

Item 73 The specimen measurement system of Item 58, wherein the interface interacts with the application software to utilize the metadata and the images, in which each of the metadata and the images is associated with identification information regarding the specimen, and a function of the specimen measurement system is extended by the application software, to display a ruler indicating a size index of the blood cells included in the images.

Item 74 The specimen measurement system of Item 58, wherein the interface interacts with the application software to utilize the metadata and the images, in which each of the metadata and the images is associated with identification information regarding the specimen, and a function of the specimen measurement system is extended by the application software, to display the plurality of candidates based on a predetermined criterion.

Item 75 The specimen measurement system of Item 1, wherein the interface interacts with the application software to utilize image IDs distinguishing respective ones of the images and each of the images associated with each of the image IDs, and a function of the specimen measurement system is extended by the application software, to specify the metadata utilized via the interface, based on each of the image IDs.

Item 76 The specimen measurement system of Item 58, wherein the interface interacts with the application software to utilize the metadata, and a function of the specimen measurement system is extended by the application software, to (1) display a summary, for each attribute of the blood cells, numerically and/or linguistically representing the morphological features, wherein the summary is derived from the metadata associated with the retrieved images that visually demonstrate the morphological features of the blood cells, and (2) to modify the summary in response to a modification of the metadata.

## Claims

1. A specimen measurement system operable to optically perform a measurement on a specimen, the specimen measurement system comprising:
a specimen analyzer configured to optically perform the measurement on the specimen, the specimen analyzer including:
(i) a specimen suction part configured to suction the specimen, the specimen suction part comprising a nozzle to suction the specimen in a specimen container, a movement mechanism configured to move the nozzle into the specimen container and a pump configured to apply a negative pressure so that the nozzle suctions the specimen;
(ii) a sample preparator configured to prepare a measurement sample by mixing the suctioned specimen and a reagent in a container;
(iii) an optical detector configured to detect light from the prepared measurement sample; and
(iv) a controller configured to control, according to control software, the specimen suction part, the sample preparator and the optical detector so that the specimen analyzer optically performs the measurement on the specimen, wherein the specimen analyzer analyzes, in order to obtain a measurement result, optical information which is derived from the detected light, and manages, according to management software, data relating to the specimen analyzer, wherein the data includes the measurement result;
an imaging apparatus configured to capture images of blood cells in a smear of the specimen; and
a computer capable of running an interface which interacts with application software programmed to utilize the data and the image, wherein the application software is independent of the specimen analyzer,
wherein the interface executes operations including:
(a) receiving a request from the application software;
(b) retrieving, according to a predetermined rule, the images corresponding to the request, wherein the retrieved images are associated with metadata including morphological features of the blood cells; and
(c) providing the retrieved images to the application software so that the application software displays, based on the metadata, a plurality of candidates indicating classification for a specific blood cell in the retrieved images that visually demonstrate the morphological features of the blood cells.

2. The specimen measurement system of claim 1, wherein
the interface interacts with the application software to utilize the metadata indicating the plurality of candidates classified by an image analysis of the images.

3. The specimen measurement system of claim 1, wherein
the interface interacts with the application software to utilize the metadata, and
a function of the specimen measurement system is extended by the application software, to display, based on the metadata, the plurality of candidates associated with each classification probability of the plurality of candidates.

4. The specimen measurement system of claim 1, wherein
the interface interacts with the application software to utilize the metadata associated with identification information regarding the specimen.

5. The specimen measurement system of claim 1, wherein
the interface interacts with the application software to utilize the metadata and the images, in which each of the metadata and the image is associated with identification information regarding the specimen.

6. The specimen measurement system of claim 1, wherein
the interface interacts with the application software to utilize the metadata, the images, and image IDs distinguishing respective ones of the images, in which each of the metadata, the images and the image IDs is associated with identification information regarding the specimen.

7. The specimen measurement system of claim 1, wherein
the interface interacts with the application software to utilize the metadata, the images and a hematology test result included in the data, in which each of the metadata, the images and the hematology test result is associated with identification information regarding the specimen.

8. The specimen measurement system of claim 1, wherein
the interface interacts with the application software to utilize the images, the metadata, a hematology test result included in the data and attribute information of a subject, in which each of the images, the metadata, the hematology test result and the attribute information is associated with identification information regarding the specimen.

9. The specimen measurement system of claim 1, wherein
the interface interacts with the application software to utilize the metadata, the images and information regarding platelets based on the metadata, in which each of the metadata, the images and the information regarding platelets is associated with identification information regarding the specimen.

10. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display the plurality of candidates associated with the images.

11. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display the images associated with the plurality of candidates in an enlarged view.

12. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display the plurality of candidates based on a result obtained by comparing the metadata with a preset rule.

13. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display the plurality of candidates based on the metadata compared with a plurality of preset rules.

14. The specimen measurement system of claim 1, wherein
the interface interacts with the application software to utilize the metadata and the images, in which each of the metadata and the images is associated with identification information regarding the specimen, and
a function of the specimen measurement system is extended by the application software, to accept rule settings related to the plurality of candidates.

15. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display a reference image to be compared with the blood cells included in the images.

16. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display a ruler indicating a size index of the blood cells included in the images.

17. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display the plurality of candidates based on a predetermined criterion.

18. The specimen measurement system of claim 1, wherein
the interface interacts with the application software to utilize image IDs distinguishing respective ones of the images and each of the images associated with each of the image IDs, and
a function of the specimen measurement system is extended by the application software, to specify the metadata utilized via the interface, based on each of the image IDs.

19. The specimen measurement system of claim 1, wherein
the interface interacts with the application software to utilize the metadata, and
a function of the specimen measurement system is extended by the application software, to (1) display a summary, for each attribute of the blood cells, numerically and/or linguistically representing the morphological features, wherein the summary is derived from the metadata associated with the retrieved images that visually demonstrate the morphological features of the blood cells, and (2) to modify the summary in response to a modification of the metadata.

20. The specimen measurement system of claim 1, wherein
the interface interacts with the application software to utilize the metadata and the images, in which each of the metadata and the images is associated with identification information regarding the specimen, and
a function of the specimen measurement system is extended by the application software, to display, based on information regarding the classification probability, the plurality of candidates for a blood cell type of the blood cell images in an order.
